# EUROPEAN PATENT APPLICATION

(11) **EP 4 382 103 A1**
(43) Date of publication of application: **12.06.2024**
(21) Application number: 22866420.7
(22) Date of filing: 24.08.2022
(51) Int. Cl.: A61K 31/437, A61K 31/122, A61K 31/05, A61K 36/00, A61P 27/02

(54) **METHOD FOR TREATING RETINAL DEGENERATION**

(30) Priority: 13.09.2021 CN 202111067959
(71) Applicant: Smilebiotek Zhuhai Limited, Zhuhai City, Guangdong Province 519000 (CN)
(72) Inventor: WEI, Lai, Zhuhai, Guangdong 519000 (CN)
(74) Representative: Murgitroyd & Company
(86) International application number: PCT/CN2022/114540
(87) International publication number: WO 2023/035950

(57) **Abstract**

The present disclosure provides a treatment method for retinal degeneration. The method includes administering an inhibitor or microbicid for a microorganism to eyes or a gastrointestinal tract of a patient. Specifically, the present disclosure proves that the inhibitor or microbicid may be used as a drug for treating a retinal degeneration disease by establishing an eye disease model or an eye disease model carrier, especially, a retinal degeneration model or model carrier.

## Description

### FIELD OF THE INVENTION

The present disclosure belongs to the field of biological medicine, and particularly relates to a treatment method for retinal degeneration and use of an inhibitor or microbicid for a microorganism in preparation of a drug for treating retinal degeneration.

### BACKGROUND ART

Inherited retinal degeneration (IRD) is a group of hereditary diseases characterized by progressive loss of photoreceptor cells, including Leber congenital amaurosis (LCA), retinitis pigmentosa (RP), early-onset rod-cone cytodystrophy, rod-rod dystrophy, congenital stationary night blindness and colour blindness and Stargardt disease (Broadgate, et al., 2017). It is the most common cause of visual loss of workers in industrialized countries and has an estimated incidence rate of 1:2000 (Kutluer, et al., 2020). Due to heterogeneity of heredity and clinics, treatment of the IRDs needs a highly personalized treatment strategy. Though neuroprotection, a gene therapy and a cell replacement therapy are proposed methods for treating the different stages of the IRD, until now, only one gene therapy (Luxturna) for correcting RPE65 gene mutation has been approved by FDA for treating the LCA (Botto, et al., 2021; Ikelle, et al., 2020; Kutluer, et al., 2020). Therefore, a treatment method for saving CRB1-related visual loss is an urgent need.

In a previous study PCT/CN2018/112022 (its content is incorporated by reference in its entirety) of the inventor, it is discovered that a microorganism, when administered in a live state, may activate a complement system and induce a drusen-like lesion in a macaque body. Besides, administering a vancomycin antibiotic to vitreous humor for killing this type of microorganism or inhibiting its growth may reduce a size of the drusen-like lesion in retinal tissue of a macaque compared with a control, and an agent for killing this type of microorganism, such as bacillus megaterium, or inhibiting its growth may be used for treating age-related macular degeneration.

In previous studies PCT/CN2018/118929, PCT/CN2019/070572 and PCT/CN2019/117444 (their contents are incorporated by reference in their entirety) of the inventor, a compound/composition capable of being used for killing the microorganism or inhibiting its growth and a method for treating a microorganism infection and treating or preventing a disease or a disease symptom such as AMD related to this type of infection by using the compound/composition are reported.

Based on the previous studies, the inventor further discovers that an inhibitor or microbicid for a microorganism may be used as a drug for treating retinal degeneration.

### BRIEF SUMMARY OF THE INVENTION

The present disclosure proves that an inhibitor or microbicid for a microorganism may be used as a drug for treating retinal degeneration first by establishing an eye disease model or an eye disease model carrier, especially, a retinal degeneration model or model carrier.

Objectives of the present disclosure are implemented through the following technical solutions.

The present disclosure first provides a treatment method for retinal degeneration, including: administering an inhibitor or microbicid for a microorganism to eyes or a gastrointestinal tract of a patient.

Preferably, the retinal degeneration is progressive retinal degeneration; more preferably, the retinal degeneration is inherited retinal degeneration; more preferably, the inherited retinal degeneration includes Leber congenital amaurosis (LCA), retinitis pigmentosa (RP), early-onset rod-cone cytodystrophy, rod-rod dystrophy, congenital stationary night blindness and colour blindness and Stargardt disease.

Preferably, the microorganism is one or a combination of two or more of bacteria, archaebacteria, protists, fungi or viruses. The microorganism is the bacteria, and the bacteria are selected from: one or two or more of *Anearostipes, Bifidobacterium, Megamonas, Nitrosomonas, Oscillibacter, Tatumella, Thiobacillussp.,Clostridium, Acinetobacter, Streptococcus, Mannheimia, Fibrobacter, Prevotella, Campylobacter, Actinomyces, Hymenobacter, Escherichia, Tissierella, Klebsiella, Porphyromonas, Azospira, Aquimarina, Achromobacter, Acidithiobacillus, Burkholderia, Marinobacter, Treponema, Actinosporangium, Vibrio, Ruminococcus, Methanobrevibacter, Shigella,* Frankia, *Streptomyces, Anaeroplasma* and *Coprococcus.* More preferably, the bacteria are selected from: *Anearostipeshadrus, Bifidobacterium pseudocatenulatum, Nitrosomonas* sp.Is79A3, *Oscillibactervalericigenes, Tatumella* sp. TA1, *Megamonasfuniformis, Thiobacillus denitrificans* and *Akkermansia muciniphila.*

Preferably, the inhibitor or microbicid for the microorganism includes a compound, polypeptide, a polynucleotide, a natural plant or a natural plant extract.

More preferably, the inhibitor or microbicid for the microorganism is an antibiotic, for example, the antibiotic is one or two or more of a β-lactam antibiotic, an aminoglycoside antibiotic, a tetracycline antibiotic, a chloramphenicol antibiotic, a macrolide antibiotic, a glycopeptide antibiotic, a quinolone antibiotic, a nitroimidazole antibiotic, a rifamycin antibiotic, an echinocandin antibiotic, a polyene antibiotic, a pyrimidine antibiotic, an allylamine antibiotic, an azole antibiotic and other antibiotics.

For example, in some embodiments, the antibiotic may include one or more of the followings: β-lactam antibiotics, including penicillins (e.g., penicillin V), amoxicillin, ampicillin, bacampicillin, carbenicillin, cloxacillin, dicloxacillin, flucloxacillin, mezlocillin, nafcillin, oxacillin, penicillin G, piperacillin, pivampicillin, pivmecillinam, ticarcillin, cephalosporins such as cefacetrile, cefadroxil, cefalexin, cefaloglycin, cefalonium, cefaloridine, cefalotin, cefapirin, cefatrizine, cefazaflur, cefazedone, cefazolin, cefradine, cefroxadine, ceftezole, cefaclor, cefamandole, cefmetazole, cefonicid, cefotetan, cefoxitin, cefprozil, cefuroxime, cefuzonam, cefcapene, cefdaloxime, cefdinir, cefditoren, cefetamet, cefixime, cefmenoxime, cefodizime, cefotaxime, cefpimizole, cefpodoxime, cefteram, ceftibuten, ceftiofur, ceftiolene, ceftizoxime, ceftriaxone, cefoperazone, ceftazidime, cefclidine, cefepime, cefluprenam, cefoselis, cefozopran, cefpirome, cefquinome, ceftobiprole, ceftaroline, cefaclomezine, cefaloram, cefaparole, cefcanel, cefedrolor, cefempidone, cefetrizole, cefivitril, cefmatilen, cefmepidium, cefovecin, cefoxazole, cefrotil, cefsumide, cefuracetime, ceftioxide, thienamycins, monobactams, β-lactamase inhibitors, methoxypenicillins, etc.; Aminoglycoside antibiotics: including streptomycin, gentamicin, kanamycin (e.g., kanamycin A), tobramycin, amikacin, neomycin (e.g., neomycin B, neomycin C and neomycin E), ribomycin, micronomicin, azithromycin, dibekacin, sisomicin, netilmicin, paramomycin, bramycin, etc.; Tetracycline antibiotics: including tetracycline, oxytetracycline, chlortetracycline and doxycycline; chloramphenicol antibiotics: including chloramphenicol, thiamphenicol, etc.; macrolide antibiotics: including erythromycin, leucomycin, odorless erythromycin, acetylspiramycin, medimycin, josamycin, azithromycin, clarithromycin, dirithromycin, oxithromycin, telithromycin, etc.; glycopeptide antibiotics: including vancomycin, norvancomycin, teicoplanin, etc.; quinolone antibiotics : including norfloxacin, ofloxacin, ciprofloxacin, pefloxacin, gatifloxacin, enoxacin, lomefloxacin, nalidixic acid, levofloxacin, moxifloxacin, besifloxacin; nitroimidazole antibiotics: including metronidazole, tinidazole, omidazole, etc.; rifamycinoid antibiotics: including rifampicin; echinocandin antibiotics; polyene antibiotics; pyrimidines antibiotics; allylamine antibiotics; azole antibiotics; other antibiotics: fosfomycin, capreomycin, cycloserine, lincomycin, clindamycin, mitomycin, actinomycin D, bleomycin, doxorubicin, isoniazid, pyrazinamide, cyclosporine, polymyxin B combinations such as polymyxin B/trimethoprim, polymyxin B/bacitracin, polymyxin B/neomycin/gramicidin, etc.

In some embodiments, the antibiotic may be selected from Amikacin, Amoxicillin, Ampicillin, Arsphenamine, Azithromycin, Azlocillin, Aztreonam, Bacitracin, Capreomycin, Carbenicillin, Cefaclor, Cefadroxil, Cefalexin, Cefalotin, Cefamandole, Cefazolin, Cefdinir, Cefditoren, Cefixime, Cefoperazone, Cefotaxime, Cefoxitin, Cefpodoxime, Cefprozil, Ceftazidime, Ceftibuten, Ceftizoxime, Ceftriaxone, Cefuroxime, Chloramphenicol, Cilastatin, Clarithromycin, Clavulanate, Clindamycin, Clofazimine, Cloxacillin, Colistin, Cycloserine, Dalfopristin, Dapsone, Daptomycin, Dicloxacillin, Dirithromycin, Doripenem, Doxycycline, Erythromycin, Ethambutol, Ethionamide, Flucloxacillin, Fosfomycin, Furazolidone, Fusidic acid, Gentamicin, Imipenem, Isoniazid, Kanamycin, Lincomycin, Linezolid, Loracarbef, Mafenide, Meropenem, Methicillin, Metronidazole, Mezlocillin, Minocycline, Mupirocin, Nafcillin, Neomycin, Netilmicin, Nitrofurantoin, Oxacillin, Oxytetracycline, Paromomycin, Penicillin G, Penicillin V, Piperacillin, Platensimycin, Polymyxin B, Pyrazinamide, Quinupristin, Rapamycin, Rifabutin, Rifampicin, Rifampin, Rifapentine, Rifaximin, Roxithromycin, Silver sulfadiazine, Spectinomycin, Streptomycin, Sulbactam, Sulfacetamide, Sulfadiazine, Sulfamethizole, Sulfamethoxazole, Sulfanilimide, Sulfasalazine, Sulfisoxazole, Tazobactam, Teicoplanin, Telavancin, Telithromycin, Temocillin, Tetracycline, Thiamphenicol, Ticarcillin, Tigecycline, Tinidazole, Tobramycin, Trimethoprim, Troleandomycin Vancomycin, enoxacin, lomefloxacin, nalidixic acid, ciprofloxacin, levofloxacin, gatifloxacin, moxifloxacin, ofloxacin, norfloxacin, Cefotetan, Cefonicid, Cephradine, Cephapirin, Cephalothin, Cefmetazole, Cefotaxime, Moxalactam, Cefepime, Ceftaroline fosamil, Ceftobiprole, Dalbavancin, Demeclocycline, Metacycline, Ertapenem, Fidaxomicin, geldanamycin, herbimycin, Posizolid, Radezolid, Torezolid, Oritavancin, Spiramycin, Sulfadimethoxine, Sulfonamidochrysoidine, Gemifloxacin Nadifloxacin Trovafloxacin Grepafloxacin Sparfloxacin Temafloxacin, Teixobactin, Malacidins, and combinations thereof.

Preferably, the inhibitor or microbicid for the microorganism is a compound of a formula I (e.g., a formula I-1, a formula I-2, a formula I-3, a formula I-4, and a formula I-5), a formula II (e.g., a formula II-1, a formula II-2, a formula II-3, a formula II-4, a formula II-5, a formula II-6, a formula II-7, a formula II-8, a formula II-9, and a formula II-10), a formula III (e.g., a formula III-1, a formula III-2, and a formula III-3), a formula IV-1 or IV-2 (e.g., a formula IV-3, a formula IV-4, a formula IV-5, and a formula IV-6), a glycoside (e.g., a formula V), or a pharmaceutically acceptable salt or ester thereof, where an aglycone of the glycoside is a phenolic compound, a flavonoid, coumarin, benzoic acid, or a sterol, where the formula I, the formula II, the formula III, the formula IV-1, the formula IV-2, the formula V, and subformulae thereof are defined as follows.

In some embodiments, the compounds of the present disclosure may be represented by having the formula I, or a pharmaceutically acceptable salt or ester thereof:

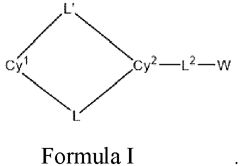

For the avoidance of doubt, in the formula I, a cyclic structure Cy¹ is connected with another cyclic structure Cy², which may be the same or different, through two linkers, L and L', which form an additional ring structure between Cy¹ and Cy². It should be understood that both Cy¹ and Cy² are separately a ring structure, which is independent of L and L'.

In the formula I, Cy¹ and Cy² are each independently an optionally substituted cycloalkyl ring (e.g., C₃₋₇ cycloalkyl ring), an optionally substituted heterocyclic ring, such as an optionally substituted 4-7 membered heterocyclic ring (e.g., having one or two ring heteroatoms independently selected from N, O, and S), an optionally substituted aryl ring (e.g., C₆₋₁₀ aryl ring (e.g., phenyl)), or an optionally substituted heteroaryl ring, such as an optionally substituted 5-10 membered heteroaryl ring (e.g., 5- or 6-membered heteroaryl ring with one or two ring heteroatoms independently selected from N, O, and S);
L and L' are each independently null or a linker (e.g., described herein); as used herein, the term "linker" is not restricted to any particular types of linking groups. For example, in some embodiments, the linker may also form a ring structure with one of the moieties that it is attached to, for example, L and Cy¹ may form a ring structure independent of Cy²;
L² is null, optionally substituted C₁₋₆ alkylene, optionally substituted C₁₋₆ heteroalkylene, optionally substituted C₂₋₆ alkenylene, optionally substituted C₂₋₆ alkynylene, optionally substituted C₃₋₆ cycloalkylene, optionally substituted arylene, optionally substituted heteroarylene, or optionally substituted 4-7 membered heterocyclylene;
W is -OR¹; -COR²; -COOR^{1a}; -OCOOR^{1a}; -NR³R⁴; -CONR^{3a}R^{4a}; -OCONR^{3b}R^{4b};-SO₂NR^{3c}R^{4c}; -OSO₂NR^{3d}R^{4d}; -SR⁵; -SO₂R^{5a}; -OCOR^{2a}; -OSO₂R^{5a} or 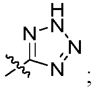
where:
   R¹ and R^{1a} are each independently hydrogen, optionally substituted alkyl, optionally substituted alkenyl, optionally substituted alkynyl, optionally substituted cycloalkyl, optionally substituted aryl, optionally substituted heteroaryl, or optionally substituted heterocyclyl;
   R³ and R⁴ are each independently hydrogen, -COR^{2b}, -SO₂R^{5b}, optionally substituted C₁₋₆ alkyl, optionally substituted C₂₋₆ alkenyl, optionally substituted C₂₋₆ alkynyl, optionally substituted C₃₋₆ cycloalkyl, optionally substituted phenyl, optionally substituted 5 or 6 membered heteroaryl, or optionally substituted 4-7 membered heterocyclyl, or R³ and R⁴ together with the atoms they are bound to form optionally substituted 4-7 membered heterocyclyl;
   R², R^{2a}, R^{2b}, R⁵, R^{5a}, and R^{5b} are each independently hydrogen, -OH, -NR^{3e}R^{4e}, optionally substituted C₁₋₆ alkyl, optionally substituted C₂₋₆ alkenyl, optionally substituted C₂₋₆ alkynyl, optionally substituted C₁₋₆ alkoxy, optionally substituted C₃₋₆ cycloalkyl, optionally substituted C₃₋₆ cycloalkoxy, optionally substituted phenyl; optionally substituted 5 or 6 membered heteroaryl; or optionally substituted 4-7 membered heterocyclyl; and
   R^{3a}, R^{3b}, R^{3c}, R^{3a}, R^{3e}, R^{4a}, R^{4b}, R^{4c}, R^{4d}, and R^{4e} are each independently hydrogen, optionally substituted C₁₋₆ alkyl, optionally substituted C₂₋₆ alkenyl, optionally substituted C₂₋₆ alkynyl, optionally substituted C₁₋₆ alkoxy, optionally substituted C₃₋₆ cycloalkyl, optionally substituted C₃₋₆ cycloalkoxy, optionally substituted phenyl; optionally substituted 5 or 6 membered heteroaryl; or optionally substituted 4-7 membered heterocyclyl; or R^{3a} and R^{4a}, R^{3b} and R^{4b}, R^{3c} and R^{4c}, R^{3d} and R^{4d}, or R^{3e} and R^{4e}, together with the atoms they are bound to form optionally substituted 4-7 membered heterocyclyl.

Cy¹ and Cy² in the formula I may be either an aromatic or non-aromatic ring system, and may in some cases include heteroatoms. In preferred embodiments, at least one of Cy¹ and Cy² in the formula I is an aryl or heteroaryl ring, such as an optionally substituted C₆₋₁₀ aryl ring, or an optionally substituted 5-10 membered heteroaryl ring. For example, in some embodiments, Cy¹ and Cy² are such that the core structure of the formula I, the structure of 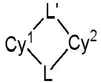 without showing optional substituents, may be any of the following: 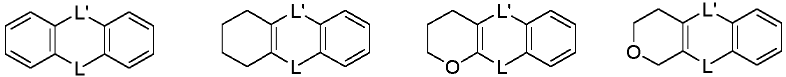 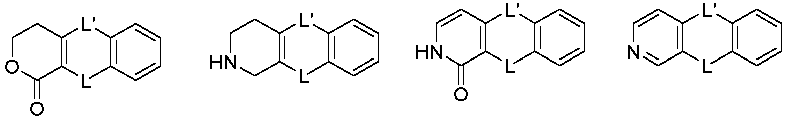 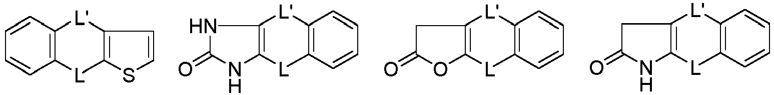 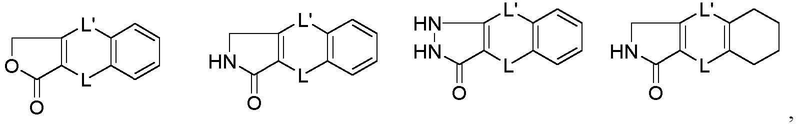 where L²-W may be attached to either the left or the right ring, where L and L' may be any of those described herein and suitable substituents for the rings are described herein.

In some embodiments, both Cy¹ and Cy² in the formula I may be an aryl or heteroaryl ring. For example, in some embodiments, the compound of the formula I may have a formula 1-1:

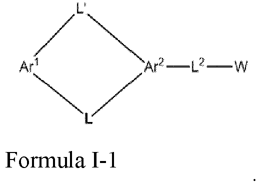

In some embodiments, Ar¹ and Ar² in the formula I-1 are each independently an optionally substituted C₆₋₁₀ aryl ring, or an optionally substituted 5-10 membered heteroaryl ring. In some embodiments, Ar¹ and Ar² in the formula 1-1 are each independently an optionally substituted phenyl ring or a 5 or 6 membered heteroaryl ring. For example, in some embodiments, Ar¹ and Ar² in the formula I-1 are each independently an optionally substituted phenyl ring, an optionally substituted thienyl ring, an optionally substituted furanyl ring, an optionally substituted pyridyl ring, or an optionally substituted pyrimidinyl ring.

The formula I-1 typically has a polycyclic core structure. For example, in some embodiments, Ar¹ and Ar² are such that the core structure of the formula I-1, 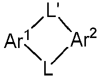 without showing optional substituents, may be any of the following: 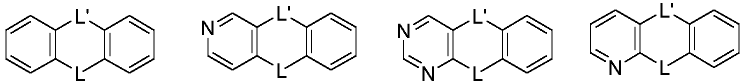 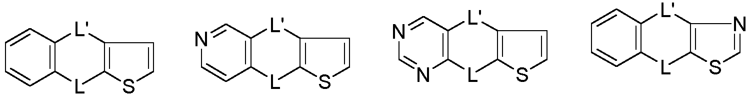 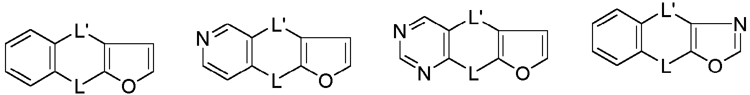 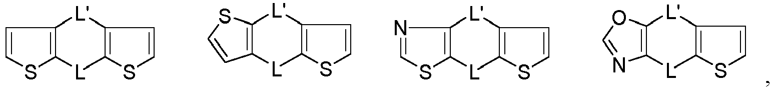 where L²-W may be attached to either the left or the right ring, where L and L' are defined herein and suitable substituents for the rings are described herein.

In some embodiments, the compound of the formula I may have a formula I-2: 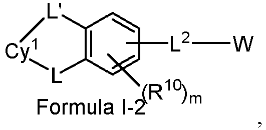 where:
m is 0, 1, 2, or 3,
R¹⁰ at each occurrence is independently halogen, L^{2'}-W^{'}, optionally substituted C₁₋₆ alkyl, optionally substituted C₂₋₆ alkenyl, optionally substituted C₂₋₆ alkynyl, optionally substituted C₁₋₆ alkoxy, optionally substituted C₃₋₆ cycloalkyl, optionally substituted C₃₋₆ cycloalkoxy, optionally substituted phenyl; optionally substituted 5 or 6 membered heteroaryl; or optionally substituted 4-7 membered heterocyclyl; or two adjacent R¹⁰, or one R¹⁰ and L or L', together with the atoms they are bound to, form optionally substituted cycloalkyl, heterocyclyl, aryl, or heteroaryl ring;
where -L^{2'}-W^{'} at each occurrence is independently selected; and
L^{2'} at each occurrence is independently null, optionally substituted C₁₋₆ alkylene, optionally substituted C₁₋₆ heteroalkylene, optionally substituted C₂₋₆ alkenylene, optionally substituted C₂₋₆ alkynylene, optionally substituted C₃₋₆ cycloalkylene, optionally substituted arylene, optionally substituted heteroarylene, or optionally substituted 4-7 membered heterocyclylene; and W^{'} at each occurrence is independently -OR¹; -COR²; -COOR^{1a}; - OCOOR^{1a}; -NR³R⁴; -CONR^{3a}R^{4a}; -OCONR^{3b}R^{4b}; -SO₂NR^{3c}R^{4c}; -OSO₂NR^{3d}R^{4d}; -SR⁵; - SO₂R^{5a}; -OCOR^{2a}; -OSO₂R^{5a} or 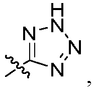 where R¹, R^{1a}, R², R^{2a}, R^{2b}, R³, R⁴, R^{3a}, R^{3b}, R^{3c}, R^{3d}, R^{3e}, R^{4a}, R^{4b}, R^{4c}, R^{4d}, R^{4e}, R⁵, R^{5a}, and R^{5b} are defined herein, see e.g., the formula I.

It should be noted that each instance of the structural units -L^{2'}-W^{'} and -L²-W is independently selected and may be the same or different.

In some embodiments, Cy¹ in the formula I-2 is an optionally substituted phenyl ring, an optionally substituted thienyl ring, an optionally substituted furanyl ring, an optionally substituted pyridyl ring, or an optionally substituted pyrimidinyl ring. In some embodiments, Cy¹ in the formula I-2 is an optionally substituted C₃₋₆ cycloalkyl ring or an optionally substituted 4-7 heterocyclic ring with 1 or 2 ring heteroatoms independently selected from N, O, and S.

In some embodiments, Cy¹ is such that the core structure of the formula I-2 may be any of the following: 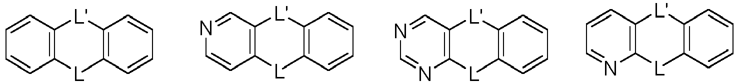 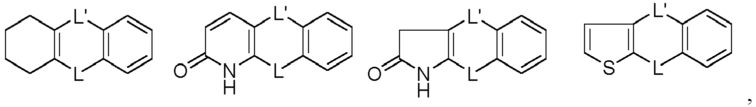 where - L²-W is attached to the right phenyl ring, L and L^{'} are defined herein and suitable substituents for the rings are described herein.

In more preferred embodiments, both Cy¹ and Cy² in the formula I are phenyl rings. For example, in some embodiments, the compound of the formula I-2 may have a formula I-3: 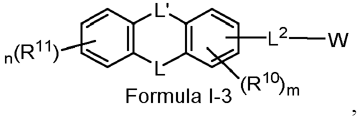 where: L, L^{'}, L², W, R¹⁰, and m are defined herein, see e.g., the formula I-2,
n is 0, 1, 2, or 3,
R¹¹ at each occurrence is independently halogen, -L^{2'}-W^{'}, optionally substituted C₁₋₆ alkyl, optionally substituted C₂₋₆ alkenyl, optionally substituted C₂₋₆ alkynyl, optionally substituted C₁₋₆ alkoxy, optionally substituted C₃₋₆ cycloalkyl, optionally substituted C₃₋₆ cycloalkoxy, optionally substituted phenyl; optionally substituted 5 or 6 membered heteroaryl; or optionally substituted 4-7 membered heterocyclyl; or two adjacent R¹¹, or one R¹¹ and L or L^{'}, together with the atoms they are bound to form an optionally substituted cycloalkyl, heterocyclyl, aryl, or heteroaryl ring; where L^{2'} and W' are defined herein, see e.g., the formula I-2, and -L^{2'}-W^{'} at each occurrence is independently selected.
L and L^{'} in the formula I (e.g., any of the formulae I-1 to I-3) may be independently null or a linker. In some embodiments, L and L^{'} in the formula I are each independently null, - C(O)-, optionally substituted C₁₋₄ alkylene, optionally substituted C₂₋₄ alkenylene, -O-, -S-, - NR¹⁰⁰-, -S(O)-, -SO₂-, -X¹-G¹-, -X²-G²-X^{2a}-, or -CR¹⁰¹R¹⁰²-,
where:
X¹, X², and X^{2a} are independently optionally substituted C₁₋₄ alkylene, optionally substituted C₂₋₄ alkenylene, -O-, -C(O)-, -S-, -NR^{100a}-, -S(O)-, -SO₂-, or -CR^{101a}R^{102a}-;
G¹ and G² are independently optionally substituted C₁₋₄ alkylene, optionally substituted C₂₋₄ alkenylene, -C(O)-, -NR^{100a}-, -S(O)-, -SO₂-, or -CR^{101a}R^{102a}-;
preferably, in some embodiments, -X¹-G¹- or -x²-G²-X^{2a}- does not contain an O-N, S-S, S-N (other than SO₂-N), or -C(O)-S bond;
R¹⁰⁰ and R^{100a} are each independently lone pair (as applicable), hydrogen, COR^{2c}, - SO₂R^{5c}, optionally substituted C₁₋₆ alkyl, optionally substituted C₂₋₆ alkenyl, optionally substituted C₂₋₆ alkynyl, optionally substituted C₃₋₆ cycloalkyl, optionally substituted phenyl, optionally substituted 5 or 6 membered heteroaryl, or optionally substituted 4-7 membered heterocyclyl; or R¹⁰⁰ or R^{100a} forms an optionally substituted heterocyclic or heteroaryl ring with an R¹⁰ or R¹¹ group;
R¹⁰¹, R^{101a}, R¹⁰², and R^{102a}, when present, are each independently hydrogen, -OH, halogen, optionally substituted C₁₋₆ alkyl, optionally substituted C₂₋₆ alkenyl, optionally substituted C₂₋₆ alkynyl, optionally substituted C₃₋₆ cycloalkyl, optionally substituted C₁₋₆ alkoxy, optionally substituted C₃₋₆ cycloalkoxy, optionally substituted amino group, optionally substituted phenyl, optionally substituted 5 or 6 membered heteroaryl, or optionally substituted 4-7 membered heterocyclyl, or R¹⁰¹ and R¹⁰², or R^{101a} and R^{102a}, together with the atoms they are bound to form an optionally substituted 3-7 membered cycloalkyl or heterocyclyl ring; or one of R¹⁰¹ and R¹⁰², or one of R^{101a} and R^{102a} forms an optionally substituted cycloalkyl or heterocyclyl ring together with an R¹⁰ or R¹¹ group; and
R^{2c} and R^{5c} are each independently hydrogen, optionally substituted C₁₋₆ alkyl, optionally substituted C₂₋₆ alkenyl, optionally substituted C₂₋₆ alkynyl, optionally substituted C₁₋₆ alkoxy, optionally substituted C₃₋₆ cycloalkyl, optionally substituted C₃₋₆ cycloalkoxy, optionally substituted phenyl; optionally substituted 5 or 6 membered heteroaryl; or optionally substituted 4-7 membered heterocyclyl.

When the linker L or L^{'} forms a double bond with one of the ring carbons, it cannot be CR¹⁰¹R¹⁰² with both R¹⁰¹ and R¹⁰² present, as the valence of the carbon will exceed 4. In such cases, it should be understood that one of R¹⁰¹ and R¹⁰² is absent and L or L^{'} is CR¹⁰¹ or CR¹⁰² as defined herein. When L or L^{'} forms a double bond with one of the ring carbons, it may be NR¹⁰⁰ with R¹⁰⁰ typically being a lone pair. Other similar situations in the present disclosure should be understood similarly.

In some embodiments, L and L^{'} in the formula I are each independently null, -O-, -C(O)-, -S-, -NR¹⁰⁰-, -S(O)-, -SO₂-, or -CR¹⁰¹R¹⁰²-. In some embodiments, the compound of the formula I has a formula according to any one of I-4 to I-5: 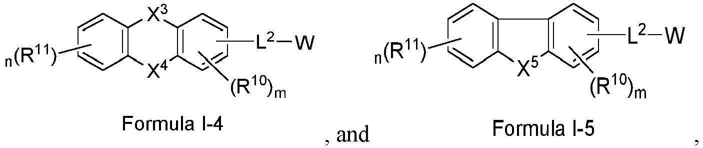 where:
X³, X⁴, and X⁵ are each independently null, -O-, -C(O)-, -S-, -NR^{100a}-, -S(O)-, -SO₂-, or - CR^{101a}R^{102a}-; and
R¹⁰, R¹¹, R^{100a}, R^{101a}, R^{102a}, W, L², m, and n are defined herein.

In some embodiments, the compound has a formula I-4, where X³ and X⁴ are each independently -O-, -C(O)-, -S-, -NR^{100a}-, or -SO₂-. In some embodiments, the compound has a formula I-5, where X⁵ is -O-, -C(O)-, -S-, -NR^{100a}-, or -SO₂-. In some embodiments, R^{100a} is hydrogen or optionally substituted C₁₋₄ alkyl.

L² in the formula I (e.g., any of the sub-formulae described herein, such as the formulae I-1 to I-5) is typically null, i.e., the W group is directly attached to Cy². In some embodiments, L² in the formula I may also be C₁₋₄ alkylene, C₂₋₄ alkenylene, C₂₋₄ alkynylene or C₁₋₄ heteroalkylene. For example, the W group may be attached to Cy², through a methylene or vinyl group.

Various W groups are suitable for compounds of the formula I (e.g., any of the sub-formulae described herein, such as the formulae I-1 to I-5). In preferred embodiments, the W group at each occurrence is independently -OH, -NH₂, -SO₂NH₂, -SO₂NH(C₁₋₄ alkyl), - SO₂NH(C₁₋₄ alkanoyl), -COOH, 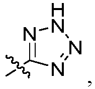 -C(O)(O-C₁₋₁₀ alkyl), -C(O)(O-C₂₋₁₀ alkenyl), - OC(O)NH₂, -OC(O)NH(C₁₋₄ alkyl)-, -O-(CO)-(C₁₋₄ alkyl), or -O-(C₁₋₄ alkyl), where each of the C₁₋₄ alkyl is independently optionally substituted with 1-3 substituents independently selected from C₁₋₄ alkyl, C₁₋₄ alkoxy, -OH, -NH₂, and fluorine. In some embodiments, W in the formula I is -OH, -NH₂, -SO₂NH₂, -SO₂NH(Acetyl), -COOH, 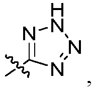 or -O-C(O)-CH₃.

As described herein, L^{2'}-W^{'} may in some embodiments be selected as a substituent for Cy¹ or Cy², such as for Ar¹ or Ar². When applicable, L^{2'} in the formula I, including any of the sub-formulae described herein, such as the formulae I-1 to I-5, at each occurrence may be independently null, i.e., the W' group is directly attached to Cy¹ or Cy², such as for Ar¹ or Ar², as applicable, or a C₁₋₄ alkylene, C₂₋₄ alkenylene, C₂₋₄ alkynylene or C₁₋₄ heteroalkylene. For example, the W' group may be attached to Cy¹ or Cy², such as for Ar¹ or Ar², as applicable, through a methylene or vinyl group. When applicable, W' in the formula I, including any of the sub-formulae described herein, such as the formulae I-1 to I-5, at each occurrence may be independently -OH, -NH₂, -SO₂NH₂, -SO₂NH(C₁₋₄ alkyl), -SO₂NH(C₁₋₄ alkanoyl), -COOH, 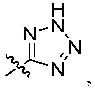 -C(O)(O-C₁₋₁₀ alkyl), -C(O)(O-C₂₋₁₀ alkenyl), -OC(O)NH₂, - OC(O)NH(C₁₋₄ alkyl)-, -O-(CO)-(C₁₋₄ alkyl), or -O-(C₁₋₄ alkyl), where each of the C₁₋₄ alkyl is independently optionally substituted with 1-3 substituents independently selected from C₁₋₄ alkyl, C₁₋₄ alkoxy, -OH, -NH₂, and fluorine. In some embodiments, each instance of W' in the formula I, when applicable, may be -OH, -NH₂, -SO₂NH₂, -SO₂NH(Acetyl), -COOH, 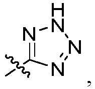 or -O-C(O)-CH₃.

Various groups may be suitable for R¹⁰ and R¹¹ in any of the applicable formula I (e.g., any of the sub-formulae described herein, such as the formulae I-2 to I-5, as applicable). In some embodiments, each of R¹⁰ and R¹¹ at each occurrence may be independently F; Cl; - OH; -NH₂; -SO₂NH₂; -SO₂NH(C₁₋₄ alkyl); -SO₂NH(C₁₋₄ alkanoyl); -COOH; 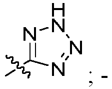 C(O)(O-C₁₋₁₀ alkyl), -C(O)(O-C₂₋₁₀ alkenyl), -OC(O)NH₂; -OC(O)NH(C₁₋₄ alkyl)-; -O-(CO)-(C₁₋₄ alkyl); C₁₋₄ alkyl optionally substituted with 1-3 substituents independently selected from C₁₋₄ alkyl, C₁₋₄ alkoxy, -OH, -NH₂, and fluorine; C₂₋₆ alkenyl optionally substituted with 1-3 substituents independently selected from C₁₋₄ alkyl, C₁₋₄ alkoxy, -OH, -NH₂, and fluorine; C₂₋₆ alkynyl optionally substituted with 1-3 substituents independently selected from C₁₋₄ alkyl, C₁₋₄ alkoxy, -OH, -NH₂, and fluorine; C₃₋₆ cycloalkyl optionally substituted with 1-3 substituents independently selected from C₁₋₄ alkyl and fluorine; C₃₋₆ cycloalkoxy optionally substituted with 1-3 substituents independently selected from C₁₋₄ alkyl and fluorine; or C₁₋₄ alkoxy optionally substituted with 1-3 substituents independently selected from C₁₋₄ alkyl, C₁₋₄ alkoxy, -OH, -NH₂, and fluorine. In some embodiments, each of R¹⁰ and R¹¹ at each occurrence may be independently -OH; -NH₂; -SO₂NH₂; -SO₂NH(C₁₋₄ alkyl); -SO₂NH(C₁₋₄ alkanoyl); -COOH; 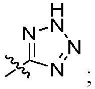 -C(O)(O-C₁₋₁₀ alkyl), -C(O)(O-C₂₋₁₀ alkenyl), -OC(O)NH₂; - OC(O)NH(C₁₋₄ alkyl)-; -O-(CO)-(C₁₋₄ alkyl); C₁₋₄ alkyl; or C₁₋₄ alkoxy. In some embodiments, one or more instances of R¹⁰ and/or one or more instances of R¹¹ may be independently selected from L^{2'}-W^{'} as described herein.

Typically, m, as applicable, is 0, 1, or 2; preferably, 1.

Typically, n, as applicable, is 0, 1, 2, or 3; preferably, 1 or 2.

In some embodiments, the compounds herein may be characterized by having a formula II, or a pharmaceutically acceptable salt or ester thereof:

Cy¹⁰-L¹⁰-Cy¹¹-L¹¹-W¹⁰ Formula II

where:
Cy¹⁰ and Cy¹¹ are each independently an optionally substituted cycloalkyl ring (e.g., C₃₋₇ cycloalkyl ring), an optionally substituted heterocyclic ring (e.g., 4-7 membered heterocyclic ring), an optionally substituted aryl ring (e.g., C₆₋₁₀ aryl ring), an optionally substituted heteroaryl ring (e.g., 5-10 membered heteroaryl ring), or an optionally substituted ring structure including a cycloalkyl ring or heterocyclic ring, and an aryl or heteroaryl ring, where the ring structure may be a fused ring or otherwise connected;
L¹⁰ is null or a linker;
L¹¹ is null, optionally substituted C₁₋₆ alkylene, optionally substituted C₁₋₆ heteroalkylene, optionally substituted C₂₋₆ alkenylene, optionally substituted C₂₋₆ alkynylene, optionally substituted C₃₋₆ cycloalkylene, optionally substituted arylene, optionally substituted heteroarylene, or optionally substituted 4-7 membered heterocyclylene,
W¹⁰ is -OR¹; -COOR^{1a}; -OCOOR^{1a}; -COR²; -NR³R⁴; -CONR^{3a}R^{4a}; -OCONR^{3b}R^{4b}; - SO₂NR^{3c}R^{4c}; -OSO₂NR^{3d}R^{4d}; -SR⁵; -SO₂R^{5a}; -OCOR^{2a}; -OSO₂R^{5a}; or 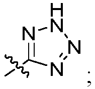 where:
   R¹ and R^{1a} are each independently hydrogen, optionally substituted alkyl, optionally substituted alkenyl, optionally substituted alkynyl, optionally substituted cycloalkyl, optionally substituted aryl, optionally substituted heteroaryl, or optionally substituted heterocyclyl;
   R³ and R⁴ are each independently hydrogen, -COR^{2b}, -SO₂R^{5b}, optionally substituted C₁₋₆ alkyl, optionally substituted C₂₋₆ alkenyl, optionally substituted C₂₋₆ alkynyl, optionally substituted C₃₋₆ cycloalkyl, optionally substituted phenyl, optionally substituted 5 or 6 membered heteroaryl, or optionally substituted 4-7 membered heterocyclyl, or R³ and R⁴ together with the atoms they are bound to form optionally substituted 4-7 membered heterocyclyl;
   R², R^{2a}, R^{2b}, R⁵, R^{5a}, and R^{5b} are each independently hydrogen, -OH, -NR^{3e}R^{4e}, optionally substituted C₁₋₆ alkyl, optionally substituted C₂₋₆ alkenyl, optionally substituted C₂₋₆ alkynyl, optionally substituted C₁₋₆ alkoxy, optionally substituted C₃₋₆ cycloalkyl, optionally substituted C₃₋₆ cycloalkoxy, optionally substituted phenyl; optionally substituted 5 or 6 membered heteroaryl; or optionally substituted 4-7 membered heterocyclyl; and
   R^{3a}, R^{3b}, R^{3c}, R^{3a}, R^{3e}, R^{4a}, R^{4b}, R^{4c}, R^{4d}, and R^{4e} are each independently hydrogen, optionally substituted C₁₋₆ alkyl, optionally substituted C₂₋₆ alkenyl, optionally substituted C₂₋₆ alkynyl, optionally substituted C₁₋₆ alkoxy, optionally substituted C₃₋₆ cycloalkyl, optionally substituted C₃₋₆ cycloalkoxy, optionally substituted phenyl; optionally substituted 5 or 6 membered heteroaryl; or optionally substituted 4-7 membered heterocyclyl; or R^{3a} and R^{4a}, R^{3b} and R^{4b}, R^{3c} and R^{4c}, R^{3d} and R^{4d}, or R^{3e} and R^{4e}, together with the atoms they are bound to form optionally substituted 4-7 membered heterocyclyl.

In some embodiments, in the formula II, at least one of Cy¹⁰ and Cy¹¹ is an optionally substituted C₆₋₁₀ aryl ring, or an optionally substituted 5-10 membered heteroaryl ring. In some embodiments, Cy¹¹ is an optionally substituted C₆₋₁₀ aryl ring, or an optionally substituted 5-10 membered heteroaryl ring. When Cy¹¹ is a bicyclic or polycyclic aryl or heteroaryl ring, L¹⁰-Cy¹⁰ and L¹¹-W¹⁰ may be independently connected to Cy¹¹ through any of the rings. In some embodiments, Cy¹¹ may have a fused ring structure including an aryl or heteroaryl ring and a cycloalkyl or heterocyclic ring structure. In such embodiments, Cy¹¹ may be connected to L¹⁰-Cy¹⁰ and L¹¹-W¹⁰ through either of the aryl or heteroaryl ring and cycloalkyl or heterocyclic ring structure; or alternatively, one of L¹⁰-Cy¹⁰ and L¹¹-W¹⁰ is connected to Cy¹¹ through the aryl or heteroaryl ring and the other of L¹⁰-Cy¹⁰ and L¹¹-W¹⁰ is connected to Cy¹¹ through the cycloalkyl or heterocyclic ring structure.

In some embodiments, the compound of the formula II has at least one phenyl ring, which may have the following core structure as Cy¹⁰-L¹⁰-Cy¹¹: 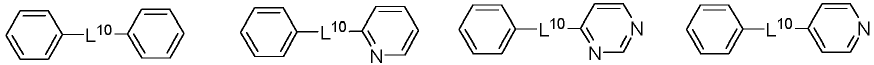 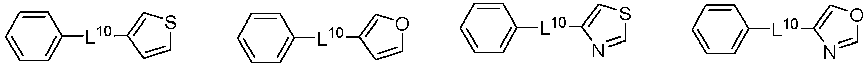 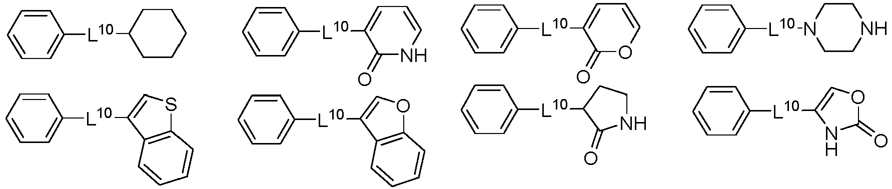 , where Cy¹⁰ may be the left ring or the right ring in the above drawing, i.e., the drawing is not limited to a particular direction, where L¹¹-W¹⁰ may be connected to either the left or the right ring, both of which may be optionally substituted.

In some embodiments, the compound of the formula II may have the following core structure as Cy¹⁰-L¹⁰-Cy¹¹: 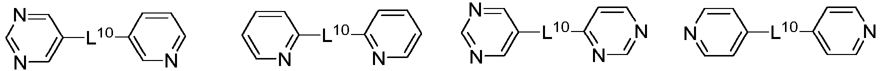 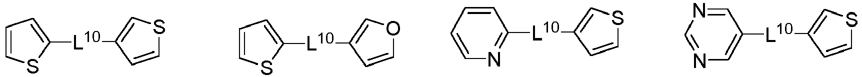 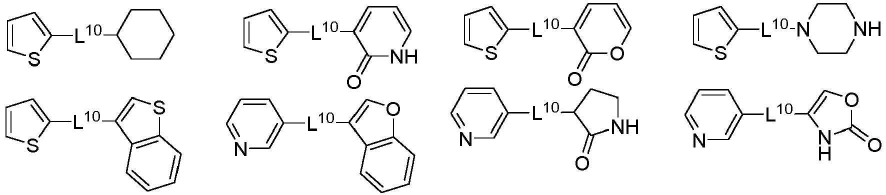 , where Cy¹⁰ may be the left ring or the right ring in the above drawing, i.e., the drawing is not limited to a particular direction, where L¹¹-W¹⁰ may be connected to either the left or the right ring, both of which may be optionally substituted.

In some embodiments, both of Cy¹⁰ and Cy¹¹ in the formula II are an aryl or heteroaryl ring. In some embodiments, the compound of the formula II has a formula II-1:

Ar¹⁰-L¹⁰-Ar¹¹-L¹¹-W¹⁰ Formula II-1

where Ar¹⁰ and Ar¹¹ are each independently an optionally substituted C₆₋₁₀ aryl ring, or an optionally substituted 5-10 membered heteroaryl ring. In some embodiments, Ar¹⁰ and Ar¹¹ in the formula II-1 are each independently an optionally substituted phenyl ring or an optionally substituted 5 or 6 membered heteroaryl ring. In some embodiments, Ar¹⁰ and Ar¹¹ in the formula II-1 are each independently an optionally substituted phenyl ring, an optionally substituted thienyl ring, an optionally substituted furanyl ring, an optionally substituted pyridyl ring, or an optionally substituted pyrimidinyl ring. In some embodiments, one of Ar¹⁰ and Ar¹¹ in the formula II-1 is a bicyclic aryl or bicyclic heteroaryl ring, each of which is optionally substituted, for example, in some embodiments, Ar¹¹ may be an optionally substituted bicyclic aryl or bicyclic heteroaryl ring.

In some embodiments, Cy¹¹ in the formula II is a phenyl ring. In some embodiments, the compound of the formula II has a formula II-2: 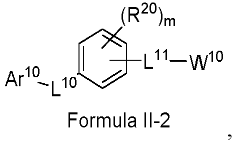
where Ar¹⁰, L¹⁰, L¹¹, and W¹⁰ are defined herein, see e.g., the formula II-1,
m is 0, 1, 2, or 3,
R²⁰ at each occurrence is independently halogen, -L^{11'}-W^{10'}, optionally substituted C₁₋₆ alkyl, optionally substituted C₂₋₆ alkenyl, optionally substituted C₂₋₆ alkynyl, optionally substituted C₁₋₆ alkoxy, optionally substituted C₃₋₆ cycloalkyl, optionally substituted C₃₋₆ cycloalkoxy, optionally substituted phenyl; optionally substituted 5 or 6 membered heteroaryl; or optionally substituted 4-7 membered heterocyclyl; or two adjacent R²⁰, or one R²⁰ and L¹⁰ or L¹¹, together with the atoms they are bound to form an optionally substituted cycloalkyl, heterocyclyl, aryl, or heteroaryl ring;
where -L^{11'}-W^{10'} at each occurrence is independently selected;
where L^{11'} at each occurrence is independently null, optionally substituted C₁₋₆ alkylene, optionally substituted C₁₋₆ heteroalkylene, optionally substituted C₂₋₆ alkenylene, optionally substituted C₂₋₆ alkynylene, optionally substituted C₃₋₆ cycloalkylene, optionally substituted arylene, optionally substituted heteroarylene, or optionally substituted 4-7 membered heterocyclylene; and W^{10'} at each occurrence is independently -OR¹; -COR²; -COOR^{1a}; - OCOOR^{1a}; -NR³R⁴; -CONR^{3a}R^{4a}; -OCONR^{3b}R^{4b}; -SO₂NR^{3c}R^{4c}; -OSO₂NR^{3d}R^{4d}; -SR⁵; - SO₂R^{5a}; -OCOR^{2a}; -OSO₂R^{5a} or 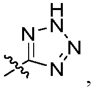 where R¹, R^{1a}, R², R^{2a}, R^{2b}, R³, R⁴, R^{3a}, R^{3b}, R^{3c}, R^{3d}, R^{3e}, R^{4a}, R^{4b}, R^{4c}, R^{4d}, R^{4e}, R⁵, R^{5a}, and R^{5b} are defined herein, see e.g., the formula II. It should be noted that each instance of the structural units -L^{11'}-W^{10'} and -L¹¹-W¹⁰ is independently selected and may be the same or different.

In some embodiments, Cy¹¹ in the formula II is a benzofused ring. In some embodiments, the compound of the formula II has a formula II-3: 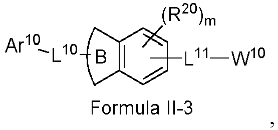
where Ar¹⁰, L¹⁰, L¹¹, and W¹⁰ are defined herein, see e.g., the formula II-1,
m is 0, 1, 2, or 3,
R²⁰ at each occurrence is independently halogen, -L^{11'}-W^{10'}, optionally substituted C₁₋₆ alkyl, optionally substituted C₂₋₆ alkenyl, optionally substituted C₂₋₆ alkynyl, optionally substituted C₁₋₆ alkoxy, optionally substituted C₃₋₆ cycloalkyl, optionally substituted C₃₋₆ cycloalkoxy, optionally substituted phenyl; optionally substituted 5 or 6 membered heteroaryl; or optionally substituted 4-7 membered heterocyclyl; or two adjacent R²⁰, or one R²⁰ and L¹⁰ or L¹¹, together with the atoms they are bound to form an optionally substituted cycloalkyl, heterocyclyl, aryl, or heteroaryl ring;
where L^{11'} and W^{10'} are defined herein, see e.g., the formula II-2, and -L^{11'}-W^{10'} at each occurrence is independently selected; and
a ring B is a 4-7 membered cycloalkyl ring, 4-7 membered heterocyclic ring, phenyl ring, 5 or 6 membered heteroaryl ring, each of which is optionally substituted.

In some embodiments, Cy¹¹ in the formula II is a benzofused bicyclic aryl or heteroaryl ring. For example, in some embodiments, Cy¹¹ in the formula II may have the following core structure: 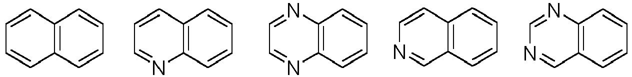 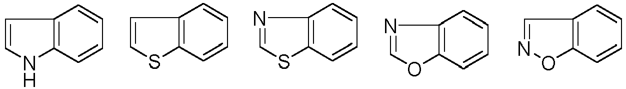 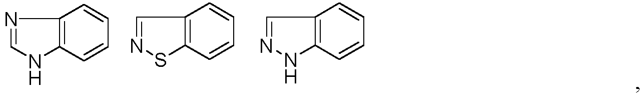 where the L¹⁰-Cy¹⁰ and L¹¹-W¹⁰ may be independently connected to Cy¹¹ through any of the two rings, where the phenyl ring may be optionally substituted with 1-3 R²⁰ groups defined herein. For example, in the case of the benzothiophene ring, in some embodiments, L¹⁰-Cy¹⁰ may be attached to the thiophene ring whereas L¹¹-W¹⁰ may be attached to the phenyl ring, or vice versa, and in some cases, both L¹⁰-Cy¹⁰ and L¹¹-W¹⁰ may be attached to the same ring, such as the phenyl ring.

In some embodiments, the compound of the formula II may have a structure of any of the following: 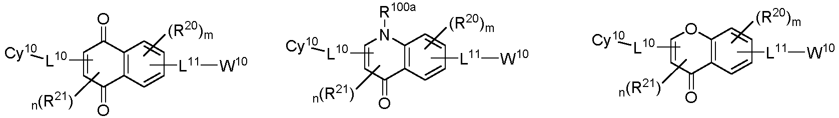 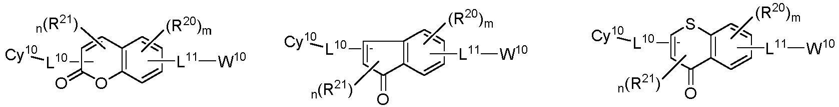 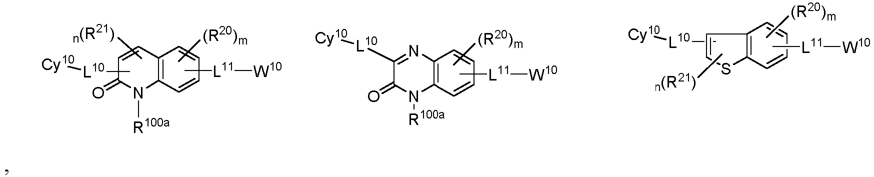 where: Cy¹⁰, L¹⁰, R²⁰, m, R²¹, n, R^{100a}, L¹¹, and W¹⁰ are defined herein, see e.g., the formula II and sub-formulae herein, such as the formula II-3. In some embodiments, Cy¹⁰ is Ar¹⁰ as defined for the formula II-3.

In some embodiments, the compound of the formula II-3 may have a formula II-4: 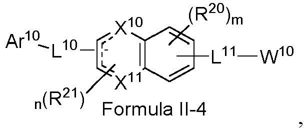
where: Ar¹⁰, L¹⁰, R²⁰, m, L¹¹, and W¹⁰ are defined herein, see e.g., the formula II-3,
n is 0 or 1,
R²¹ at each occurrence is independently halogen, oxo, -L^{11'}-W^{10'}, optionally substituted C₁₋₆ alkyl, optionally substituted C₂₋₆ alkenyl, optionally substituted C₂₋₆ alkynyl, optionally substituted C₁₋₆ alkoxy, optionally substituted C₃₋₆ cycloalkyl, optionally substituted C₃₋₆ cycloalkoxy, optionally substituted phenyl; optionally substituted 5 or 6 membered heteroaryl; or optionally substituted 4-7 membered heterocyclyl; where L^{11'} and W^{10'} are defined herein, see e.g., the formula II-2, and -L^{11'}-W^{10'} at each occurrence is independently selected;
X¹⁰ and X¹¹ are each independently null, -O-, -C(O)-, -S-, -NR^{100a}-, -S(O)-, -SO₂-, or - CR^{101a}R^{102a}-, as valence permits;
where R^{100a} is lone pair (as applicable), hydrogen, COR^{2c}, -SO₂R^{5c}, optionally substituted C₁₋₆ alkyl, optionally substituted C₂₋₆ alkenyl, optionally substituted C₂₋₆ alkynyl, optionally substituted C₃₋₆ cycloalkyl, optionally substituted phenyl, optionally substituted 5 or 6 membered heteroaryl, or optionally substituted 4-7 membered heterocyclyl; or R^{100a} forms an optionally substituted heterocyclic or heteroaryl ring with an R²⁰ or R²¹ group;
R^{101a} and R^{102a}, when present, are each independently hydrogen, -OH, halogen; optionally substituted C₁₋₆ alkyl, optionally substituted C₂₋₆ alkenyl, optionally substituted C₂₋₆ alkynyl, optionally substituted C₃₋₆ cycloalkyl, optionally substituted C₁₋₆ alkoxy, optionally substituted C₃₋₆ cycloalkoxy, optionally substituted amino group, optionally substituted phenyl, optionally substituted 5 or 6 membered heteroaryl, or optionally substituted 4-7 membered heterocyclyl; or R^{101a} and R^{102a}, together with the atoms they are bound to form an optionally substituted 3-7 membered cycloalkyl or heterocyclyl ring; or one of R^{101a} and R^{102a} forms an optionally substituted cycloalkyl or heterocyclyl ring together with an R²⁰ or R²¹ group; and
R^{2c} and R^{5c} are each independently hydrogen, optionally substituted C₁₋₆ alkyl, optionally substituted C₂₋₆ alkenyl, optionally substituted C₂₋₆ alkynyl, optionally substituted C₁₋₆ alkoxy, optionally substituted C₃₋₆ cycloalkyl, optionally substituted C₃₋₆ cycloalkoxy, optionally substituted phenyl; optionally substituted 5 or 6 membered heteroaryl; or optionally substituted 4-7 membered heterocyclyl;
or R²⁰ or R²¹ and L¹⁰, X¹⁰ or X¹¹, together with the atoms they are bound to form an optionally substituted cycloalkyl, heterocyclyl, aryl, or heteroaryl ring.

When X¹⁰ or X¹¹ forms a double bond with one of the ring carbons, it cannot be CR^{101a}R^{102a} with both R^{101a} and R^{102a} present, as the valence of the carbon will exceed 4. In such cases, it should be understood that one of R^{101a} and R^{102a} is absent and X¹⁰ or X¹¹ is CR^{101a} or CR^{102a} as defined herein. When X¹⁰ or X¹¹ forms a double bond with one of the ring carbons, it may be NR^{100a} with R^{100a} typically being a lone pair.

In some embodiments, the compound of the formula II has a formula II-5: 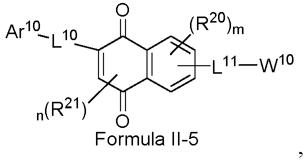 where: Ar¹⁰, L¹⁰, R²⁰, m, R²¹, n, L¹¹, and W¹⁰ are defined herein, see e.g., the formula II-4.

Cy¹⁰ and Cy¹¹ in the formula II (e.g., sub-formulae described herein, such as the formulae II-1 to II-4) may be connected directly or via various groups. For example, in some embodiments, L¹⁰ in the formula II (e.g., formulae II-1 to II-5) is null, -C(O)-, optionally substituted C₁₋₄ alkylene, optionally substituted C₂₋₄ alkenylene, optionally substituted C₃₋₆ cycloalkylene, optionally substituted 4-7 membered heterocyclylene, optionally substituted phenylene, optionally substituted 5 or 6 membered heteroarylene, -O-, -S-, -NR¹⁰⁰-, -S(O)-, - SO₂-, -X¹-G¹-, -X²-G²-X^{2a}-, -X¹²-G¹⁰-, -X¹³-G¹¹-X^{13a}-, or -CR¹⁰¹R¹⁰²-,
where:
X¹, X², and X^{2a} are independently optionally substituted C₁₋₄ alkylene, optionally substituted C₂₋₄ alkenylene, optionally substituted C₃₋₆ cycloalkylene, optionally substituted 4-7 membered heterocyclylene, optionally substituted phenylene, optionally substituted 5 or 6 membered heteroarylene, -O-, -C(O)-, -S-, -NR^{100a}-, -S(O)-, -SO₂-, or -CR^{101a}R^{102a}-;
G¹ and G² are independently optionally substituted C₁₋₄ alkylene, optionally substituted C₂₋₄ alkenylene, optionally substituted C₃₋₆ cycloalkylene, optionally substituted 4-7 membered heterocyclylene, optionally substituted phenylene, optionally substituted 5 or 6 membered heteroarylene, -C(O)-, -NR^{100a}-, -S(O)-, -SO₂-, or -CR^{101a}R^{102a}-;
preferably, in some embodiments, -X¹-G¹- or -x²-G²-X^{2a}- does not contain an O-N, S-S, S-N (except SO₂-N bond), or -C(O)-S bond;
X¹², X¹³, and X^{13a} are independently optionally substituted C₁₋₄ alkylene, optionally substituted C₂₋₄ alkenylene, optionally substituted C₃₋₆ cycloalkylene, optionally substituted 4-7 membered heterocyclylene, optionally substituted phenylene, optionally substituted 5 or 6 membered heteroarylene, -O-, -C(O)-, -S-, -NR^{100a}-, -S(O)-, -SO₂-, or -CR^{101a}R^{102a}-;
and G¹⁰ and G¹¹ are independently -X¹-G¹- or -X²-G²-X^{2a}-;
in some embodiments, preferably, -X¹²-G¹⁰- or -X¹³-G¹¹-X^{13a}- does not contain an O-O, O-N, S-S, S-N (except SO₂-N bond), or -C(O)-S bond or three (or more) consecutive heteroatoms, with the exception of O-SO₂-O, O-SO₂-N, and N-SO₂-N;
R¹⁰⁰ and R^{100a} are each independently lone pair (as applicable), hydrogen, COR^{2c}, - SO₂R^{5c}, optionally substituted C₁₋₆ alkyl, optionally substituted C₂₋₆ alkenyl, optionally substituted C₂₋₆ alkynyl, optionally substituted C₃₋₆ cycloalkyl, optionally substituted phenyl, optionally substituted 5 or 6 membered heteroaryl, or optionally substituted 4-7 membered heterocyclyl;
R¹⁰¹, R^{101a}, R¹⁰², and R^{102a} are each independently hydrogen, -OH, halogen; optionally substituted C₁₋₆ alkyl, optionally substituted C₂₋₆ alkenyl, optionally substituted C₂₋₆ alkynyl, optionally substituted C₃₋₆ cycloalkyl, optionally substituted C₁₋₆ alkoxy, optionally substituted C₃₋₆ cycloalkoxy, optionally substituted amino group, optionally substituted phenyl, optionally substituted 5 or 6 membered heteroaryl, or optionally substituted 4-7 membered heterocyclyl; or R¹⁰¹ and R¹⁰², or R^{101a} and R^{102a}, together with the atoms they are bound to form an optionally substituted 3-7 membered cycloalkyl or heterocyclyl ring.

In some embodiments, L¹⁰ in the formula II may be null, and Cy¹⁰ is directly linked with Cy¹¹. In some embodiments, L¹⁰ in the formula II may be null, -O-, -C(O)-, -S-, -NR¹⁰⁰-, - S(O)-, -SO₂-, or -CR¹⁰¹R¹⁰²-. In some embodiments, L¹⁰ in the formula II may be -X¹-G¹- or - X²-G²-X^{2a}-, where: X¹, X², and X^{2a} are independently -O-, -C(O)-, -S-, -NR^{100a}-, -S(O)-, - SO₂-, or -CR^{101a}R^{102a}-; and G¹ and G² are independently -C(O)-, -NR^{100a}-, -S(O)-, -SO₂-, or - CR^{101a}R^{102a}-.

In some embodiments, L¹⁰ in the formula II may be -X¹²-G¹⁰-. In some embodiments, X¹² is optionally substituted C₂₋₄ alkenylene, preferably, 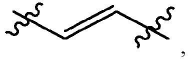 and G¹⁰ is -X¹-G¹- or -X²-G²-X^{2a}-; where: X¹, X², and X^{2a} are independently -O-, -C(O)-, -S-, -NR^{100a}-, -S(O)-, - SO₂-, or -CR^{101a}R^{102a}-; and G¹ and G² are independently -C(O)-, -NR^{100a}-, -S(O)-, -SO₂-, or - CR^{101a}R^{102a}-.

In some preferred embodiments, L¹⁰ in the formula II may be

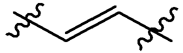

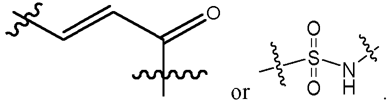

In some embodiments, the compound of the formula II may have the following core structure: 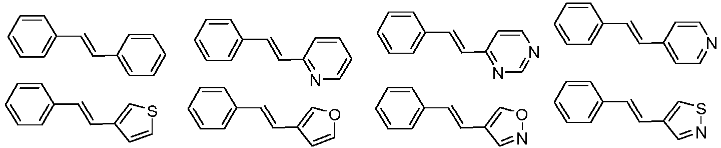 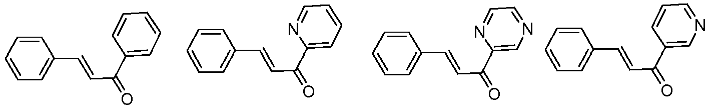 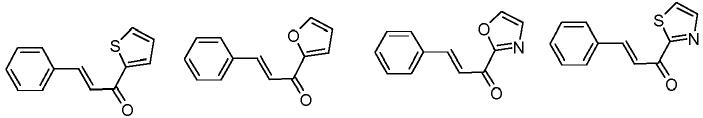 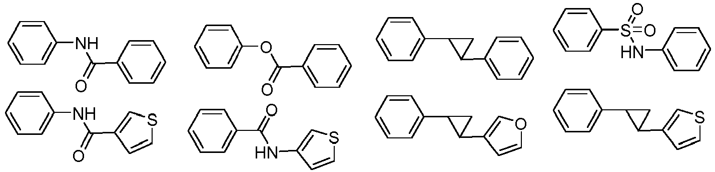 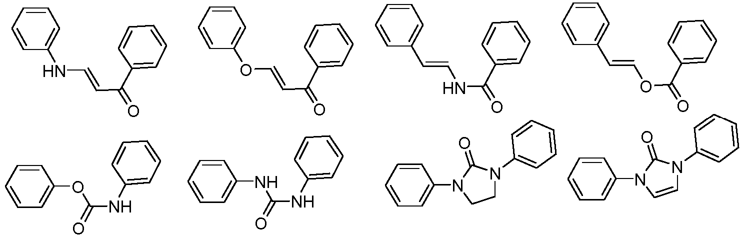 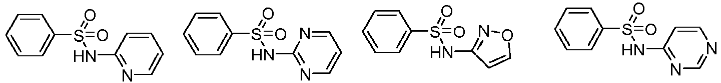 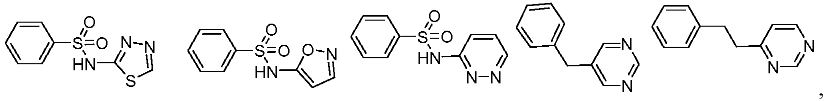 where L¹¹-W¹⁰ may be attached to either of the rings, preferably to one of the two phenyl rings or the sole phenyl ring, where each of the rings may be optionally substituted with one or more suitable substituents described herein, for example, each substituent may be independently selected from F; Cl; -OH; -NH₂; -SO₂NH₂; -SO₂NH(C₁₋₄ alkyl); -SO₂NH(C₁₋₄ alkanoyl); -COOH; 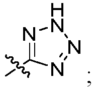 -C(O)(O-C₁₋₁₀ alkyl), -C(O)(O-C₂₋₁₀ alkenyl), -OC(O)NH₂; - OC(O)NH(C₁₋₄ alkyl)-; -O-(CO)-(C₁₋₄ alkyl); C₁₋₄ alkyl optionally substituted with 1-3 substituents independently selected from C₁₋₄ alkyl, C₁₋₄ alkoxy, -OH, -NH₂, and fluorine; C₂₋₆ alkenyl optionally substituted with 1-3 substituents independently selected from C₁₋₄ alkyl, C₁₋₄ alkoxy, -OH, -NH₂, and fluorine; C₂₋₆ alkynyl optionally substituted with 1-3 substituents independently selected from C₁₋₄ alkyl, C₁₋₄ alkoxy, -OH, -NH₂, and fluorine; C₃₋₆ cycloalkyl optionally substituted with 1-3 substituents independently selected from C₁₋₄ alkyl and fluorine; C₃₋₆ cycloalkoxy optionally substituted with 1-3 substituents independently selected from C₁₋₄ alkyl and fluorine; or C₁₋₄ alkoxy optionally substituted with 1-3 substituents independently selected from C₁₋₄ alkyl, C₁₋₄ alkoxy, -OH, -NH₂, and fluorine; optionally substituted C₃₋₆ cycloalkyl; optionally substituted 4-10 membered heterocyclyl; optionally substituted 5-10 membered heteroaryl; or optionally substituted C₆₋₁₀ aryl. For example, in some embodiments, the L¹¹-W¹⁰ is NH₂ or NH(C₁₋₄ alkanoyl), which is connected to one of the two phenyl rings or the sole phenyl ring, whereas the other ring is optionally substituted with 1 or 2 substituents selected from methyl and methoxy.

In some particular embodiments, the compound of the formula II has a formula according to a formula II-6 or II-7: 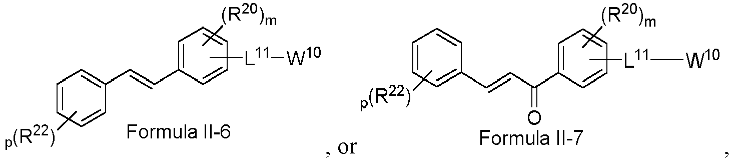
where: L¹¹, W¹⁰, R²⁰, and m are defined herein, see e.g., the formula II-3,
p is 0, 1, 2, 3, or 4,
R²² at each occurrence is independently halogen, -L^{11'}-W^{10'}, optionally substituted C₁₋₆ alkyl, optionally substituted C₂₋₆ alkenyl, optionally substituted C₂₋₆ alkynyl, optionally substituted C₁₋₆ alkoxy, optionally substituted C₃₋₆ cycloalkyl, optionally substituted C₃₋₆ cycloalkoxy, optionally substituted phenyl; optionally substituted 5 or 6 membered heteroaryl; or optionally substituted 4-7 membered heterocyclyl; or two adjacent R²² together with the atoms they are bound to form an optionally substituted cycloalkyl, heterocyclyl, aryl, or heteroaryl ring;
where L^{11'} and W^{10'} are defined herein, see e.g., the formula II-2, and -L^{11'}-W^{10'} at each occurrence is independently selected.
L¹¹ in the formula II (e.g., any of the sub-formulae, such as the formulae II-1 to II-7) is typically null, i.e., the W¹⁰ group is directly attached to Cy¹¹, as applicable. In some embodiments, L¹¹ in the formula II may also be C₁₋₄ alkylene, C₂₋₄ alkenylene, C₂₋₄ alkynylene or C₁₋₄ heteroalkylene. For example, the W¹⁰ group may be attached to Cy¹¹ through a methylene or vinyl group.
Various W¹⁰ groups are suitable for compounds of the formula II (e.g., the formulae II-1 to II-7). In preferred embodiments, the W¹⁰ group at each occurrence is independently -OH, - NH₂, -SO₂NH₂, -SO₂NH(C₁₋₄ alkyl); -SO₂NH(C₁₋₄ alkanoyl), -COOH, 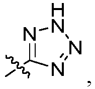 -C(O)(O-C₁₋₁₀ alkyl), -C(O)(O-C₂₋₁₀ alkenyl), -OC(O)NH₂, -OC(O)NH(C₁₋₄ alkyl)-, -O-(CO)-(C₁₋₄ alkyl), - O-(C₁₋₄ alkyl), where each of the C₁₋₄ alkyl is independently optionally substituted with 1-3 substituents independently selected from C₁₋₄ alkyl, C₁₋₄ alkoxy, -OH, -NH₂, and fluorine. In some embodiments, the W¹⁰ group in the formula II is -OH, -OMe, -NH₂, -SO₂NH₂, - SO₂NH(Acetyl), -COOH, 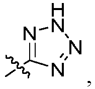 or -O-C(O)-CH_{3.}

As described herein, L^{11'}-W^{10'} may in some embodiments be selected as a substituent for Cy¹⁰ or Cy¹¹, such as for Ar¹⁰ or Ar¹¹. When applicable, L^{11'} in the formula II, including any of the sub-formulae described herein, such as the ormulae II-1 to II-7, at each occurrence may be independently null, i.e., the W^{10'} group is directly attached to Cy¹⁰ or Cy¹¹, such as for Ar¹⁰ or Ar¹¹, as applicable, or C₁₋₄ alkylene, C₂₋₄ alkenylene, C₂₋₄ alkynylene or C₁₋₄ heteroalkylene. For example, the W^{10'} group may be attached to Cy¹⁰ or Cy¹¹, such as for Ar¹⁰ or Ar¹¹, as applicable, through a methylene or vinyl group. When applicable, W^{10'} in the formula II, including any of the sub-formulae described herein, such as the formulae II-1 to II-7, at each occurrence may be independently -OH, -NH₂, -SO₂NH₂, -SO₂NH(C₁₋₄ alkyl), - SO₂NH(C₁₋₄ alkanoyl), -COOH, 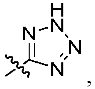 -C(O)(O-C₁₋₁₀ alkyl), -C(O)(O-C₂₋₁₀ alkenyl), - OC(O)NH₂, -OC(O)NH(C₁₋₄ alkyl)-, -O-(CO)-(C₁₋₄ alkyl), -O-(C₁₋₄ alkyl), where each of the C₁₋₄ alkyl is independently optionally substituted with 1-3 substituents independently selected from C₁₋₄ alkyl, C₁₋₄ alkoxy, -OH, -NH₂, and fluorine. In some embodiments, each instance of W^{10'} in the formula II, when applicable, may be -OH, -OMe, -NH₂, -SO₂NH₂, - SO₂NH(Acetyl), -COOH, or -O-C(O)-CH_{3.}

Various groups may be suitable for R²⁰, R²¹, and R²² in any of the applicable formula II (e.g., the formulae II-1 to II-7, as applicable). In some embodiments, each of R²⁰, R²¹, and R²² at each occurrence may be independently F; Cl; -OH; -NH₂; -SO₂NH₂; -SO₂NH(C₁₋₄ alkyl); - SO₂NH(C₁₋₄ alkanoyl); -COOH; 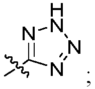 -C(O)(O-C₁₋₁₀ alkyl), -C(O)(O-C₂₋₁₀ alkenyl), - OC(O)NH₂; -OC(O)NH(C₁₋₄ alkyl)-; -O-(CO)-(C₁₋₄ alkyl); C₁₋₄ alkyl optionally substituted with 1-3 substituents independently selected from C₁₋₄ alkyl, C₁₋₄ alkoxy, -OH, -NH₂, and fluorine; C₂₋₆ alkenyl optionally substituted with 1-3 substituents independently selected from C₁₋₄ alkyl, C₁₋₄ alkoxy, -OH, -NH₂, and fluorine; C₂₋₆ alkynyl optionally substituted with 1-3 substituents independently selected from C₁₋₄ alkyl, C₁₋₄ alkoxy, -OH, -NH₂, and fluorine; C₃₋₆ cycloalkyl optionally substituted with 1-3 substituents independently selected from C₁₋₄ alkyl and fluorine; C₃₋₆ cycloalkoxy optionally substituted with 1-3 substituents independently selected from C₁₋₄ alkyl and fluorine; or C₁₋₄ alkoxy optionally substituted with 1-3 substituents independently selected from C₁₋₄ alkyl, C₁₋₄ alkoxy, -OH, -NH₂, and fluorine. In some embodiments, each of R²⁰, R²¹, and R²² at each occurrence may be independently F; Cl; -OH; -NH₂, -SO₂NH₂, -SO₂NH(C₁₋₄ alkyl), -SO₂NH(C₁₋₄ alkanoyl), -COOH; 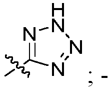 C(O)(O-C₁₋₁₀ alkyl), -C(O)(O-C₂₋₁₀ alkenyl), -OC(O)NH₂; -OC(O)NH(C₁₋₄ alkyl)-; -O-(CO)-(C₁₋₄ alkyl); -O-(C₁₋₆ alkyl); -O-(C₂₋₆ alkenyl); C₁₋₆ alkyl optionally substituted with 1-3 substituents independently selected from C₁₋₄ alkyl, C₁₋₆ alkoxy, -OH, -NH₂, and fluorine; or C₂₋₆ alkenyl optionally substituted with 1-3 substituents independently selected from C₁₋₄ alkyl, C₁₋₆ alkoxy, -OH, -NH₂, and fluorine. In some embodiments, each of R²⁰, R²¹, and R²² at each occurrence may be independently -OH, C₁₋₄ alkyl, C₂₋₆ alkenyl, or -O-(C₁₋₄ alkyl). In some embodiments, each of R²⁰, R²¹, and R²² at each occurrence may be independently -OH, -OMe, or 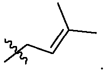 In some embodiments, one or more instances of R²⁰, one or more instances of R²¹, and/or one or more instances of R²² may be independently selected L^{11'}-W^{10'} as described herein.

Typically, m and p, as applicable, are 0, 1, 2, or 3; preferably, 1 or 2.

Typically, n, as applicable, is 0, 1, or 2; preferably, 0 or 1.

In some embodiments, the compound of the formula II may have a formula according to any of formulae II-8 to II-10: 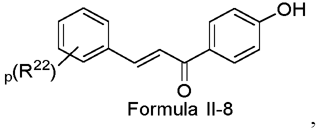 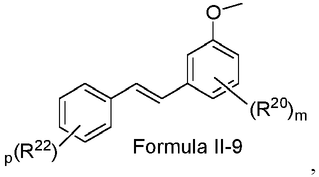 or 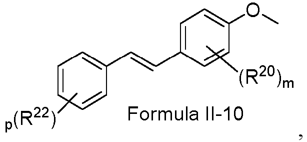 where R²⁰, R²², m, and p are defined herein. In some embodiments, m is 1 or 2, p is 1, 2, or 3. In some embodiments, each of R²⁰ and R²² at each occurrence is independently F; Cl; -OH; -NH₂, -SO₂NH₂, -SO₂NH(C₁₋₄ alkyl), -SO₂NH(C₁₋₄ alkanoyl), -COOH; 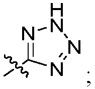 -C(O)(O-C₁₋₁₀ alkyl), -C(O)(O-C₂₋₁₀ alkenyl), -OC(O)NH₂; -OC(O)NH(C₁₋₄ alkyl)-; -O-(CO)-(C₁₋₄ alkyl); -O-(C₁₋₆ alkyl); -O-(C₂₋₆ alkenyl); C₁₋₆ alkyl optionally substituted with 1-3 substituents independently selected from C₁₋₄ alkyl, C₁₋₆ alkoxy, -OH, -NH₂, and fluorine; or C₂₋₆ alkenyl optionally substituted with 1-3 substituents independently selected from C₁₋₄ alkyl, C₁₋₆ alkoxy, -OH, -NH₂, and fluorine.

In some embodiments, the structural unit 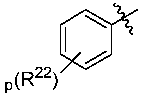 in any of the applicable formula II may be selected from

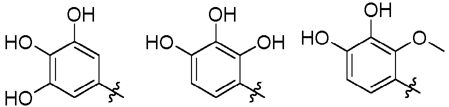

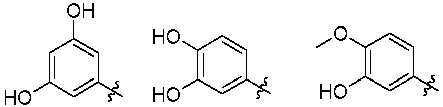

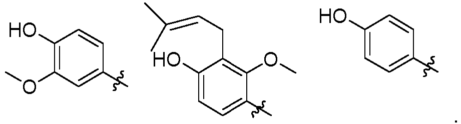

In some specific embodiments, the compound of the formula II may be: 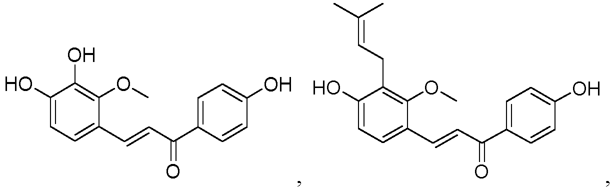 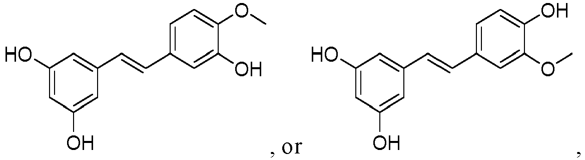 or a pharmaceutically acceptable salt or ester thereof.

In some embodiments, the compounds herein may be characterized by having a formula III, or a pharmaceutically acceptable salt or ester thereof:

Ar²⁰-L²⁰-W²⁰ Formula III

where Ar²⁰ is an optionally substituted aryl ring (e.g., C₆₋₁₀ aryl ring), or an optionally substituted heteroaryl ring (e.g., 5-10 membered heteroaryl ring);
L²⁰ is null, optionally substituted C₁₋₆ alkylene, optionally substituted C₁₋₆ heteroalkylene, optionally substituted C₂₋₆ alkenylene, optionally substituted C₂₋₆ alkynylene, an optionally substituted C₃₋₆ cycloalkylene, optionally substituted arylene, optionally substituted heteroarylene, or optionally substituted 4-7 membered heterocyclylene,
W²⁰ is -OR¹; -COR²; -COOR^{1a}; -OCOOR^{1a}; -NR³R⁴; -CONR^{3a}R^{4a}; -OCONR3^{b}R^{4b}; - SO₂NR^{3c}R^{4c}; -OSO₂NR^{3d}R^{4d}; -SR⁵; -SO₂R^{5a}; -OCOR^{2a}; -OSO₂R^{5a}; or 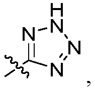 where:
   R¹ and R^{1a} are each independently hydrogen, optionally substituted alkyl, optionally substituted alkenyl, optionally substituted alkynyl, optionally substituted cycloalkyl, optionally substituted aryl, optionally substituted heteroaryl, or optionally substituted heterocyclyl;
   R³ and R⁴ are each independently hydrogen, -COR^{2b}, -SO₂R^{5b}, optionally substituted C₁₋₆ alkyl, optionally substituted C₂₋₆ alkenyl, optionally substituted C₂₋₆ alkynyl, optionally substituted C₃₋₆ cycloalkyl, optionally substituted phenyl, optionally substituted 5 or 6 membered heteroaryl, or optionally substituted 4-7 membered heterocyclyl, or R³ and R⁴ together with the atoms they are bound to form optionally substituted 4-7 membered heterocyclyl;
   R², R^{2a}, R^{2b}, R⁵, R^{5a}, and R^{5b} are each independently hydrogen, -OH, -NR^{3e}R^{4e}, optionally substituted C₁₋₆ alkyl, optionally substituted C₂₋₆ alkenyl, optionally substituted C₂₋₆ alkynyl, optionally substituted C₁₋₆ alkoxy, optionally substituted C₃₋₆ cycloalkyl, optionally substituted C₃₋₆ cycloalkoxy, optionally substituted phenyl; optionally substituted 5 or 6 membered heteroaryl; or optionally substituted 4-7 membered heterocyclyl; and
   R^{3a}, R^{3b}, R^{3c}, R^{3a}, R^{3e}, R^{4a}, R^{4b}, R^{4c}, R^{4d}, and R^{4e} are each independently hydrogen, optionally substituted C₁₋₆ alkyl, optionally substituted C₂₋₆ alkenyl, optionally substituted C₂₋₆ alkynyl, optionally substituted C₁₋₆ alkoxy, optionally substituted C₃₋₆ cycloalkyl, optionally substituted C₃₋₆ cycloalkoxy, optionally substituted phenyl; optionally substituted 5 or 6 membered heteroaryl; or optionally substituted 4-7 membered heterocyclyl; or R^{3a} and R^{4a}, R^{3b} and R^{4b}, R^{3c} and R^{4c}, R^{3d} and R^{4d}, or R^{3e} and R^{4e}, together with the atoms they are bound to form optionally substituted 4-7 membered heterocyclyl.

In some embodiments, Ar²⁰ in the formula III is an optionally substituted phenyl ring or an optionally substituted 5 or 6 membered heteroaryl ring. For example, in some embodiments, Ar²⁰ in the formula III may be an optionally substituted phenyl ring, an optionally substituted thienyl ring, an optionally substituted furanyl ring, an optionally substituted pyridyl ring, or an optionally substituted pyrimidinyl ring. In some embodiments, Ar²⁰ in the formula III may also be an optionally substituted bicyclic aryl or bicyclic heteroaryl ring, each of which is optionally substituted. In such embodiments, L²⁰-W²⁰ may be attached to either of the bicyclic rings.

In some embodiments, Ar²⁰ in the formula III may be an optionally substituted phenyl ring, where two adjacent substituents together with the carbon they are attached to form an optionally substituted cycloalkyl, heterocyclyl, aryl, or heteroaryl ring.

For example, in some embodiments, Ar²⁰ in the formula III may be a benzofused bicyclic aryl or heteroaryl ring. For example, in some embodiments, Ar²⁰ in the formula III may have the following structure: 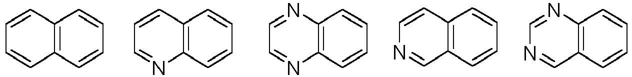 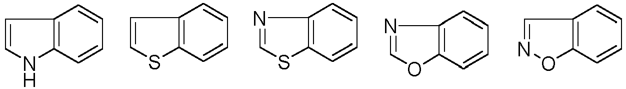 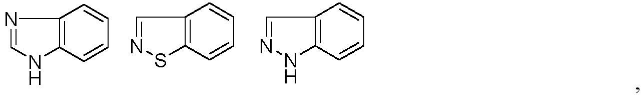 where -L²⁰-W²⁰ may be attached to either of the two rings, where either or both of the rings may be optionally substituted.

In some embodiments, the compound of the formula III may have a formula III-1, III-2, or III-3: 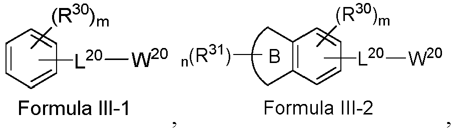 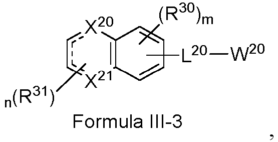
where L²⁰ and W²⁰ are defined herein,
m is 0, 1, 2, or 3; n is 0, 1, 2, or 3;
each of R³⁰ and R³¹ at each occurrence is independently halogen, -L^{20'}-W^{20'}, optionally substituted C₁₋₆ alkyl, optionally substituted C₂₋₆ alkenyl, optionally substituted C₂₋₆ alkynyl, optionally substituted C₁₋₆ alkoxy, optionally substituted C₃₋₆ cycloalkyl, optionally substituted C₃₋₆ cycloalkoxy, optionally substituted phenyl; optionally substituted 5 or 6 membered heteroaryl; or optionally substituted 4-7 membered heterocyclyl; where -L^{20'}-W^{20'} at each occurrence is independently selected; where L^{20'} at each occurrence is independently null, optionally substituted C₁₋₆ alkylene, optionally substituted C₁₋₆ heteroalkylene, optionally substituted C₂₋₆ alkenylene, optionally substituted C₂₋₆ alkynylene, optionally substituted C₃₋₆ cycloalkylene, optionally substituted arylene, optionally substituted heteroarylene, or optionally substituted 4-7 membered heterocyclylene; and W^{20'} at each occurrence is independently -OR¹; -COR²; -COOR^{1a}; -OCOOR^{1a}; -NR³R⁴; -CONR^{3a}R^{4a}; -OCONR3^{b}R^{4b}; - SO₂NR^{3c}R^{4c}, -OSO₂NR^{3d}R^{4d}; -SR⁵; -SO₂R^{5a}; -OCOR^{2a}; -OSO₂R^{5a} or 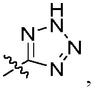 where R¹, R^{1a}, R², R^{2a}, R^{2b}, R³, R⁴, R^{3a}, R^{3b}, R^{3c}, R^{3d}, R^{3e}, R^{4a}, R^{4b}, R^{4c}, R^{4d}, R^{4e}, R⁵, R^{5a}, and R^{5b} are defined herein, see e.g., the formula III,
a ring B is a 4-7 membered cycloalkyl ring, 4-7 membered heterocyclic ring, phenyl ring, or 5 or 6 membered heteroaryl ring, each of which is optionally substituted with 1-3 independently selected R³¹;
X²⁰ and X²¹ are each independently null, -O-, -C(O)-, -S-, -NR^{100a}-, -S(O)-, -SO₂-, or - CR^{101a}R^{102a}-, as valence permits;
where R^{100a} is lone pair (as applicable), hydrogen, COR^{2c}, -SO₂R^{5c}, optionally substituted C₁₋₆ alkyl, optionally substituted C₂₋₆ alkenyl, optionally substituted C₂₋₆ alkynyl, optionally substituted C₃₋₆ cycloalkyl, optionally substituted phenyl, optionally substituted 5 or 6 membered heteroaryl, or optionally substituted 4-7 membered heterocyclyl; or R^{100a} and one of R³⁰ or R³¹, together with the atoms they are bound to, form an optionally substituted heterocyclic or heteroaryl ring, e.g., optionally substituted 5 or 6 membered heteroaryl, or optionally substituted 4-7 membered heterocyclyl;
R^{101a} and R^{102a}, when present, are each independently hydrogen, -OH, halogen; optionally substituted C₁₋₆ alkyl, optionally substituted C₂₋₆ alkenyl, optionally substituted C₂₋₆ alkynyl, optionally substituted C₃₋₆ cycloalkyl, optionally substituted C₁₋₆ alkoxy, optionally substituted C₃₋₆ cycloalkoxy, optionally substituted amino group, optionally substituted phenyl, optionally substituted 5 or 6 membered heteroaryl, or optionally substituted 4-7 membered heterocyclyl, or R^{101a} and R^{102a}, together with the atoms they are bound to form an optionally substituted 3-7 membered cycloalkyl or heterocyclyl ring; or one of R^{101a} and R^{102a} forms an optionally substituted cycloalkyl or heterocyclyl ring together with an R³⁰ or R³¹ group; and
R^{2c} and R^{5c} are each independently hydrogen, optionally substituted C₁₋₆ alkyl, optionally substituted C₂₋₆ alkenyl, optionally substituted C₂₋₆ alkynyl, optionally substituted C₁₋₆ alkoxy, optionally substituted C₃₋₆ cycloalkyl, optionally substituted C₃₋₆ cycloalkoxy, optionally substituted phenyl; optionally substituted 5 or 6 membered heteroaryl; or optionally substituted 4-7 membered heterocyclyl;
or two adjacent R³⁰ or two adjacent R³¹, or R³⁰ or R³¹ and X²⁰ or X²¹, together with the atoms they are bound to form an optionally substituted cycloalkyl, heterocyclyl, aryl, or heteroaryl ring.

When X²⁰ or X²¹ forms a double bond with one of the ring carbons, it cannot be CR^{101a}R^{102a} with both R^{101a} and R^{102a} present, as the valence of the carbon will exceed 4. In such cases, it should be understood that one of R^{101a} and R^{102a} is absent and X²⁰ or X²¹ is CR^{101a} or CR^{102a} as defined herein. When X²⁰ or X²¹ forms a double bond with one of the ring carbons, it may be NR^{100a} with R^{100a} typically being a lone pair.

It should be noted that each instance of the structural unit -L^{20'}-W^{20'} and -L²⁰-W²⁰ is independently selected and may be the same or different.

In some embodiments, the compound of the formula III may have a structure of any of the following: 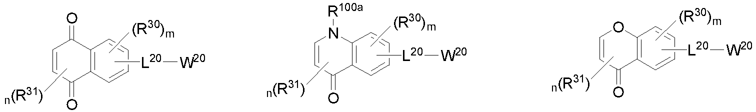 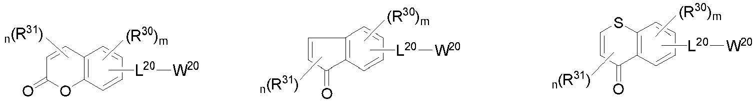 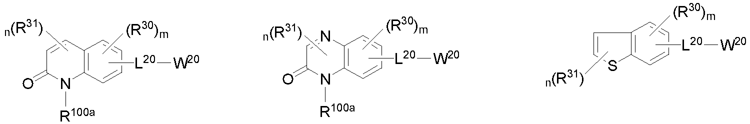 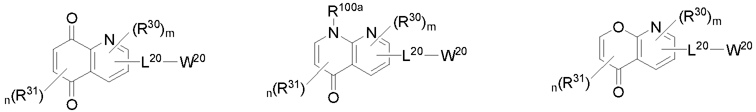 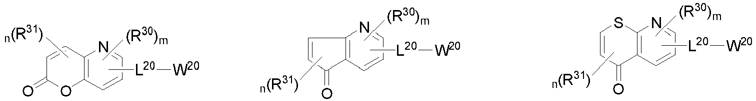 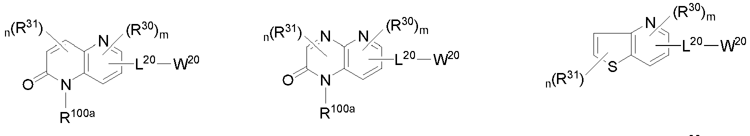 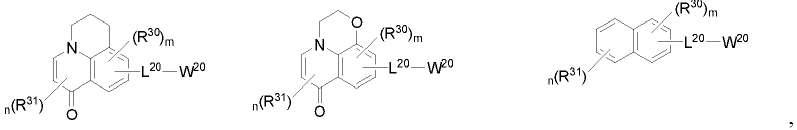 where: R³⁰, m, R³¹, n, R^{100a}, L²⁰, and W²⁰ are defined herein, see e.g., the formula III and sub-formulae herein, such as the formulae III-1 to III-3, where for the tricyclic structures, the piperidine ring or the morpholine ring may be optionally substituted.

L²⁰ in the formula III (e.g., any of the sub-formulae such as the formulae III-1 to III-3) is typically null, i.e., the W²⁰ group is directly attached to Ar²⁰. In some embodiments, L²⁰ in the formula III may also be C₁₋₄ alkylene, C₂₋₄ alkenylene, C₂₋₄ alkynylene or C₁₋₄ heteroalkylene. For example, the W²⁰ group may be attached to Ar²⁰, through a methylene or vinyl group.

Various W²⁰ groups are suitable for compounds of the formula III (e.g., any of the sub-formulae such as the formulae III-1 to III-3). In preferred embodiments, W²⁰ in the formula III may be -OH, -COOH, 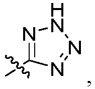 -C(O)(O-C₁₋₁₀ alkyl), -C(O)(O-C₂₋₁₀ alkenyl),-OC(O)NH₂, -NH₂, -SO₂NH₂, -SO₂NH(C₁₋₄ alkyl); -SO₂NH(C₁₋₄ alkanoyl), -OC(O)NH(C₁₋₄ alkyl)-, -O-(CO)-(C₁₋₄ alkyl), -O-(C₁₋₄ alkyl), where each of the C₁₋₄ alkyl is independently optionally substituted with 1-3 substituents independently selected from C₁₋₄ alkyl, C₁₋₄ alkoxy, -OH, - NH₂, and fluorine. In some embodiments, the W²⁰ group in the formula III (e.g., any of the sub-formulae such as the formulae III-1 to III-3) is -OH, -NH₂, -SO₂NH₂, -SO₂NH(Acetyl), 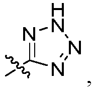 -C(O)-(O-C₈ alkyl), -COOH, or -O-C(O)-CH_{3.}

As described herein, L^{20'}-W^{20'} may in some embodiments be selected as a substituent for Ar²⁰. When applicable, L^{20'} in the formula III, including any of the sub-formulae described herein, such as the formulae III-1 to III-3, at each occurrence may be independently null, i.e., the W^{20'} group is directly attached to Ar²⁰, as applicable, or C₁₋₄ alkylene, C₂₋₄ alkenylene, C₂₋₄ alkynylene or C₁₋₄ heteroalkylene. For example, the W^{20'} group may be attached to Ar²⁰, as applicable, through a methylene or vinyl group. When applicable, W^{20'} in the formula III, including any of the sub-formulae described herein, such as the formulae III-1 to III-3, at each occurrence may be independently -OH, -COOH, 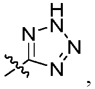 -C(O)(O-C₁₋₁₀ alkyl), - C(O)(O-C₂₋₁₀ alkenyl),-OC(O)NH₂, -NH₂, -SO₂NH₂, -SO₂NH(C₁₋₄ alkyl); -SO₂NH(C₁₋₄ alkanoyl), -OC(O)NH(C₁₋₄ alkyl)-, -O-(CO)-(C₁₋₄ alkyl), -O-(C₁₋₄ alkyl), where each of the C₁₋₄ alkyl is independently optionally substituted with 1-3 substituents independently selected from C₁₋₄ alkyl, C₁₋₄ alkoxy, -OH, -NH₂, and fluorine. In some embodiments, each instance of W^{20'} in the formula III, when applicable, may be -OH, -NH₂, -SO₂NH₂, -SO₂NH(Acetyl), 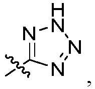 -COOH, -C(O)(O-Cs alkyl) or -O-C(O)-CH_{3.}

Various groups may be suitable for R³⁰ and R³¹ in any of the applicable formula III (e.g., any of the sub-formulae such as the formulae III-1 to III-3). In some embodiments, each of R³⁰ and R³¹ at each occurrence may be independently F; Cl; -OH; -COOH; -OC(O)NH₂; - OC(O)NH(C₁₋₄ alkyl)-; -O-(CO)-(C₁₋₄ alkyl); C₁₋₄ alkyl optionally substituted with 1-3 substituents independently selected from C₁₋₄ alkyl, C₁₋₄ alkoxy, -OH, -NH₂, and fluorine; C₂₋₆ alkenyl optionally substituted with 1-3 substituents independently selected from C₁₋₄ alkyl, C₁₋₄ alkoxy, -OH, -NH₂, and fluorine; C₂₋₆ alkynyl optionally substituted with 1-3 substituents independently selected from C₁₋₄ alkyl, C₁₋₄ alkoxy, -OH, -NH₂, and fluorine; C₃₋₆ cycloalkyl optionally substituted with 1-3 substituents independently selected from C₁₋₄ alkyl and fluorine; C₃₋₆ cycloalkoxy optionally substituted with 1-3 substituents independently selected from C₁₋₄ alkyl and fluorine; or C₁₋₄ alkoxy optionally substituted with 1-3 substituents independently selected from C₁₋₄ alkyl, C₁₋₄ alkoxy, -OH, -NH₂, and fluorine. In some embodiments, each of R³⁰ and R³¹ at each occurrence may be independently -OH, C₂₋₆ alkenyl, -O-(C₁₋₄ alkyl), -COOH, or -C(O)(O-C₁₋₁₀ alkyl). In some embodiments, each of R³⁰ and R³¹ at each occurrence may be -OH or -OMe. In some embodiments, one or more instances of R³⁰ and/or one or more instances of R³¹ may be independently selected from L^{20'}-W^{20'} as described herein.

Typically, m is 0, 1, 2, or 3; preferably, 2 or 3. Typically, n is 1, 2 or 3.

In some embodiments, the present disclosure further provides the following compound, 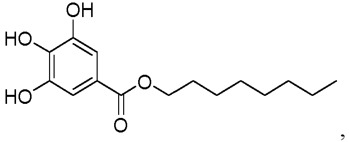 or a pharmaceutically acceptable salt or ester thereof.

In some embodiments, the present disclosure further provides the following compound, 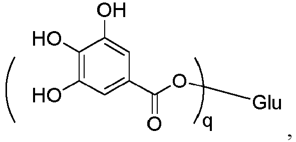 or a pharmaceutically acceptable salt or ester thereof, where q is 1, 2, 3, 4, or 5, and Glu is a residue of glucose. In some specific embodiments, the present disclosure further provides 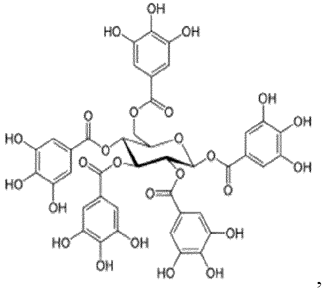 or a pharmaceutically acceptable salt or ester thereof.

In some embodiments, the compounds herein may also be an alkaloid having antibacterial activity. As shown herein, certain indole alkaloids, such as *Vinca* alkaloids, tabersonine, vindoline, vinblastine, vincristine, etc., are shown to be effective in killing the microorganisms such as *B. megaterium.* In some embodiments, the compounds herein are characterized by a formula IV-1 or IV-2, which are tabersonine or vindoline and derivatives: 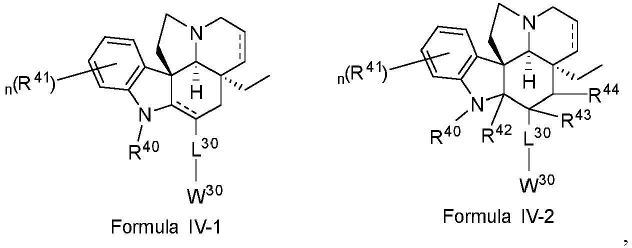 where:
R⁴⁰ is hydrogen; -COR²; -COOR^{1a}; -SO₂R^{5a}; optionally substituted alkyl, optionally substituted alkenyl, optionally substituted alkynyl, optionally substituted cycloalkyl, optionally substituted aryl, optionally substituted heteroaryl, or optionally substituted heterocyclyl;
R⁴¹ is -OR¹; -OCOOR^{1a}; -OCONR3^{b}R^{4b}; -OCOR^{2a}; or -OSO₂R^{5a}; n is 0 or 1;
R⁴², R⁴³, and R⁴⁴ are each independently hydrogen, -OR¹, OCOR^{2a}; or -OSO₂R^{5a} ;
L³⁰ is null or methylene,
W³⁰ is -OR¹; -COR²; -COOR^{1a}; -OCOOR^{1a}; -NR³R⁴; -CONR^{3a}R^{4a}; -OCONR3^{b}R^{4b}; - OSO₂NR^{3d}R^{4d}; -OCOR^{2a}; or -OSO₂R^{5a},
where:
   R¹ and R^{1a} are each independently hydrogen, optionally substituted alkyl, optionally substituted alkenyl, optionally substituted alkynyl, optionally substituted cycloalkyl, optionally substituted aryl, optionally substituted heteroaryl, or optionally substituted heterocyclyl;
   R³ and R⁴ are each independently hydrogen, -COR^{2b}, -SO₂R^{5b}, optionally substituted C₁₋₆ alkyl, optionally substituted C₂₋₆ alkenyl, optionally substituted C₂₋₆ alkynyl, optionally substituted C₃₋₆ cycloalkyl, optionally substituted phenyl, optionally substituted 5 or 6 membered heteroaryl, or optionally substituted 4-7 membered heterocyclyl, or R³ and R⁴ together with the atoms they are bound to form optionally substituted 4-7 membered heterocyclyl;
   R², R^{2a}, R^{2b}, R⁵, R^{5a}, and R^{5b} are each independently hydrogen, -OH, -NR^{3e}R^{4e}, optionally substituted C₁₋₆ alkyl, optionally substituted C₂₋₆ alkenyl, optionally substituted C₂₋₆ alkynyl, optionally substituted C₁₋₆ alkoxy, optionally substituted C₃₋₆ cycloalkyl, optionally substituted C₃₋₆ cycloalkoxy, optionally substituted phenyl; optionally substituted 5 or 6 membered heteroaryl; or optionally substituted 4-7 membered heterocyclyl; and
   R^{3a}, R^{3b}, R^{3c}, R^{3a}, R^{3e}, R^{4a}, R^{4b}, R^{4c}, R^{4d}, and R^{4e} are each independently hydrogen, optionally substituted C₁₋₆ alkyl, optionally substituted C₂₋₆ alkenyl, optionally substituted C₂₋₆ alkynyl, optionally substituted C₁₋₆ alkoxy, optionally substituted C₃₋₆ cycloalkyl, optionally substituted C₃₋₆ cycloalkoxy, optionally substituted phenyl; optionally substituted 5 or 6 membered heteroaryl; or optionally substituted 4-7 membered heterocyclyl; or R^{3a} and R^{4a}, R^{3b} and R^{4b}, R^{3c} and R^{4c}, R^{3d} and R^{4d}, or R^{3e} and R^{4e}, together with the atoms they are bound to form optionally substituted 4-7 membered heterocyclyl.

In some embodiments, the compound of the formula IV-1 or IV-2 has a formula according to one of formulae IV-3 to IV-6: 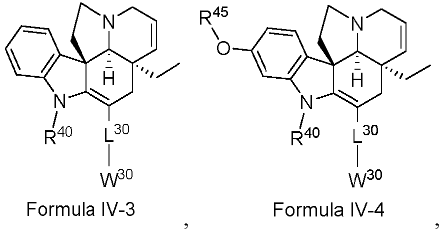 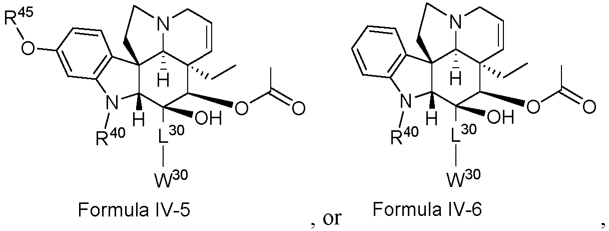 where R⁴⁵ is hydrogen or methyl.

In some embodiments, R⁴⁰ in any of the formulae IV-1 to IV-6 may be hydrogen, C₁₋₄ alkyl, or C₁₋₄ alkanoyl.

L³⁰ in the formulae IV-1 to IV-6 is typically null. However, in some embodiments, L³⁰ in the formulae IV-1 to IV-6 may also be CH₂.

W³⁰ in the formulae IV-1 to IV-6 is typically a carboxylic acid derivative, an amine derivative or an alcohol derivative, which may be useful for the compositions and methods herein. The naturally occurring indole alkaloid tabersonine contains a CO₂Me group as W³⁰, with L³⁰ being null. The CO₂Me group may be transformed into the corresponding acid, amide etc. via routine transformations, or it may be reduced or transformed into an amine through rearrangement such as Curtius rearrangement. In some embodiments, W³⁰ in the formulae IV-1 to IV-6 may be -OH, -NH₂, -OSO₂NH₂, -COOH, -C(O)(O-C₁₋₁₀ alkyl), - C(O)(O-C₂₋₁₀ alkenyl), -OC(O)NH₂, -OC(O)NH(C₁₋₄ alkyl)-, -O-(CO)-(C₁₋₄ alkyl), -O-(C₁₋₄ alkyl), where each of the C₁₋₄ alkyl is independently optionally substituted with 1-3 substituents independently selected from C₁₋₄ alkyl, C₁₋₄ alkoxy, -OH, -NH₂, and fluorine. In some embodiments, W³⁰ in the formulae IV-1 to IV-6 may be -OH, -NH₂, -OSO₂NH₂, -C(O)-(O-C₈ alkyl), -COOH, or -OC(O)NH₂.

In some specific embodiments, the compound may have the following structure:

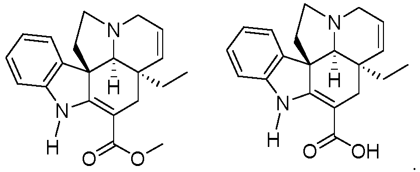

In some embodiments, the compounds herein may also be a glycoside having antibacterial activity, or a pharmaceutically acceptable salt or ester thereof. As shown herein, certain glycosides, such as ginsenosides, and gallic acid glycosides, are shown to be effective in killing the microorganisms such as *B. megaterium.* Other useful glycosides include any of those known in the art to have antibacterial activity, which may for example, include glycosides characterized by their corresponding aglyone being a phenolic compound, a flavonoid, a coumarin, a benzoic acid, or a sterol. Typically, the glycoside is a glucoside, although other glycosides may also be useful. In some embodiments, the glycosides may be characterized as amphiphilic, which can destroy biological membranes and confer antimicrobial activity to the glycosides. In some embodiments, the glycosides may also be characterized as a saponin, which for example, include various plant derived glycosides that can act as "surfactants" and can help to kill bacteria.

In some embodiments, the glycosides herein may be characterized by a formula V: 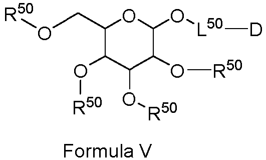 where each R⁵⁰ is independently hydrogen, -L⁵⁰-D, an oxygen protecting group, or a sugar residue;
L⁵⁰ is null or -C(O)-;
D is optionally substituted aryl (e.g., C₆₋₁₀ aryl), optionally substituted heteroaryl (e.g., 5 to 14 membered heteroaryl), an optionally substituted fused ring including two or more rings independently selected from aryl, heteroaryl, cycloalkyl and heterocyclyl (e.g., 8-14 membered, e.g., benzofused cycloalkyl/heterocyclyl, pyridofused cycloalkyl/heterocyclyl), or a steroid residue having a formula V-A: 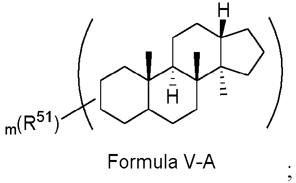
   where 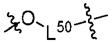 may be connected to the formula V-A via the steroid backbone or any of the R⁵¹ group(s), as valence permits,
   where R⁵¹ at each occurrence is independently optionally substituted alkyl, optionally substituted alkenyl, optionally substituted alkynyl, -OH optionally substituted with an oxygen protecting group, oxo, halogen, optionally substituted cycloalkyl, optionally substituted alkoxy, optionally substituted cycloalkoxy, optionally substituted amino group, optionally substituted phenyl, optionally substituted heteroaryl, or optionally substituted heterocyclyl, or two R⁵¹ groups together with the atoms they are bound to form an optionally substituted cycloalkyl, heterocyclyl, aryl, or heteroaryl ring;
   m is an integer of 1-8; and
   where -L⁵⁰-D at each occurrence is independently selected.

In some embodiments, each R⁵⁰ is hydrogen.

In some embodiments, one to four R⁵⁰ may be -L⁵⁰-D which are independently selected. When two or more -L⁵⁰-D units are linked to the pyranose unit in the formula V, they are preferably the same. In some embodiments, one or more (e.g., 1 or 2) R⁵⁰ may be a sugar residue which is connected to the remainder of the formula V via a glycoside bond. In some embodiments, the sugar residue is a glucose residue or a rhamnose residue.

L⁵⁰ in the formula V may be null or a carbonyl group, namely -C(O)-, depending on whether the linking group is a phenolic -OH or a COOH group from a benzoic acid or a heteraryl counterpart.

Various residues may be used as D, which are typically residues from a phenolic compound, a coumarin, a flavonoid, or a sterol, which in some embodiments may have antibacterial activity without the glycoside unit.

In some embodiments, D may be an optionally substituted ring selected from: 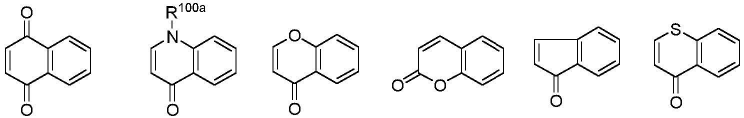 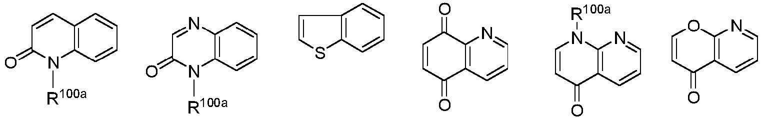 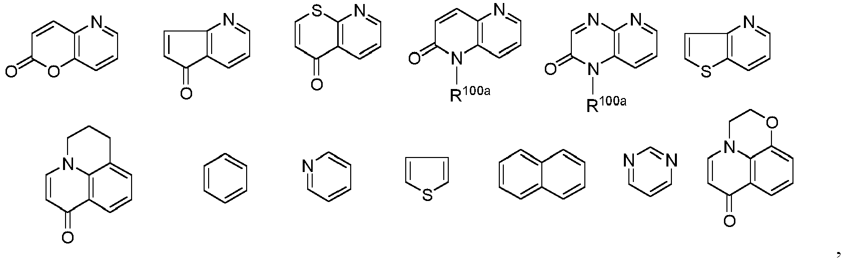 where
R^{100a} is lone pair (as applicable), hydrogen, nitrogen protecting group, optionally substituted C₁₋₆ alkyl, optionally substituted C₂₋₆ alkenyl, optionally substituted C₂₋₆ alkynyl, optionally substituted C₃₋₆ cycloalkyl, optionally substituted phenyl, optionally substituted 5 or 6 membered heteroaryl, or optionally substituted 4-7 membered heterocyclyl; or R^{100a} forms an optionally substituted heterocyclic or heteroaryl ring with the phenyl or pyridyl ring;
where 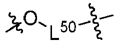 may be connected to D via any of the available positions, and
each of the ring systems of D is optionally substituted with 1-5 (e.g., 1, 2, or 3) substituents each independently selected from -OH; -COOH; -C(O)(O-C₁₋₁₀ alkyl); -C(O)(O-C₂₋₁₀ alkenyl); -OC(O)NH₂; -OC(O)NH(C₁₋₄ alkyl)-; -O-(CO)-(C₁₋₄ alkyl); -NH₂; -SO₂NH₂; - SO₂NH(C₁₋₄ alkyl); -SO₂NH(C₁₋₄ alkanoyl); halogen; optionally substituted C₁₋₆ alkyl; optionally substituted C₂₋₆ alkenyl; optionally substituted C₂₋₆ alkynyl; optionally substituted C₃₋₆ cycloalkyl; optionally substituted C₁₋₆ alkoxy; optionally substituted C₃₋₆ cycloalkoxy; optionally substituted amino group; optionally substituted phenyl; optionally substituted 5 or 6 membered heteroaryl; or optionally substituted 4-7 membered heterocyclyl.

In some embodiments, each of the ring systems of D as shown above may be optionally substituted with 1-5 substituents each independently selected from F; Cl; -OH; -COOH; - C(O)(O-C₁₋₁₀ alkyl); -C(O)(O-C₂₋₁₀ alkenyl); -OC(O)NH₂; -OC(O)NH(C₁₋₄ alkyl)-; -O-(CO)-(C₁₋₄ alkyl); -NH₂; -SO₂NH₂; -SO₂NH(C₁₋₄ alkyl); -SO₂NH(C₁₋₄ alkanoyl); C₁₋₄ alkyl optionally substituted with 1-3 substituents independently selected from C₁₋₄ alkyl; C₁₋₄ alkoxy, -OH, -NH₂, and fluorine; C₂₋₆ alkenyl optionally substituted with 1-3 substituents independently selected from C₁₋₄ alkyl, C₁₋₄ alkoxy, -OH, -NH₂, and fluorine; C₂₋₆ alkynyl optionally substituted with 1-3 substituents independently selected from C₁₋₄ alkyl, C₁₋₄ alkoxy, -OH, -NH₂, and fluorine; C₃₋₆ cycloalkyl optionally substituted with 1-3 substituents independently selected from C₁₋₄ alkyl and fluorine; C₃₋₆ cycloalkoxy optionally substituted with 1-3 substituents independently selected from C₁₋₄ alkyl and fluorine; or C₁₋₄ alkoxy optionally substituted with 1-3 substituents independently selected from C₁₋₄ alkyl, C₁₋₄ alkoxy, -OH, -NH₂, and fluorine.

In some embodiments, D may be selected from: 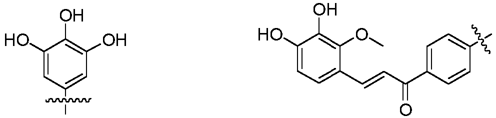 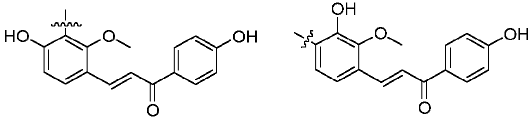 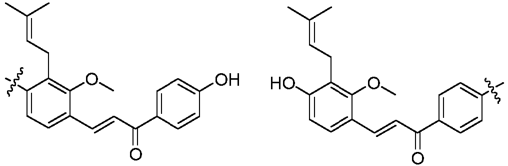 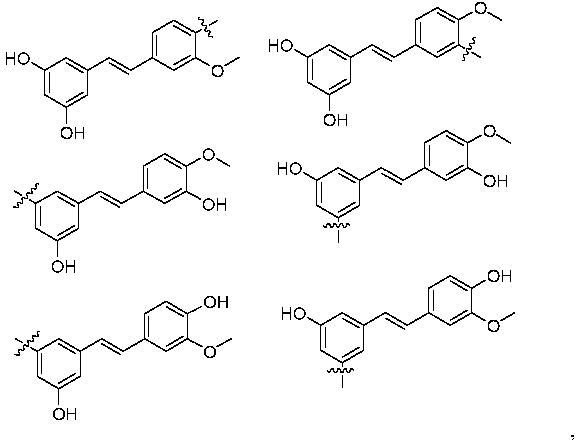 where each of the phenolic OH groups is optionally connected to a sugar (such as glucose) via a glycoside bond.

In some embodiments, D is derived from a sterol. For example, in some embodiments, D is 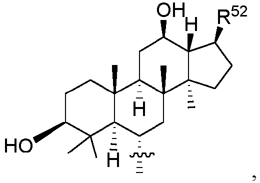
where R⁵² is optionally substituted alkyl or optionally substituted alkenyl,
where each of the remaining -OH groups in D is optionally connected to a sugar via a glycoside bond.

Preferably, R⁵² may be

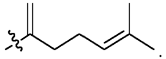

In any of the embodiments described above, the glycoside may have a formula V-1 or V-2:

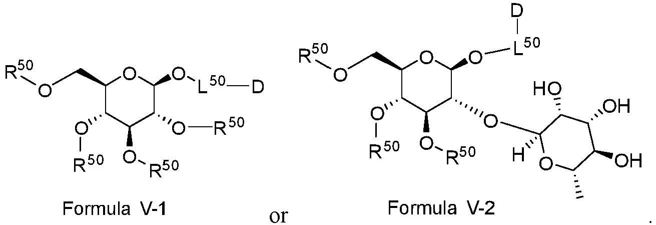

In some embodiments, the glycoside may be a compound selected from:

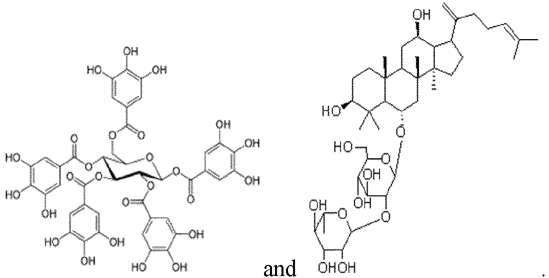

In some embodiments, the compounds herein may be any one or more of compounds selected from benzoid acid, benzyl alcohol, coumarins, catechols, polyphenols, chalconoids (including licochalcones), etc., stilbenes such as resveratrol, isoresveratrol, etc., phenolic acids, such as p-hydroxbenzoic acid, 2,4-dihydroxbenzoid acid, protocatechuic acid, gallic acid, vanillic acid, syringic acid, cinnamic acid, coumaric acids, caffeic acids, ferulic acids, chlorogenic acid, sinapic acids etc., flavonoids such as catechin, narigenin, quercetin, rutin, chrysin, etc., tannins, such as ellagic acid, and pharmaceutically acceptable salts or esters thereof and glycosides thereof.

In an implementation of the present disclosure, the inhibitor or microbicid for the microorganism is compounds 1-8, having the following chemical structures:

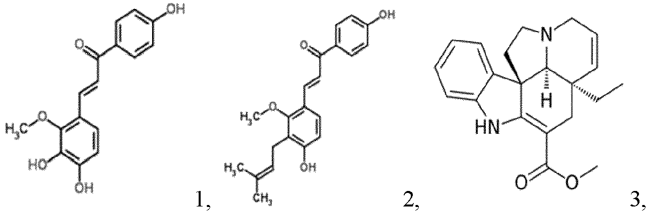

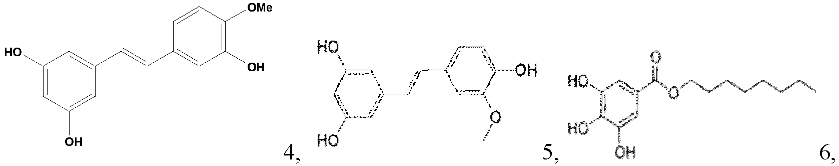

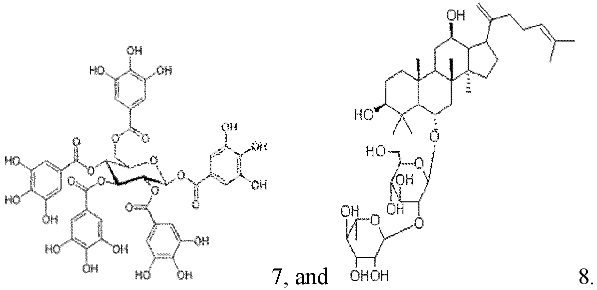

Preferably, the inhibitor or microbicid for the microorganism in the present disclosure is a natural plant or a natural plant extract or a combination of the natural plant and the natural plant extract, preferably, the natural plant is selected from one or a combination of two or more of radix paeoniae alba, fructus forsythiae, fructus aurantii, rhizoma rehmanniae, dried tangerine peel, pseudo-ginseng, turtle shells, roots of kirilow rhodiola, dried rehmannia roots, coix seeds, semen cassiae and liquorice.

In a specific implementation of the present disclosure, the inhibitor or microbicid for the microorganism is a composition including turtle shells, roots of kirilow rhodiola, dried rehmannia roots, coix seeds, radix paeoniae alba, fructus forsythiae, semen cassiae and liquorice, preferably, the composition includes, by weight, 5-15 parts the turtle shells, 10-30 parts the roots of kirilow rhodiola, 5-15 parts the dried rehmannia roots, 5-15 parts the coix seeds, 8-20 parts the radix paeoniae alba, 5-15 parts the fructus forsythiae, 5-15 parts the semen cassiae and 5-15 parts the liquorice, more preferably, the composition includes 10 parts the turtle shells, 20 parts the roots of kirilow rhodiola, 10 parts the dried rehmannia roots, 10 parts the coix seeds, 12 parts the radix paeoniae alba, 10 parts the fructus forsythiae, 10 parts the semen cassiae and 9 parts the liquorice.

Preferably, administering to the eyes of the patient includes administering to aqueous humor, a suspensory ligament, a ciliary body, a ciliary body and ciliary muscle in an anterior chamber of each eye of the patient as well as to vitreous humor, a retina, a choroid membrane, an optic nerve, a crystalline lens or an iris in a posterior chamber of each eye of the patient.

Preferably, administering to the gastrointestinal tract of the patient includes administering to a stomach, a jejunum, an ileum, a cecum, a colon or a rectum of the patient.

A further solution of the present disclosure provides use of an inhibitor or microbicid for a microorganism in preparation of a drug for treating retinal degeneration.

Preferably, the retinal degeneration is progressive retinal degeneration; more preferably, the retinal degeneration is inherited retinal degeneration; more preferably, the inherited retinal degeneration includes Leber congenital amaurosis (LCA), retinitis pigmentosa (RP), early-onset rod-cone cytodystrophy, rod-rod dystrophy, congenital stationary night blindness and colour blindness and Stargardt disease.

Preferably, the microorganism is one or a combination of two or more of bacteria, archaebacteria, protists, fungi or viruses. The microorganism is the bacteria, and the bacteria are selected from: one or two or more of *Anearostipes, Bifidobacterium, Megamonas, Nitrosomonas, Oscillibacter, Tatumella*, *Thiobacillussp.,Clostridium, Acinetobacter, Streptococcus, Mannheimia*, *Fibrobacter, Prevotella, Campylobacter, Actinomyces, Hymenobacter*, *Escherichia, Tissierella*, *Klebsiella, Porphyromonas, Azospira*, *Aquimarina*, *Achromobacter, Acidithiobacillus, Burkholderia, Marinobacter, Treponema, Actinosporangium, Vibrio, Ruminococcus, Methanobrevibacter, Shigella,* Frankia, *Streptomyces, Anaeroplasma* and *Coprococcus.* More preferably, the bacteria are selected from: *Anearostipeshadrus*, *Bifidobacterium pseudocatenulatum*, *Nitrosomonas* sp.Is79A3, *Oscillibactervalericigenes, Tatumella* sp.TA1, *Megamonasfuniformis*, *Thiobacillus denitrificans* and *Akkermansia muciniphila.*

Preferably, the inhibitor or microbicid for the microorganism includes a compound, polypeptide, a polynucleotide, a natural plant or a natural plant extract.

More preferably, the inhibitor or microbicid for the microorganism is an antibiotic, for example, the antibiotic is one or two or more of a β-lactam antibiotic, an aminoglycoside antibiotic, a tetracycline antibiotic, a chloramphenicol antibiotic, a macrolide antibiotic, a glycopeptide antibiotic, a quinolone antibiotic, a nitroimidazole antibiotic, a rifamycin antibiotic, an echinocandin antibiotic, a polyene antibiotic, a pyrimidine antibiotic, an allylamine antibiotic, an azole antibiotic and other antibiotics.

For example, in some embodiments, the antibiotic may include one or more of the followings: β-lactam antibiotics, including penicillins (e.g., penicillin V), amoxicillin, ampicillin, bacampicillin, carbenicillin, cloxacillin, dicloxacillin, flucloxacillin, mezlocillin, nafcillin, oxacillin, penicillin G, piperacillin, pivampicillin, pivmecillinam, ticarcillin, cephalosporins such as cefacetrile, cefadroxil, cefalexin, cefaloglycin, cefalonium, cefaloridine, cefalotin, cefapirin, cefatrizine, cefazaflur, cefazedone, cefazolin, cefradine, cefroxadine, ceftezole, cefaclor, cefamandole, cefmetazole, cefonicid, cefotetan, cefoxitin, cefprozil, cefuroxime, cefuzonam, cefcapene, cefdaloxime, cefdinir, cefditoren, cefetamet, cefixime, cefmenoxime, cefodizime, cefotaxime, cefpimizole, cefpodoxime, cefteram, ceftibuten, ceftiofur, ceftiolene, ceftizoxime, ceftriaxone, cefoperazone, ceftazidime, cefclidine, cefepime, cefluprenam, cefoselis, cefozopran, cefpirome, cefquinome, ceftobiprole, ceftaroline, cefaclomezine, cefaloram, cefaparole, cefcanel, cefedrolor, cefempidone, cefetrizole, cefivitril, cefmatilen, cefmepidium, cefovecin, cefoxazole, cefrotil, cefsumide, cefuracetime, ceftioxide, thienamycins, monobactams, β-lactamase inhibitors, methoxypenicillins; Aminoglycoside antibiotics: including streptomycin, gentamicin, kanamycin (e.g., kanamycin A), tobramycin, amikacin, neomycin (e.g., neomycin B, neomycin C and neomycin E), ribomycin, micronomicin, azithromycin, dibekacin, sisomicin, netilmicin, paramomycin, bramycin, etc.; Tetracycline antibiotics: including tetracycline, oxytetracycline, chlortetracycline, doxycycline, etc.; chloramphenicol antibiotics: including chloramphenicol, thiamphenicol, etc.; macrolide antibiotics: including erythromycin, leucomycin, odorless erythromycin, acetylspiramycin, medimycin, josamycin, azithromycin, clarithromycin, dirithromycin, oxithromycin, telithromycin, etc.; glycopeptide antibiotics: including vancomycin, norvancomycin, teicoplanin, etc.; quinolone antibiotics: including norfloxacin, ofloxacin, ciprofloxacin, pefloxacin, gatifloxacin, enoxacin, lomefloxacin, nalidixic acid, levofloxacin, moxifloxacin, besifloxacin; nitroimidazole antibiotics: including metronidazole, tinidazole, omidazole, etc.; rifamycinoid antibiotics: including rifampicin; echinocandin antibiotics; polyene antibiotics; pyrimidines antibiotics; allylamine antibiotics; azole antibiotics; other antibiotics: fosfomycin, capreomycin, cycloserine, lincomycin, clindamycin, mitomycin, actinomycin D, bleomycin, doxorubicin, isoniazid, pyrazinamide, cyclosporine, polymyxin B combinations such as polymyxin B/trimethoprim, polymyxin B/bacitracin, polymyxin B/neomycin/gramicidin, etc.

In some embodiments, the antibiotic may be selected from Amikacin, Amoxicillin, Ampicillin, Arsphenamine, Azithromycin, Azlocillin, Aztreonam, Bacitracin, Capreomycin, Carbenicillin, Cefaclor, Cefadroxil, Cefalexin, Cefalotin, Cefamandole, Cefazolin, Cefdinir, Cefditoren, Cefixime, Cefoperazone, Cefotaxime, Cefoxitin, Cefpodoxime, Cefprozil, Ceftazidime, Ceftibuten, Ceftizoxime, Ceftriaxone, Cefuroxime, Chloramphenicol, Cilastatin, Clarithromycin, Clavulanate, Clindamycin, Clofazimine, Cloxacillin, Colistin, Cycloserine, Dalfopristin, Dapsone, Daptomycin, Dicloxacillin, Dirithromycin, Doripenem, Doxycycline, Erythromycin, Ethambutol, Ethionamide, Flucloxacillin, Fosfomycin, Furazolidone, Fusidic acid, Gentamicin, Imipenem, Isoniazid, Kanamycin, Lincomycin, Linezolid, Loracarbef, Mafenide, Meropenem, Methicillin, Metronidazole, Mezlocillin, Minocycline, Mupirocin, Nafcillin, Neomycin, Netilmicin, Nitrofurantoin, Oxacillin, Oxytetracycline, Paromomycin, Penicillin G, Penicillin V, Piperacillin, Platensimycin, Polymyxin B, Pyrazinamide, Quinupristin, Rapamycin, Rifabutin, Rifampicin, Rifampin, Rifapentine, Rifaximin, Roxithromycin, Silver sulfadiazine, Spectinomycin, Streptomycin, Sulbactam, Sulfacetamide, Sulfadiazine, Sulfamethizole, Sulfamethoxazole, Sulfanilimide, Sulfasalazine, Sulfisoxazole, Tazobactam, Teicoplanin, Telavancin, Telithromycin, Temocillin, Tetracycline, Thiamphenicol, Ticarcillin, Tigecycline, Tinidazole, Tobramycin, Trimethoprim, Troleandomycin Vancomycin, enoxacin, lomefloxacin, nalidixic acid, ciprofloxacin, levofloxacin, gatifloxacin, moxifloxacin, ofloxacin, norfloxacin, Cefotetan, Cefonicid, Cephradine, Cephapirin, Cephalothin, Cefmetazole, Cefotaxime, Moxalactam, Cefepime, Ceftaroline fosamil, Ceftobiprole, Dalbavancin, Demeclocycline, Metacycline, Ertapenem, Fidaxomicin, geldanamycin, herbimycin, Posizolid, Radezolid, Torezolid, Oritavancin, Spiramycin, Sulfadimethoxine, Sulfonamidochrysoidine, Gemifloxacin Nadifloxacin Trovafloxacin Grepafloxacin Sparfloxacin Temafloxacin, Teixobactin, Malacidins, and combinations thereof.

Preferably, the inhibitor or microbicid for the microorganism is a compound of a formula I (e.g., a formula I-1, a formula I-2, a formula I-3, a formula I-4, and a formula I-5), a formula II (e.g., a formula II-1, a formula II-2, a formula II-3, a formula II-4, a formula II-5, a formula II-6, a formula II-7, a formula II-8, a formula II-9, and a formula II-10), a formula III (e.g., a formula III-1, a formula III-2, and a formula III-3), a formula IV-1 or IV-2 (e.g., a formula IV-3, a formula IV-4, a formula IV-5, and a formula IV-6), a glycoside (e.g., a formula V), or a pharmaceutically acceptable salt or ester thereof, where an aglycone of the glycoside is a phenolic compound, a flavonoid, coumarin, benzoic acid, or a sterol, where the formula I, the formula II, the formula III, the formula IV-1, the formula IV-2, the formula V, and subformulae thereof are defined as above.

In a specific implementation of the present disclosure, the inhibitor or microbicid for the microorganism is compounds 1-8, having the following chemical structures:

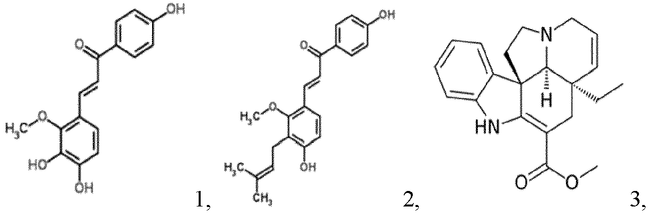

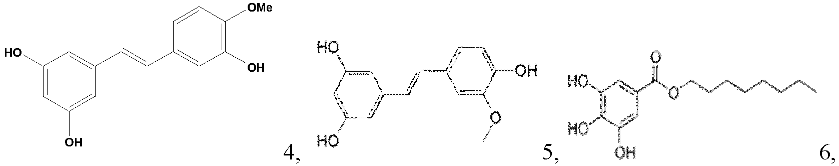

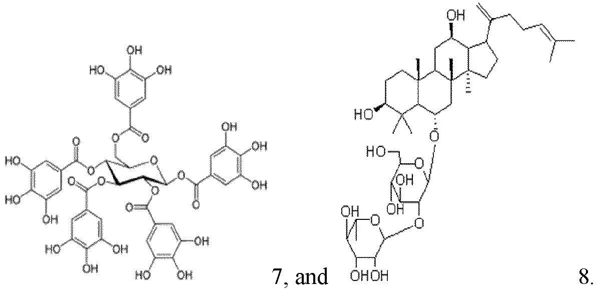

Preferably, the inhibitor or microbicid for the microorganism in the present disclosure is a natural plant or a natural plant extract or a combination of the natural plant and the natural plant extract, preferably, the natural plant is selected from one or a combination of two or more of radix paeoniae alba, fructus forsythiae, fructus aurantii, rhizoma rehmanniae, dried tangerine peel, pseudo-ginseng, turtle shells, roots of kirilow rhodiola, dried rehmannia roots, coix seeds, semen cassiae and liquorice.

In a specific implementation of the present disclosure, the inhibitor or microbicid for the microorganism is a composition including turtle shells, roots of kirilow rhodiola, dried rehmannia roots, coix seeds, radix paeoniae alba, fructus forsythiae, semen cassiae and liquorice, preferably, the composition includes, by weight, 5-15 parts the turtle shells, 10-30 parts the roots of kirilow rhodiola, 5-15 parts the dried rehmannia roots, 5-15 parts the coix seeds, 8-20 parts the radix paeoniae alba, 5-15 parts the fructus forsythiae, 5-15 parts the semen cassiae and 5-15 parts the liquorice, more preferably, the composition includes 10 parts the turtle shells, 20 parts the roots of kirilow rhodiola, 10 parts the dried rehmannia roots, 10 parts the coix seeds, 12 parts the radix paeoniae alba, 10 parts the fructus forsythiae, 10 parts the semen cassiae and 9 parts the liquorice.

Preferably, the drug is a preparation for ophthalmic administration or a preparation for gastrointestinal administration. More preferably, the preparation for ophthalmic administration is an eye drop, an eye ointment, an eye gel, an ocular insert, an eye solution or an intraocular injection. More preferably, the preparation for gastrointestinal administration is a tablet (including a sugar-coated tablet, a film-coated tablet, a sublingual tablet, an orally disintegrating tablet, an oral tablet, etc.), a pill, powder, a granule, a capsule (including a soft capsule and a microcapsule), a troche, syrup, a solution, an emulsion, a suspension, a controlled release preparation (for example, an instantaneous release preparation, a sustained release preparation and a sustained-release microcapsule), an aerosol or a film agent (for example, an oral disintegrating film agent and a mouth mucosa-adhesive film agent).

Specifically, the above pharmaceutically acceptable excipient may include one or more of a sweetening agent (specifically, for example, sucrose, xylitol, fructooligosaccharide, sodium cyclamate, stevia sugar, or aspartame), a flavoring agent (for example, spices, or essence), mucilage (specifically, for example, sodium alga acid, Arabic gum, gelatin, methylcellulose, or sodium carboxymethylcellulose), a clarifying agent (specifically, for example, chitosan, or gelatin), a preservative (specifically, for example, benzoic acid and salt thereof, sorbic acid and salt thereof, or nipagin series), a disintegrating agent (specifically, for example, low-substituted hydroxypropyl cellulose, crospovidone, sodium starch glycolate, croscarmellose sodium, or starch), a binder (specifically, for example, hydroxy propyl cellulose, hydroxypropyl methylcellulose, povidone, copovidone, or pregelatinized starch), a lubricant (specifically, for example, stearic acid, magnesium stearate, or sodium fumaryl stearate.), a wetting agent (specifically, for example, polyoxyethylene sorbide fatty acid ester, poloxamer, or a polyoxyethylene castor oil derivative), a suspending agent (specifically, for example, hydroxypropyl methylcellulose, hydroxy propyl cellulose, povidone, copovidone, sodium carboxymethylcellulose, or methylcellulose), a stabilizer (specifically, for example, citric acid, fumaric acid, or succinic acid), a filler (specifically, for example, starch, sucrose, lactose, or microcrystalline cellulose), a binding agent (specifically, for example, a cellulose derivative, alginate, gelatin, or polyvinylpyrrolidone), etc. The present disclosure further provides a method for establishing an eye disease model. The method includes infecting the eye disease model with a microorganism.

Preferably, the infection includes direct contact with the microorganism or indirect contact with the microorganism. In a specific implementation, the eye disease model with eyes infected with the microorganism is obtained by raising a non-human animal in an SPF environment.

Preferably, the microorganism is from intestinal bacteria of the same individual or is the same as the intestinal bacteria of the individual.

Preferably, an eye disease includes retinal degeneration; more preferably, the retinal degeneration is progressive retinal degeneration.

Preferably, the retinal degeneration is inherited retinal degeneration (IRD).

Preferably, the eye disease includes LCA, RP, arRP, EORD, EORP, PPRPE, rettelangiectasia and/or choroideremia like fundus.

Preferably, the eye disease includes ocular inflammation, for example, uveitis, glaucoma, age-related macular degeneration (AMD), hyalitis, choroiditis, retinitis, retinal vasculitis and optic neuritis as well as uveitis, a behcet disease, a Vogt-Koyanagi-Harada syndrome, uveitis, retinopathy, sympathetic ophthalmia, cataract, conjunctivitis, or glaucoma.

Preferably, the model is a non-human animal, preferably, a monkey, a dog, a chimpanzee, a rat and a mouse.

Preferably, a model carrier is a cell, tissue or an organ, and the cell, tissue or organ is from a human or the non-human animal.

Preferably, the cell is a primary cell or cell line.

Preferably, the tissue is ocular tissue, and the organ is an ocular organ.

Preferably, the tissue or the organ is regenerated tissue or a regenerated organ.

Preferably, the model has a gene pathogenic mutation.

Preferably, a gene to which the pathogenic mutation occurs is a gene related to maintaining a retinal barrier structure, and the retinal barrier is an outer blood-retinal barrier and/or an inner blood-retinal barrier.

Preferably, the model or the model carrier has a pathogenic mutation of an eye disease related gene, and the gene to which the eye gene pathogenic mutation occurs is selected from one or a combination of two or more of the following genes: ABCA4, ABCC6, ABCC9, ACBD5, ACO2, ACO2, ACTG1, ADGRV1, AHI1, AIPL1, ALMS1, AMY2B, APC, ARFGEF1, ARL13B, ARL13B, ARL6, ARMC9, ATOH7, B9D1, BAG3, BBS1, BBS1, BBS2, BBS5, BEST1, C2CD3, CA4, CABP4, CACNA1F, CBS, CC2D2A, CDH23, CDH23, CDHR1, CEMIP2, CEP104, CEP250, CEP290, CEP290, CEP41, CEP78, CERKL, CFAP410, CFAP418, CHM, CLCC1, CLCN7, CLN3, CLN5, CLN8, CLRN1, CLRN1, CNGA1, CNGA1, CNGA3, CNGB1, CNGB3, CNNM4, COL11A1, COL11A2, COL18A1, COL2A1, COL4A1, COL9A1, COL9A2, CP, CP, CPLANE1, CRB1, ERCC4, CSPP1, CTNNA1, CYP4V2, DHDDS, DYNC2H1, DYNC2I1, DYNC2I2, ENPP1, ERCC4, EVC2, EYS, EYS, F5, FAM161A, FBN1, FKRP, FKTN, FLG, FLVCR1, FOXE3, FUZ, GLB1, GMPPB, GNAT1, GRK1, GRM6, GUCA1A, GUCA1B, GUCY2D, HADHA, HGSNAT, HPS3, HPS5, IDH3B, IFT122, IFT140, IFT140, IFT43, IFT52, IFT74, IFT80, IFT80, IFT81, IFT88, IKBKG, IMPDH1, IMPG2, INPP5E, INTU, IQCB1, IQCE, IREB2, KCNJ13, KCNQ1, KCNV2, KIAA0586, KIAA0753, KIF7, KIZ, KIZ-AS1, KLHL7, KRIT1, LBR, LCA5, LOC101927157, LOC111365204, LRP2, LRP5, MAK, MAPKAPK3, MATK, MCOLN1, MERTK, MKS1, MPDZ, MT-ATP6, MT-CO3, MT-TE, MT-TL1, MTHFR, MUTYH, MYO7A, MYO7A, NMNAT1, NPHP1, NR2E3, OCA2, OTX2, PANK2, PAX6, PCARE, PCDH15, PDE6A, PDE6B, PDE6B, PDE6D, PEX1, PEX1, PEX12, PEX26, PEX6, PHF3, PITPNM3, PKD2, PLA2G5, POC5, POMT1, PRCD, PRDM13, PROM1, PRPF3, PRPF31, PRPF8, PRPH2, RAD51C, RBP3, RBP4, RD3, RDH12, RDH5, RGR, RGR, RHO, RIMS1, RLBP1, ROM1, RP1, RP1L1, RP2, RPE65, RPE65, RPGR, RPGRIP1, RPGRIP1L, RS1, SACS, SAG, SCAPER, SDCCAG8, SIX6, SLC19A1, SLC22A5, SLC26A4, SLC2A9, SLTM, SNRNP200, SPAG17, SPATA7, SPG11, TFAP2A, TGFB2, TGFBR2, TMEM107, TMEM237, TMEM67, TOGARAM1, TOPORS, TPP1, TRAF3IP1, TREX1, TRIM59-IFT80, TSPAN12, TTC21B, TTC21B, TTC8, TULP1, USH1C, USH2A, USH2A, USH2A, USH2A, USH2A-AS1, VAC14, VCAN, VCAN, VCAN-AS1, VHL, VPS13B, WDR19, WDR19, WDR35, WDR73, YARS1, ZFYVE26, ZFYVE26 and ZNF408.

In a specific implementation, the eye disease related gene to which the pathogenic mutation occurs in the model or the model carrier includes the CRB1 gene.

Preferably, the mutation of the CRB1 gene of the model or the model carrier includes one or two or more of the following mutations: c. 107C>G, c.111delT, c.135C>G, c.257_258dupTG, c.258C>T, c.428_432delGATTC, c.430T>G, c.470G>C, c.481dupG, c.482C>T, c.584G>T, c.613_619del, c.717_718insG, c.750T>G, c.915T>A, c.929G>A, c.936T>G, c.998G>A, c.1084C>T, c.1125C>G, c.1148G>A, c.1208C>G, c.1269C>A, c.1298A>G, c.1313G>A, c.1438T>C, c.1438T>G, c.1576C>T, c.1604T>C, c.1690G>T, c.1733T>A, c.1750G>T, c.1760G>A, c.1834T>C, c.1963delC, c.2025G>T, c.2042G>A, c.2128G>C, c.2129C>T, c.2185_2186insAlu, c.2219C>T, c.2222T>C, c.2234C>T, c.2245_2247del 3bp (TCA), c.2258T>C, c.2290C>T, c.2365_2367del AAT, in frame deletion, c.2401A>T, c.2438_2439ins>100A, c.2441_2442del, c.2465G>A, c.2479G>T, c.2506C>A, c.2509G>C, c.2536G>A, c.2548_2551delGGCT, c.2548G>A, c.2555T>C, c.2611_2613insT, c.2671T>G, c.2676delG, c.2681A>G, c.2688T>A, c.2816G>A, c.2843G>A, c.2853dupT, c.2884_2886delTTA, c.2957A>T, c.2966T>C, c.2983G>T, c.3002A>T, c.3008T>C, c.3035T>C, c.3037C>T, c.3074G>A, c.3074G>T, c.3122T>C, c.3212T>C, c.3296C>A, c.3299T>C, c.3299T>G, c.3307G>A/C, c.3320T>C, c.3320T>G, c.3331G>T, c.3343_3352del, c.3347delT, c.3343_3352del, c.3347delT, c.3427delT, c.3482A>G, c.3493T>C, c.3655T>G, c.3541T>C, c.3542dupG, c.3593A>G, c.3613G>A, c.3653G>T, c.3659_3660delinsA, c.3664C>T, c.3668G>C, c.3676G>T, c.3713_3716dup, c.3879G>A, c.3914C>T, c.3949A>C, c.3961T>A, c.3988delG, c.3988G>T, c.3995G>T, c.3996C>A, c.3997G>T, c.4094C>A, c.4121_4130del, c.4142C>T, c.4148G>A, c.2128+2T>G, c.2842+5G>A, c.3878+1G>T, c.4005+1G>A, c.4005+2T>G, c.4006-2A>G, c.4006-1G>T, c.619G>A, c.614T>C, c.1472A>T, c.1903T>C, c.2809G>A, c.3103C> T, c.4082G> A, c.4060G>A, c.866C>T, c.1463T>C, c.2035C>G, c.2306_2307GC>AG, c.2306G>A, c.2714G>A, c.2875G>A and c.3992G>A.

Further preferably, the mutation of the CRB1 gene of the model or the model carrier includes one or two or more of the following mutations: c.4006-1G>T, c.3686G>C, (p.Cys1229Ser), c.2842+1delinsAA, c.4060G>A, (p.Ala1354Thr), c.3991C>T, (p.Arg1331Cys), c.3014A>T, (p.Asp1005Val), c.4005+1G>A, c.2680_2684del, (p.Asn894fs), c.1733T>A, (p.Val578Glu), c.455G>A, (p.Cys152Tyr), c.3462_3463del, (p.Cys1154_Glu1155delinsTer), c.3037C>T, (p.Gln1013Ter), c.2673C>A, (p.Cys891Ter), c.2230C>T, (p.Arg744Ter), c.3676G>T, (p.Gly1226Ter), c.2842+5G>A, c.2842T>C, (p.Cys948Arg), c.3988del, (p.Glu1330fs), c.2506C>A, (p.Pro836Thr), c.2291G>A, (p.Arg764His), c.1576C>T, (p.Arg526Ter), c.613_619del, (p.Ile205fs), c.3320T>C, (p.Leu1107Pro), c.2688T>A, (p.Cys896Ter), c.2555T>C, (p.Ile852Thr), c.2222T>C, (p.Met741Thr), c.1148G>A, (p.Cys383Tyr), c.2843G>A, (p.Cys948Tyr), c.4121_4130del, (p.Ala1374fs), c.3307G>A, (p.Gly1103Arg), c.484G>A, (p.Val162Met), c.2401A>T, (p.Lys801Ter), c.2234C>T, (p.Thr745Met), c.2290C>T, (p.Arg764Cys), c.3122T>C and (p.Met1041Thr).

Preferably, the mutation of the CRB 1 gene of the model or the model carrier is the Rd8 mutation.

Preferably, the mutation is a homozygous mutation or a heterozygous mutation.

Preferably, the aforementioned gene mutation exists innately in a body of the model or the model carrier or the mutation is acquired due to a gene recombination operation.

Preferably, a humanized CRB1 gene or a human CRB1 gene exists in the body of the model or the model carrier, and an endogenous CRB1 gene is missing or not expressed.

Preferably, the non-human animal has a colonic epithelium barrier defect and/or related colonic wall inflammation.

In a specific example, protein Occludin in the model body is significantly missing. In a specific example, the protein Occludin in the model body is significantly missing and Claudin1 expression is not significant.

The microorganism is one or a combination of two or more of the bacteria, archaebacteria, protists, fungi or viruses, preferably, the microorganism is the bacteria, and the bacteria are selected from: one or two or more of *Anearostipes*, *Bifidobacterium, Megamonas, Nitrosomonas, Oscillibacter, Tatumella, Thiobacillussp.,Clostridium, Acinetobacter, Streptococcus, Mannheimia*, *Fibrobacter, Prevotella, Campylobacter, Actinomyces, Hymenobacter*, *Escherichia, Tissierella*, *Klebsiella, Porphyromonas, Azospira*, *Aquimarina*, *Achromobacter, Acidithiobacillus, Burkholderia, Marinobacter, Treponema, Actinosporangium, Vibrio, Ruminococcus, Methanobrevibacter, Shigella,* Frankia, *Streptomyces, Anaeroplasma* and *Coprococcus.*

Specifically, the bacteria are selected from: one or two or more of Anearostipeshadrus, Bifidobacterium pseudocatenulatum, Nitrosomonas sp.Is79A3, Oscillibactervalericigenes, Tatumella sp.TA1, Megamonasfuniformis, Thiobacillus denitrificans, Clostridium tetani, Clostridium perfringens, Clostridium botulinum, Acinetobacter calcoaceticus, Acinetobacter Lwoffii, Acinetobacter baumanii, Acinetobacter haemolyticus, Acinetobacter junii, Acinetobacter johnsonii, Pyogenic streptococcus, Hemolytic streptococcus, Fibrobacter succinogenes, Intestinal Bacillus filiformis, Porphyromonas asaccharolytica, Porphyromonas endodontalis, Porphyromonas gingivalis, Campylobacter jejuni, Campylobacter coli, Campylobacter lari, Campylobacter upsaliensis, Campylobacter concisus, Campylobacter fetus, Actinomyces israelii, Actinomyces naeslundii, Actinomyces odontolyticus, Actinomyces viscosus, Actinomyces neuii, Escherichia coli, Escherichia blattae, Escherichia fergusonii, Escherichia hermannii, Escherichia vulneris, Tissierella praeacuta, Klebsiella pneumoniae, Klebsiella ozaenae, Azospirillum brasilense, Achromobacter, Thiobacillus denitrificans, Thiobacillus ferrooxidans, Thiobacillus thiooxidans, Thiobacillus neapolitanus, Burkholderia, Mycobacterium marinum, Treponema pallidum, Treponema hyodysenteriae, Vibrio metschnikovi, Ruminococcus albus, Ruminococcus flavefaciens, Methanobrevibacter ruminantium, Shigella dysenteriae, Shigella flexneri, Shigella bogdii, Shigella sonnei, Frankia, Coprococcus eutactus, Streptomyces albus, Pseudomonas mendocina, Micrococcus sedentarius, Alicycline denitrifying bacteria, Achromobacter xylosoxidans, Sphingomonas, Mycobacterium abscessus, Arthrobacter aurescens, Prevotella, Sinorhizobium meliloti, Acid yeast, Staphylococcus epidermidis, Pseudomonas aeruginosa, Staphylococcus aureus, Staphylococcus haemolyticus, Pseudomonas putida, Stenotrophomonas maltophilia, Bacillus cereus, Bacillus megaterium, Lactobacillus reuteri, Haemophilus vaginalis, Bee enterococcus faecium, Normal cytophaga hutchinsonii, Bacillus licheniformis, Xanthomonas oryzae pv. oryzae, Acinetobacter baumannii, Acinetobacter calcoaceticus, Comamonas testosteroni, Mycobacterium kansasii, Bacillus thuringiensis, Citrobacter koseri, Dyadobacter fermentans, Serratia marcescens, Sphingomonas wittichii, Klebsiella pneumoniae, Pseudomonas fluorescens, Ralstonia pickettii, Lactobacillus crispatus, Burkholderia, Lactobacillus delbrueckii, Meiothermussilvanus(D), Colon bacillus, Micrococcus luteus, Bacillus subtilis, Corynebacterium aurimucosum and Finegoldia magna.

Preferably, the infection method includes causing the microorganism to directly or indirectly make contact with a to-be-infected part of the model carrier, and the indirect contact refers to a blood-retinal barrier existing between the microorganism and the to-be-infected part, preferably, the blood-retinal barrier is the outer blood-retinal barrier or the inner blood-retinal barrier.

In a specific implementation, the infection method includes infecting eyes with intestinal bacteria through peripheral blood. Specifically, an intestinal epithelial barrier of the non-human animal model is significantly damaged, resulting in that the intestinal bacteria enter the peripheral blood. In a model body, the retinal barrier is also significantly damaged, so the retina is infected with the intestinal bacteria entering the peripheral blood. In a specific implementation, the mutation of the CRB 1 gene occurs to the non-human animal model. In a specific implementation, the mutation of the CRB 1 gene is the Rd8 mutation.

Further, the present disclosure provides a method for preparing an ocular inflammation model, and the method model is infecting the non-human animal with the microorganism.

Preferably, the ocular inflammation is caused by an intestinal flora or a flora which is the same as the intestinal flora.

The non-human animal is the aforementioned animal.

The microorganism is the aforementioned microorganism.

Further, the present disclosure provides a method for preparing a retinal degeneration disease model, and the method includes infecting the non-human animal suffering from the retinal degeneration with the microorganism.

Preferably, the ocular inflammation retinal degeneration disease is the aforementioned retinal disease.

The non-human animal is the aforementioned animal.

The microorganism is the aforementioned microorganism.

Further, the present disclosure provides a model carrier suffering from the ocular inflammation, and the ocular inflammation is caused by being infected with the microorganism.

Preferably, the microorganism is from intestinal bacteria of the same individual or is the same as the intestinal bacteria of the individual.

Preferably, an eye disease includes retinal degeneration; more preferably, the retinal degeneration is progressive retinal degeneration.

Preferably, the retinal degeneration is inherited retinal degeneration (IRD).

Preferably, the retinal degeneration is an inherent disease of the model animal or the retinal degeneration from which the model animal suffers due to a gene manipulation.

Preferably, the eye disease includes LCA, RP, arRP, EORD, EORP, PPRPE, rettelangiectasia and/or choroideremia like fundus.

Preferably, the eye disease includes ocular inflammation, for example, uveitis, glaucoma, age-related macular degeneration (AMD), hyalitis, choroiditis, retinitis, retinal vasculitis and optic neuritis as well as uveitis, a behcet disease, a Vogt-Koyanagi-Harada syndrome, uveitis, retinopathy, sympathetic ophthalmia, cataract, conjunctivitis, or glaucoma.

Preferably, the model is a non-human animal, preferably, a monkey, a dog, a chimpanzee, a rat and a mouse.

Preferably, a model carrier is a cell, tissue or an organ, and the cell, tissue or organ is from a human or the non-human animal.

Preferably, the cell is a primary cell or cell line.

Preferably, the tissue is ocular tissue, and the organ is an ocular organ.

Preferably, the tissue or the organ is regenerated tissue or a regenerated organ.

Preferably, the model has a gene pathogenic mutation.

Preferably, a gene to which the pathogenic mutation occurs is a gene related to maintaining a retinal barrier structure, and the retinal barrier is the outer blood-retinal barrier and/or the inner blood-retinal barrier.

Preferably, in the model carrier, the mutation occurs to one or two or more of the following genes: one or a combination of two or more of ABCA4, ABCC6, ABCC9, ACBD5, ACO2, ACO2, ACTG1, ADGRV1, AHI1, AIPL1, ALMS1, AMY2B, APC, ARFGEF1, ARL13B, ARL13B, ARL6, ARMC9, ATOH7, B9D1, BAG3, BBS1, BBS1, BBS2, BBS5, BEST1, C2CD3, CA4, CABP4, CACNA1F, CBS, CC2D2A, CDH23, CDH23, CDHR1, CEMIP2, CEP104, CEP250, CEP290, CEP290, CEP41, CEP78, CERKL, CFAP410, CFAP418, CHM, CLCC1, CLCN7, CLN3, CLN5, CLN8, CLRN1, CLRN1, CNGA1, CNGA1, CNGA3, CNGB1, CNGB3, CNNM4, COL11A1, COL11A2, COL18A1, COL2A1, COL4A1, COL9A1, COL9A2, CP, CP, CPLANE1, CRB1, ERCC4, CSPP1, CTNNA1, CYP4V2, DHDDS, DYNC2H1, DYNC2I1, DYNC2I2, ENPP1, ERCC4, EVC2, EYS, EYS, F5, FAM161A, FBN1, FKRP, FKTN, FLG, FLVCR1, FOXE3, FUZ, GLB1, GMPPB, GNAT1, GRK1, GRM6, GUCA1A, GUCA1B, GUCY2D, HADHA, HGSNAT, HPS3, HPS5, IDH3B, IFT122, IFT140, IFT140, IFT43, IFT52, IFT74, IFT80, IFT80, IFT81, IFT88, IKBKG, IMPDH1, IMPG2, INPP5E, INTU, IQCB1, IQCE, IREB2, KCNJ13, KCNQ1, KCNV2, KIAA0586, KIAA0753, KIF7, KIZ, KIZ-AS1, KLHL7, KRIT1, LBR, LCA5, LOC101927157, LOC111365204, LRP2, LRP5, MAK, MAPKAPK3, MATK, MCOLN1, MERTK, MKS1, MPDZ, MT-ATP6, MT-CO3, MT-TE, MT-TL1, MTHFR, MUTYH, MYO7A, MYO7A, NMNAT1, NPHP1, NR2E3, OCA2, OTX2, PANK2, PAX6, PCARE, PCDH15, PDE6A, PDE6B, PDE6B, PDE6D, PEX1, PEX1, PEX12, PEX26, PEX6, PHF3, PITPNM3, PKD2, PLA2G5, POC5, POMT1, PRCD, PRDM13, PROM1, PRPF3, PRPF31, PRPF8, PRPH2, RAD51C, RBP3, RBP4, RD3, RDH12, RDH5, RGR, RGR, RHO, RIMS1, RLBP1, ROM1, RP1, RP1L1, RP2, RPE65, RPE65, RPGR, RPGRIP1, RPGRIP1L, RS1, SACS, SAG, SCAPER, SDCCAG8, SIX6, SLC19A1, SLC22A5, SLC26A4, SLC2A9, SLTM, SNRNP200, SPAG17, SPATA7, SPG11, TFAP2A, TGFB2, TGFBR2, TMEM107, TMEM237, TMEM67, TOGARAM1, TOPORS, TPP1, TRAF3IP1, TREX1, TRIM59-IFT80, TSPAN12, TTC21B, TTC21B, TTC8, TULP1, USH1C, USH2A, USH2A, USH2A, USH2A, USH2A-AS1, VAC14, VCAN, VCAN, VCAN-AS1, VHL, VPS13B, WDR19, WDR19, WDR35, WDR73, YARS1, ZFYVE26, ZFYVE26 and ZNF408.

In a specific implementation, the eye disease related gene to which the pathogenic mutation occurs in the model carrier includes the CRB1 gene.

Preferably, the mutation of the CRB1 gene of the model carrier includes one or two or more of the following mutations: c.107C>G, c.111delT, c.135C>G, c.257_258dupTG, c.258C>T, c.428_432delGATTC, c.430T>G, c.470G>C, c.481dupG, c.482C>T, c.584G>T, c.613_619del, c.717_718insG, c.750T>G, c.915T>A, c.929G>A, c.936T>G, c.998G>A, c.1084C>T, c.1125C>G, c.1148G>A, c.1208C>G, c.1269C>A, c.1298A>G, c.1313G>A, c.1438T>C, c.1438T>G, c.1576C>T, c.1604T>C, c.1690G>T, c.1733T>A, c.1750G>T, c.1760G>A, c.1834T>C, c.1963delC, c.2025G>T, c.2042G>A, c.2128G>C, c.2129C>T, c.2185_2186insAlu, c.2219C>T, c.2222T>C, c.2234C>T, c.2245_2247del 3bp (TCA), c.2258T>C, c.2290C>T, c.2365_2367del AAT, in frame deletion, c.2401A>T, c.2438_2439ins>100A, c.2441_2442del, c.2465G>A, c.2479G>T, c.2506C>A, c.2509G>C, c.2536G>A, c.2548_2551delGGCT, c.2548G>A, c.2555T>C, c.2611_2613insT, c.2671T>G, c.2676delG, c.2681A>G, c.2688T>A, c.2816G>A, c.2843G>A, c.2853dupT, c.2884_2886delTTA, c.2957A>T, c.2966T>C, c.2983G>T, c.3002A>T, c.3008T>C, c.3035T>C, c.3037C>T, c.3074G>A, c.3074G>T, c.3122T>C, c.3212T>C, c.3296C>A, c.3299T>C, c.3299T>G, c.3307G>A/C, c.3320T>C, c.3320T>G, c.3331G>T, c.3343_3352del, c.3347delT, c.3343_3352del, c.3347delT, c.3427delT, c.3482A>G, c.3493T>C, c.3655T>G, c.3541T>C, c.3542dupG, c.3593A>G, c.3613G>A, c.3653G>T, c.3659_3660delinsA, c.3664C>T, c.3668G>C, c.3676G>T, c.3713_3716dup, c.3879G>A, c.3914C>T, c.3949A>C, c.3961T>A, c.3988delG, c.3988G>T, c.3995G>T, c.3996C>A, c.3997G>T, c.4094C>A, c.4121_4130del, c.4142C>T, c.4148G>A, c.2128+2T>G, c.2842+5G>A, c.3878+1G>T, c.4005+1G>A, c.4005+2T>G, c.4006-2A>G, c.4006-1G>T, c.619G>A, c.614T>C, c.1472A> T, c.1903T>C, c.2809G> A, c.3103C> T, c.4082G> A, c.4060G>A, c.866C>T, c.1463T>C, c.2035C>G, c.2306_2307GC>AG, c.2306G>A, c.2714G>A, c.2875G>A and c.3992G>A.

Further preferably, the mutation of the CRB1 gene of the model or the model carrier includes one or two or more of the following mutations: c.4006-1G>T, c.3686G>C, (p.Cys1229Ser), c.2842+1delinsAA, c.4060G>A, (p.Ala1354Thr), c.3991C>T, (p.Arg1331Cys), c.3014A>T, (p.Asp1005Va1), c.4005+1G>A, c.2680_2684del, (p.Asn894fs), c.1733T>A, (p.Val578Glu), c.455G>A, (p.Cys152Tyr), c.3462_3463del, (p.Cys1154_Glu1155delinsTer), c.3037C>T, (p.Gln1013Ter), c.2673C>A, (p.Cys891Ter), c.2230C>T, (p.Arg744Ter), c.3676G>T, (p.Gly1226Ter), c.2842+5G>A, c.2842T>C, (p.Cys948Arg), c.3988del, (p.Glu1330fs), c.2506C>A, (p.Pro836Thr), c.2291G>A, (p.Arg764His), c.1576C>T, (p.Arg526Ter), c.613_619del, (p.Ile205fs), c.3320T>C, (p.Leu1107Pro), c.2688T>A, (p.Cys896Ter), c.2555T>C, (p.Ile852Thr), c.2222T>C, (p.Met741Thr), c.1148G>A, (p.Cys383Tyr), c.2843G>A, (p.Cys948Tyr), c.4121_4130del, (p.Ala1374fs), c.3307G>A, (p.Gly1103Arg), c.484G>A, (p.Val162Met), c.2401A>T, (p.Lys801Ter), c.2234C>T, (p.Thr745Met), c.2290C>T, (p.Arg764Cys), c.3122T>C and (p.Met1041Thr).

Preferably, the mutation of the CRB 1 gene of the model carrier is the Rd8 mutation.

Preferably, the mutation is a homozygous mutation or a heterozygous mutation.

The aforementioned gene mutation exists innately in a body of the model carrier or the mutation is acquired due to a gene recombination manipulation.

Preferably, a humanized CRB1 gene or a human CRB1 gene exists in the body of the model carrier, and an endogenous CRB1 gene is missing or not expressed.

Preferably, the non-human animal has a colonic epithelium barrier defect and/or related colonic wall inflammation.

In a specific example, protein Occludin in the model body is significantly missing. In a specific example, the protein Occludin in the model body is significantly missing and Claudin1 expression is not significant.

The microorganism is one or a combination of two or more of the bacteria, archaebacteria, protists, fungi or viruses, preferably, the microorganism is the bacteria, and the bacteria are selected from: one or two or more of Anearostipes, *Bifidobacterium, Megamonas, Nitrosomonas, Oscillibacter, Tatumella, Thiobacillussp.,Clostridium, Acinetobacter, Streptococcus, Mannheimia*, *Fibrobacter, Prevotella, Campylobacter, Actinomyces, Hymenobacter*, *Escherichia, Tissierella*, *Klebsiella, Porphyromonas, Azospira*, *Aquimarina*, *Achromobacter, Acidithiobacillus, Burkholderia, Marinobacter, Treponema, Actinosporangium, Vibrio, Ruminococcus, Methanobrevibacter*, *Shigella,* Frankia, *Streptomyces, Anaeroplasma* and *Coprococcus.*

Specifically, the bacteria are selected from: one or two or more of Anearostipeshadrus, Bifidobacterium pseudocatenulatum, Nitrosomonas sp.Is79A3, Oscillibactervalericigenes, Tatumella sp.TA1, Megamonasfuniformis, Thiobacillus denitrificans, Clostridium tetani, Clostridium perfringens, Clostridium botulinum, Acinetobacter calcoaceticus, Acinetobacter Lwoffii, Acinetobacter baumanii, Acinetobacter haemolyticus, Acinetobacter junii, Acinetobacter johnsonii, Pyogenic streptococcus, Hemolytic streptococcus, Fibrobacter succinogenes, Intestinal Bacillus filiformis, Porphyromonas asaccharolytica, Porphyromonas endodontalis, Porphyromonas gingivalis, Campylobacter jejuni, Campylobacter coli, Campylobacter lari, Campylobacter upsaliensis, Campylobacter concisus, Campylobacter fetus, Actinomyces israelii, Actinomyces naeslundii, Actinomyces odontolyticus, Actinomyces viscosus, Actinomyces neuii, Escherichia coli, Escherichia blattae, Escherichia fergusonii, Escherichia hermannii, Escherichia vulneris, Tissierella praeacuta, Klebsiella pneumoniae, Klebsiella ozaenae, Azospirillum brasilense, Achromobacter, Thiobacillus denitrificans, Thiobacillus ferrooxidans, Thiobacillus thiooxidans, Thiobacillus neapolitanus, Burkholderia, Mycobacterium marinum, Treponema pallidum, Treponema hyodysenteriae, Vibrio metschnikovi, Ruminococcus albus, Ruminococcus flavefaciens, Methanobrevibacter ruminantium, Shigella dysenteriae, Shigella flexneri, Shigella bogdii, Shigella sonnei, Frankia, Coprococcus eutactus, Streptomyces albus, Pseudomonas mendocina, Micrococcus sedentarius, Alicycline denitrifying bacteria, Achromobacter xylosoxidans, Sphingomonas, Mycobacterium abscessus, Arthrobacter aurescens, Prevotella, Sinorhizobium meliloti, Acid yeast, Staphylococcus epidermidis, Pseudomonas aeruginosa, Staphylococcus aureus, Staphylococcus haemolyticus, Pseudomonas putida, Stenotrophomonas maltophilia, Bacillus cereus, Bacillus megaterium, Lactobacillus reuteri, Haemophilus vaginalis, Bee enterococcus faecium, Normal cytophaga hutchinsonii, Bacillus licheniformis, Xanthomonas oryzae pv. oryzae, Acinetobacter baumannii, Acinetobacter calcoaceticus, Comamonas testosteroni, Mycobacterium kansasii, Bacillus thuringiensis, Citrobacter koseri, Dyadobacter fermentans, Serratia marcescens, Sphingomonas wittichii, Klebsiella pneumoniae, Pseudomonas fluorescens, Ralstonia pickettii, Lactobacillus crispatus, Burkholderia, Lactobacillus delbrueckii, Meiothermussilvanus(D), Colon bacillus, Micrococcus luteus, Bacillus subtilis, Corynebacterium aurimucosum and Finegoldia magna.

Preferably, the infection method includes causing the microorganism to directly or indirectly make contact with the to-be-infected part of the model carrier, and the indirect contact refers to the blood-retinal barrier existing between the microorganism and the to-be-infected part, preferably, the blood-retinal barrier is the outer blood-retinal barrier or the inner blood-retinal barrier.

In a specific implementation, the infection method includes infecting the eyes with the intestinal bacteria through the peripheral blood. Specifically, the intestinal epithelial barrier of the non-human animal model is significantly damaged, resulting in that the intestinal bacteria enter the peripheral blood. In a model body, the retinal barrier is also significantly damaged, so the retina is infected with the intestinal bacteria entering the peripheral blood. In a specific implementation, the mutation of the CRB1 gene occurs to the non-human animal model. In a specific implementation, the mutation of the CRB1 gene is the Rd8 mutation.

The model carrier suffering from the inflammation is from the disease model prepared through the aforementioned method, or is obtained by infecting ocular cells, tissue or organs from the aforementioned non-human animal with the microorganism.

The present disclosure provides use of the aforementioned method in evaluation of an eye disease targeted therapy therapeutic effect, and an eye disease includes the aforementioned eye diseases.

In a specific implementation, a targeted therapy for the aforementioned gene mutation is performed on the aforementioned model or model carrier, the model or the model carrier subjected to the targeted therapy or not subjected to the targeted therapy is divided into two groups, an eye disease model is established respectively according to the aforementioned method, and if by contrast, modeling is not successful for the group subjected to the targeted therapy, it indicates that the targeted therapy achieves a beneficial effect.

In a specific implementation, the targeted therapy is targeted at one or a combination of two or more of the following genes: ABCA4, ABCC6, ABCC9, ACBD5, ACO2, ACO2, ACTG1, ADGRV1, AHI1, AIPL1, ALMS1, AMY2B, APC, ARFGEF1, ARL13B, ARL13B, ARL6, ARMC9, ATOH7, B9D1, BAG3, BBS1, BBS1, BBS2, BBS5, BEST1, C2CD3, CA4, CABP4, CACNA1F, CBS, CC2D2A, CDH23, CDH23, CDHR1, CEMIP2, CEP104, CEP250, CEP290, CEP290, CEP41, CEP78, CERKL, CFAP410, CFAP418, CHM, CLCC1, CLCN7, CLN3, CLN5, CLN8, CLRN1, CLRN1, CNGA1, CNGA1, CNGA3, CNGB1, CNGB3, CNNM4, COL11A1, COL11A2, COL18A1, COL2A1, COL4A1, COL9A1, COL9A2, CP, CP, CPLANE1, CRB1, ERCC4, CSPP1, CTNNA1, CYP4V2, DHDDS, DYNC2H1, DYNC2I1, DYNC2I2, ENPP1, ERCC4, EVC2, EYS, EYS, F5, FAM161A, FBN1, FKRP, FKTN, FLG, FLVCR1, FOXE3, FUZ, GLB1, GMPPB, GNAT1, GRK1, GRM6, GUCA1A, GUCA1B, GUCY2D, HADHA, HGSNAT, HPS3, HPS5, IDH3B, IFT122, IFT140, IFT140, IFT43, IFT52, IFT74, IFT80, IFT80, IFT81, IFT88, IKBKG, IMPDH1, IMPG2, INPP5E, INTU, IQCB1, IQCE, IREB2, KCNJ13, KCNQ1, KCNV2, KIAA0586, KIAA0753, KIF7, KIZ, KIZ-AS1, KLHL7, KRIT1, LBR, LCA5, LOC101927157, LOC111365204, LRP2, LRP5, MAK, MAPKAPK3, MATK, MCOLN1, MERTK, MKS1, MPDZ, MT-ATP6, MT-CO3, MT-TE, MT-TL1, MTHFR, MUTYH, MYO7A, MYO7A, NMNAT1, NPHP1, NR2E3, OCA2, OTX2, PANK2, PAX6, PCARE, PCDH15, PDE6A, PDE6B, PDE6B, PDE6D, PEX1, PEX1, PEX12, PEX26, PEX6, PHF3, PITPNM3, PKD2, PLA2G5, POC5, POMT1, PRCD, PRDM13, PROM1, PRPF3, PRPF31, PRPF8, PRPH2, RAD51C, RBP3, RBP4, RD3, RDH12, RDH5, RGR, RGR, RHO, RIMS1, RLBP1, ROM1, RP1, RP1L1, RP2, RPE65, RPE65, RPGR, RPGRIP1, RPGRIP1L, RS1, SACS, SAG, SCAPER, SDCCAG8, SIX6, SLC19A1, SLC22A5, SLC26A4, SLC2A9, SLTM, SNRNP200, SPAG17, SPATA7, SPG11, TFAP2A, TGFB2, TGFBR2, TMEM107, TMEM237, TMEM67, TOGARAM1, TOPORS, TPP1, TRAF3IP1, TREX1, TRIM59-IFT80, TSPAN12, TTC21B, TTC21B, TTC8, TULP1, USH1C, USH2A, USH2A, USH2A, USH2A, USH2A-AS1, VAC14, VCAN, VCAN, VCAN-AS1, VHL, VPS13B, WDR19, WDR19, WDR35, WDR73, YARS1, ZFYVE26, ZFYVE26 and ZNF408.

In a specific implementation, the targeted therapy is targeted at one or two or more of the following mutations of the CRB1 gene: c.257_258dupTG, c.258C>T, c.428_432delGATTC, c.430T>G, c.470G>C, c.481dupG, c.482C>T, c.584G>T, c.613_619del, c.717_718insG, c.750T>G, c.915T>A, c.929G>A, c.936T>G, c.998G>A, c.1084C>T, c.1125C>G, c.1148G>A, c.1208C>G, c.1269C>A, c.1298A>G, c.1313G>A, c.1438T>C, c.1438T>G, c.1576C>T, c.1604T>C, c.1690G>T, c.1733T>A, c.1750G>T, c.1760G>A, c.1834T>C, c.1963delC, c.2025G>T, c.2042G>A, c.2128G>C, c.2129C>T, c.2185_2186insAlu, c.2219C>T, c.2222T>C, c.2234C>T, c.2245_2247del 3bp (TCA), c.2258T>C, c.2290C>T, c.2365_2367del AAT, in frame deletion, c.2401A>T, c.2438_2439ins>100A, c.2441_2442del, c.2465G>A, c.2479G>T, c.2506C>A, c.2509G>C, c.2536G>A, c.2548_2551delGGCT, c.2548G>A, c.2555T>C, c.2611_2613insT, c.2671T>G, c.2676delG, c.2681A>G, c.2688T>A, c.2816G>A, c.2843G>A, c.2853dupT, c.2884_2886delTTA, c.2957A>T, c.2966T>C, c.2983G>T, c.3002A>T, c.3008T>C, c.3035T>C, c.3037C>T, c.3074G>A, c.3074G>T, c.3122T>C, c.3212T>C, c.3296C>A, c.3299T>C, c.3299T>G, c.3307G>A/C, c.3320T>C, c.3320T>G, c.3331G>T, c.3343_3352del, c.3347delT, c.3343_3352del, c.3347delT, c.3427delT, c.3482A>G, c.3493T>C, c.3655T>G, c.3541T>C, c.3542dupG, c.3593A>G, c.3613G>A, c.3653G>T, c.3659_3660delinsA, c.3664C>T, c.3668G>C, c.3676G>T, c.3713_3716dup, c.3879G>A, c.3914C>T, c.3949A>C, c.3961T>A, c.3988delG, c.3988G>T, c.3995G>T, c.3996C>A, c.3997G>T, c.4094C>A, c.4121_4130del, c.4142C>T, c.4148G>A, c.2128+2T>G, c.2842+5G>A, c.3878+1G>T, c.4005+1G>A, c.4005+2T>G, c.4006-2A>G, c.4006-1G>T, c.619G>A, c.614T>C, c.1472A>T, c.1903T>C, c.2809G> A, c.3103C> T, c.4082G>A, c.4060G>A, c.866C>T, c.1463T>C, c.2035C>G, c.2306_2307GC>AG, c.2306G>A, c.2714G>A, c.2875G>A and c.3992G>A.

In a specific implementation, a targeted therapy drug includes modified cells, modified protein, RNA targeted at the aforementioned gene or at the aforementioned mutation site and/or DNA targeted at the aforementioned gene or at the aforementioned mutation site.

The present disclosure provides use of the disease model prepared by the aforementioned method in eye disease related study. The eye disease includes the aforementioned eye diseases. The study includes a mutual response between a disease related to inherited retinal degeneration and intestinal flora, and the like.

In a specific implementation, the disease model is infected with a type of intestinal bacteria, in a case that a pathogenic mutation carried thereby is subjected to a cell therapy, a therapeutic effect with drug administration and a therapeutic effect without drug administration are observed, and the drug is a small molecule drug, preferably, a broad-spectrum antibiotic or an antibiotic for infecting bacteria. In a specific implementation, the disease model is infected with a type of intestinal bacteria, in a case that a pathogenic mutation carried thereby is subjected to an RNA therapy, a therapeutic effect with drug administration and a therapeutic effect without drug administration are observed, and the drug is a small molecule drug, preferably, a broad-spectrum antibiotic or an antibiotic for infecting bacteria. In a specific implementation, the disease model is infected with two or more types of intestinal bacteria, in a case that a pathogenic mutation carried thereby is subjected to the cell therapy, a therapeutic effect with drug administration and a therapeutic effect without drug administration are observed, and the drug is a small molecule drug, preferably, a broad-spectrum antibiotic or an antibiotic for infecting bacteria. In a specific implementation, the disease model is infected with two types of intestinal bacteria, in a case that a pathogenic mutation carried thereby is subjected to the RNA therapy, a therapeutic effect with drug administration and a therapeutic effect without drug administration are observed, and the drug is a small molecule drug, preferably, a broad-spectrum antibiotic or an antibiotic for infecting bacteria.

Preferably, the pathogenic mutation occurs to the aforementioned gene, or the pathogenic mutation is the mutation of the aforementioned CRB gene.

The present disclosure provides use of the disease model carrier prepared by the aforementioned method in eye disease related study. The eye disease includes the aforementioned eye diseases. The study includes a synergistic effect between a disease related to inherited retinal degeneration and intestinal flora, and the like.

In a specific implementation, the disease model carrier is infected with a type of intestinal bacteria, in a case that a pathogenic mutation carried thereby is subjected to the cell therapy, a therapeutic effect with drug administration and a therapeutic effect without drug administration are observed, and the drug is a small molecule drug, preferably, a broad-spectrum antibiotic or an antibiotic for infecting bacteria. In a specific implementation, the disease model carrier is infected with a type of intestinal bacteria, in a case that a pathogenic mutation carried thereby is subjected to the RNA therapy, a therapeutic effect with drug administration and a therapeutic effect without drug administration are observed, and the drug is a small molecule drug, preferably, a broad-spectrum antibiotic or an antibiotic for infecting bacteria. In a specific implementation, the disease model carrier is infected with two or more types of intestinal bacteria, in a case that a pathogenic mutation carried thereby is subjected to the cell therapy, a therapeutic effect with drug administration and a therapeutic effect without drug administration are observed, and the drug is a small molecule drug, preferably, a broad-spectrum antibiotic or an antibiotic for infecting bacteria. In a specific implementation, the disease model carrier is infected with two types of intestinal bacteria, in a case that a pathogenic mutation carried thereby is subjected to the RNA therapy, a therapeutic effect with drug administration and a therapeutic effect without drug administration are observed, and the drug is a small molecule drug, preferably, a broad-spectrum antibiotic or an antibiotic for infecting bacteria.

Preferably, the pathogenic mutation occurs to the aforementioned gene, or the pathogenic mutation is the mutation of the aforementioned CRB gene.

The present disclosure provides use of the aforementioned disease model or disease model carrier in eye disease related drug screening. The drug includes one or a combination of two or more of a small molecule drug, a chemical drug, a polymer drug, a biological drug or a natural drug (for example, Chinese herbs or Chinese herb extracts), a cell drug, an RNA drug and a DNA drug.

The eye disease includes the aforementioned eye diseases.

Preferably, a small molecule compound is an antibiotic, and the antibiotic is usually a broad-spectrum antibiotic drug well known to those skilled in the art.

Preferably, the small molecule compound is a non-broad-spectrum antibiotic targeted at specific bacteria.

Preferably, the cell includes a modified immune cell, for example, one or a combination of two or more of a T-cell, a B-cell and a stem cell.

Preferably, the RNA includes mRNA, siRNA, sgRNA, miRNA, ASO and/or replicon RNA.

In a specific implementation, the targeted therapy is performed on the disease model or the disease model carrier, at the same time, the aforementioned drug is administered or not administered to the disease model or the disease model carrier subjected to the targeted therapy and the disease model or the disease model carrier not subjected to the targeted therapy, inflammatory progressions of the several groups are observed, and a drug therapeutic effect of the targeted therapy is evaluated.

In a specific implementation, the targeted therapy is performed on the disease model or the disease model carrier, at the same time, the aforementioned drug is administered or not administered to the disease model or the disease model carrier subjected to the targeted therapy and the disease model or the disease model carrier not subjected to the targeted therapy, inflammatory progressions of the several groups are observed, and a therapeutic effect of the small molecule drug is evaluated.

### BRIEF DESCRIPTION OF THE DRAWINGS/FIGURES

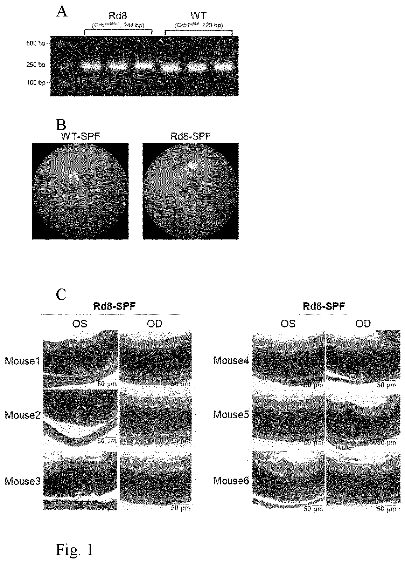

Fig. 1 is a genotype and a phenotype of Crb1^{rd8/rd8}(rd8)-SPF mice. Fig. 1A is a genotype gel electrophoresis map of Crb1^{rd8/rd8}(rd8) and Crb1^{wt/wt}(C57BL/J, wt for short) mice, Fig. 1B is representative eye fundus images of Rd8-SPF and WT-SPF mice, where a typical white spot appears in an inferior nasal quadrant of a retina of the Rd8-SPF mouse, and Fig. 1C is H&E staining for detecting two-eye pathological changes of the Rd8-SPF mice in E18.

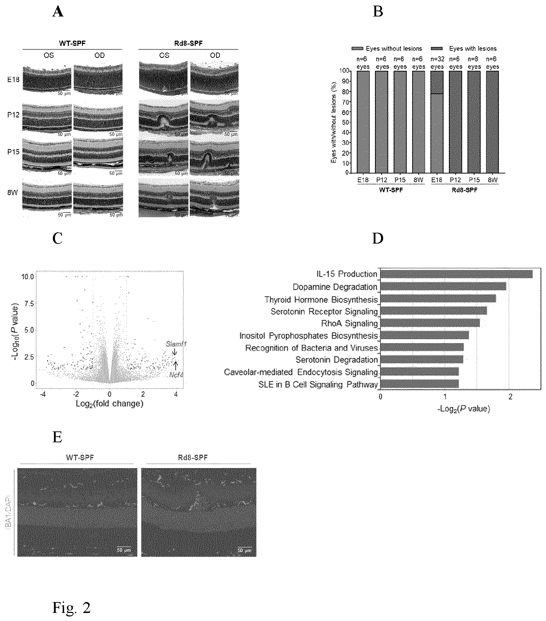

Fig. 2 is an underlying mechanism of occurrence of retinal abnormality and retinopathy of the Rd8-SPF mouse. Fig. 2A is H&E staining for detecting two-eye pathological changes of the Rd8-SPF mouse and a WT-SPF mouse in E18, P12 (before opening eyes), P15 (after opening eyes) and 8 weeks (8W), where the Rd8 mouse shows a typical retinal abnormality, including progressive retinal dysplasia (folds and Weiss ring) and degeneration, and the WT mouse shows a normal retinal structure; Fig. 2B is statistical data of eyes with lesions and eyes without lesions of the Rd8- and WT-SPF mice in E18, P12, P15 and 8W, where typical lesions can be observed from all retinae of the Rd8 mouse in P12, P15 and 8W, but no lesion is found in the WT-SPF mouse, WT-SPF: in E18, P12, P15 and 8W, each group n=6; Rd8-SPF: in E18, n=32, in P12, n=6, in P15, n=8; and Fig. 2C is DEGs volcano plot analysis of a retinal superior (without lesions) region and inferior (with lesions) region of the Rd8 mouse. Slamf1 (signalling lymphocyte activationmolecule family member 1) and Ncf4 (neutrophile granulocyte cytoplasmic factor) are two genes with the highest proliferation multiple in the retinal lesion of the Rd8-SPF mouse; Fig. 2D is IPA functional analysis of DEGs; and Fig. 2E is immunofluorescent staining for displaying that abundant IBA1+ microglial cells (red) exist in a retinal lesion region of the Rd8 mouse.

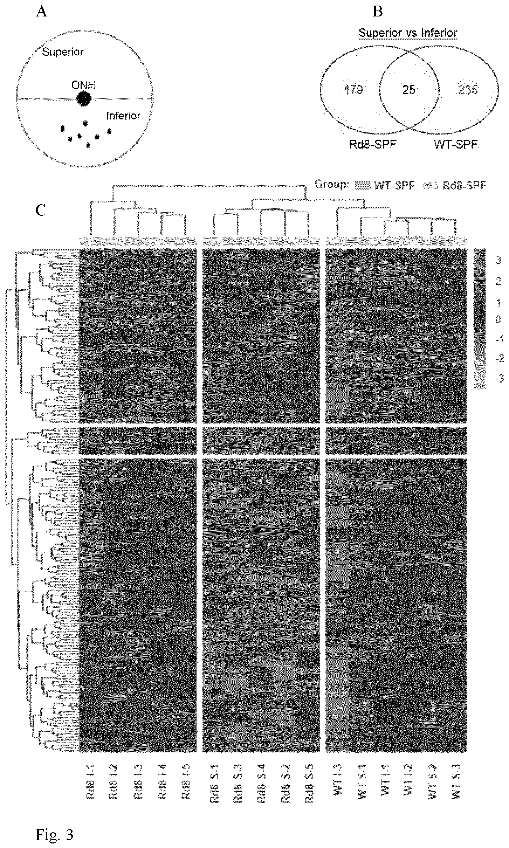

Fig. 3 is a gene expression profile for comparing a retinal superior region and inferior region of the Rd8-SPF mouse through RNA sequence analysis. Fig. 3A is a diagram of the superior (without lesions) region and inferior (with lesions) region of the Rd8-SPF mouse; Fig. 3B is an expression difference of at least two times between the retinal superior region and inferior region of the Rd8-SPF mouse through the RNA-seq analysis (P<0.05); and Fig. 3C is an expression pattern of 179 DEGs of the retinal superior region and inferior region of the Rd8- and WT-SPF mice.

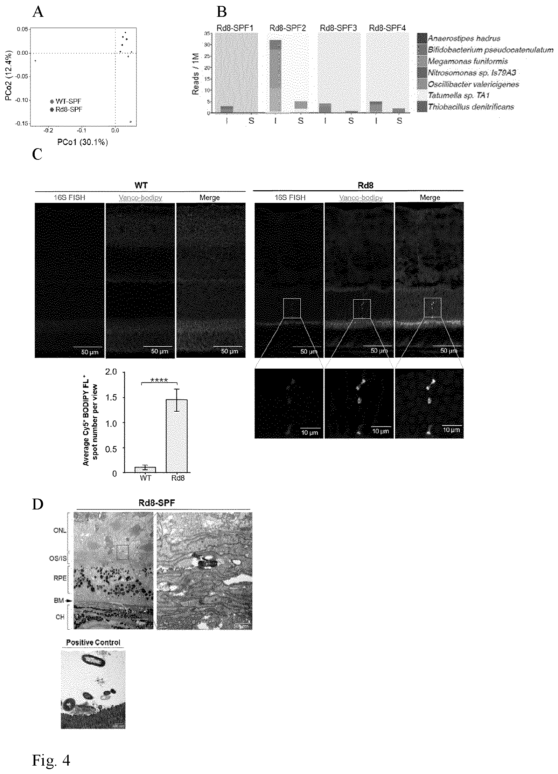

Fig. 4 is identification of bacteria in a retinal lesion of an Rd8-SPF mouse. Fig. 4A is principal coordinate analysis (PCoA), which shows that a bacteria composition in retinal tissue of the Rd8 mouse is significantly different from a WT mouse; Fig. 4B is identification of a microorganism species composition (n=4) of retinal superior (S) and inferior (I) tissue of the Rd8 mouse through metagenomics sequencing; Fig. 4C is detection of retinal bacteria 16srDNA and vanco-bodipy of Rd8- and WT-SPF mice (4-week-old) through fluorescent in situ hybridization; and Fig. 4D is transmission electron microscope (TEM) observation of a distribution of bacteria in the retinal lesion of the Rd8 mouse, where a positive control is a coli group.

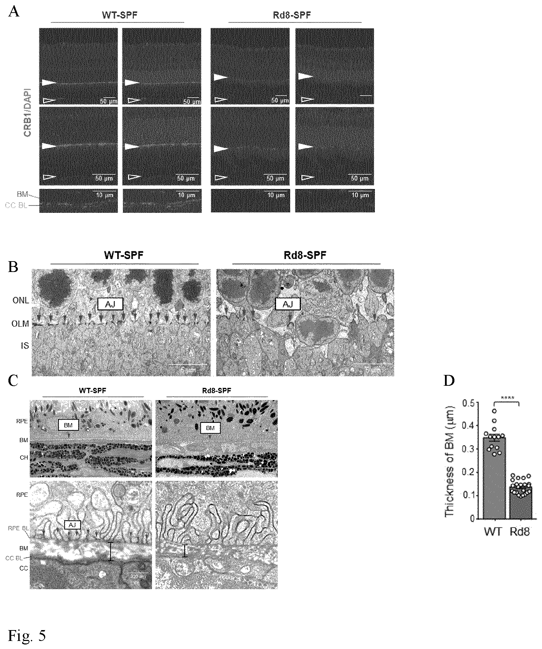

Fig. 5: breakdown of an adherent junction of a retina of an Rd8-SPF mouse. Fig. 5A is immunofluorescent staining of retinal CRB1 (red) of Rd8- and WT-SPF mice, where BM is a Bruch membrane, CC is chorion capillary, and BL is a basal lamina; Fig. 5B is transmission electron microscope observation of the adherent junction of outer limiting membrane retinae of the Rd8- and WT-SPF mice, where a red arrow is the adherent junction, AJ refers to the adherent junction, OLM is an outer limiting membrane, ONL is an outer nuclear layer, and IS refers to an inner segment; Fig. 5C is transmission electron microscope observation of basal layer adhesion and dissolution of a retinal pigment epithelium (RPE) of the Rd8-SPF mouse, basal layer distortion of a chorion capillary and collagen layer disturbance, where CC is the chorion capillary, CH is a choroid membrane, and BL is a basal lamina; and Fig. 5D is statistic analysis ****P<0.0001 of a thickness of a Bruch membrane of the Rd8- and WT-SPF mice.

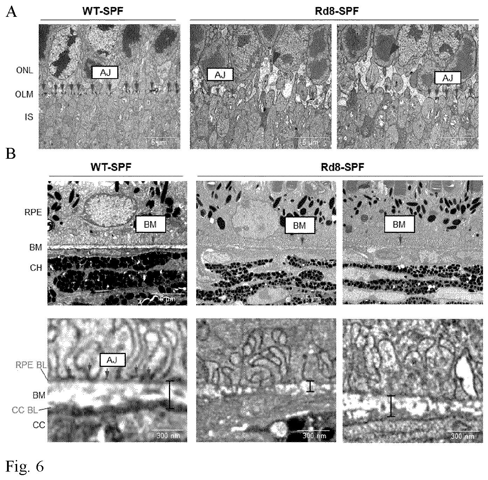

Fig. 6A is transmission electron microscope (TEM) display of outer limiting membrane (OLM) adherent junction (AJ) breakdown and an outer nuclear layer (ONL) of an Rd8-SPF mouse.
Fig. 6B is transmission electron microscope (TEM) display of thicknesses of Bruch membranes of an Rd8-SPF mouse and a WT-SPF mouse.

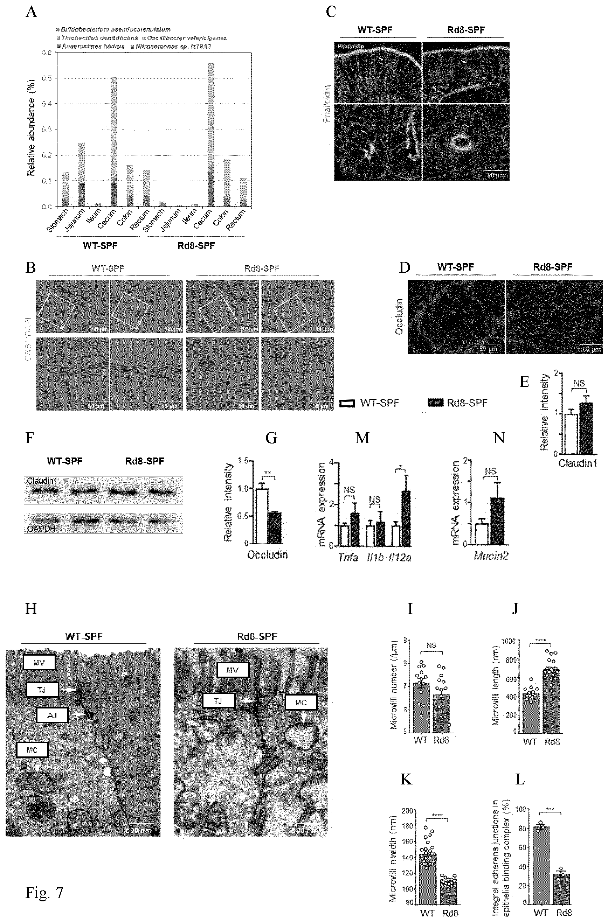

Fig. 7 is a colonic epithelium barrier defect and a related inflammation of an Rd8-SPF mouse. Fig. 7A is relative abundance (n=5) of bacteria in Rd8 retinae of different parts of gastrointestinal tracts of Rd8 and WT mice; Figs. 7B-D are immunofluorescent staining of colonic intestinal cell CRB1 protein (B), phalloidin (C) and occludin (D) of the Rd8- and WT-SPF mice; Fig. 7E is statistic analysis of relative intensities of claudin of the Rd8- and WT-SPF mice; Fig. 7F is westemblot test of expression of colon intestinal epithelial cell Claudin1 of the Rd8- and WT-SPF mice; Fig. 7G is statistic analysis of relative intensities of Claudin1 of the Rd8- and WT-SPF mice; Fig. 7H is transmission electron microscope observation of an attachment junction and a tight junction of a colonic epithelium of the Rd8- and WT-SPF mice; Fig. 7I-L are statistic analysis of the number (I), length (J) and width (K) of epithelial microvilli and the total number (L) of integral adherent junctions of the Rd8- and WT-SPF mice; and Fig. 7M-N are comparison of mRNA expression level of Tnfa, Il1b, Il12a, (M) and Mucin2 (N) of the Rd8- and WT-SPF mice.

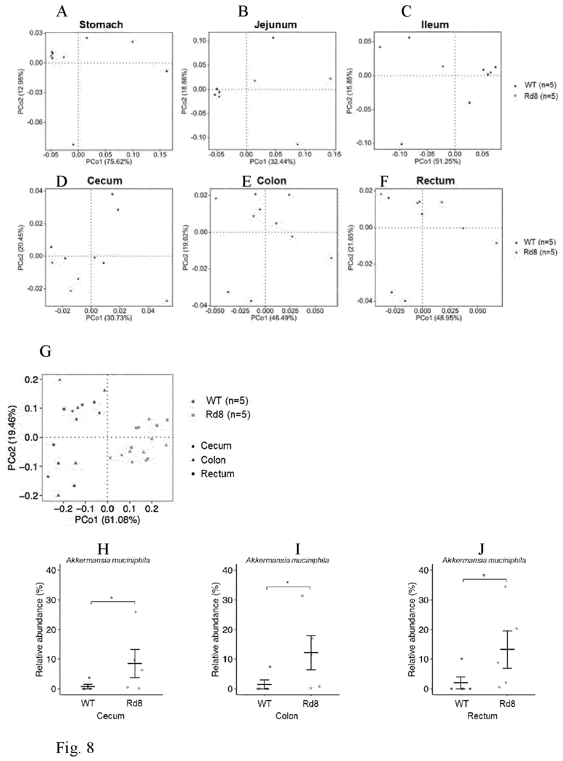

Fig. 8 is a microorganism species composition of a flora of different parts of a gastrointestinal tract of an Rd8-SPF mouse. Figs. 8A-F are principal coordinate analysis (PCoA) of a microorganism species composition of a gastrointestinal sample of WT (n=5) and Rd8 (n=5) mice based on a Bray-Curtis distance, including a stomach (Fig. 8A), a jejunum (Fig. 8B), an ileum (Fig. 8C), a cecum (Fig. 8D), a colon (Fig. 8E) and a rectum (Fig. 8F); Fig. 8G is PCoA distinguishing of a microorganism composition (H-J) of a lower digestive tract (the cecum, colon and rectum) of the Rd8 mouse and the WT-SPF mouse for comparing relative abundance of Akkermansia muciniphila in the cecum (H), colon (I) and rectum (J) of the Rd8 and WT mice. Data is represented as an average value ±SEM*P<0.05.

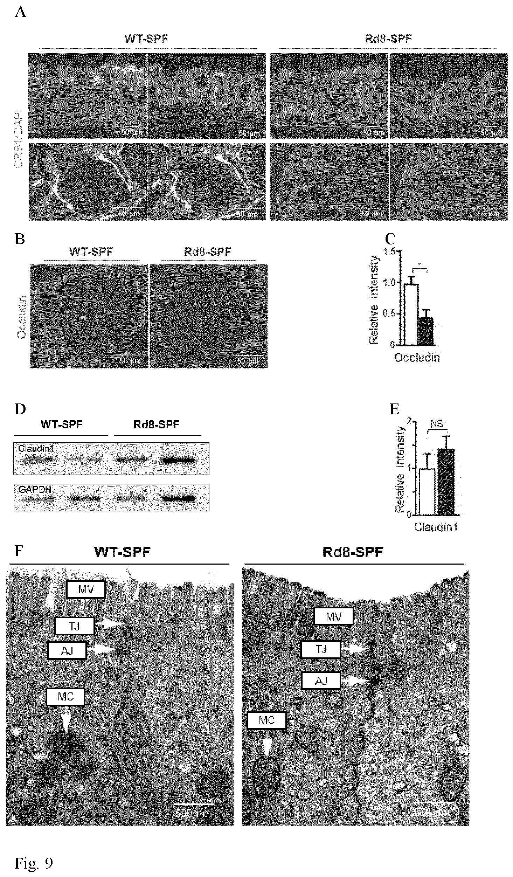

Fig. 9 is a tight junction and an adherent junction of a cecum of an Rd8 mouse. Fig. 9A is immunofluorescent staining of cecum intestinal cell CRB1 protein (green) of Rd8- and WT-SPF mice; Fig. 9B is immunofluorescent staining of cecum intestinal cell occludin (red) of the Rd8- and WT-SPF mice; Fig. 9C is statistic analysis *P<0.05 of relative intensities of claudin of the Rd8- and WT-SPF mice; Fig. 9D is westernblot test of expression of cecum Claudin1 of the Rd8- and WT-SPF mice; Fig. 9E is statistic analysis of relative intensities of Claudin1 of the Rd8- and WT-SPF mice, where NS represents no significance; and Fig. 9F is transmission electron microscope display that a normal tight junction and adherent junction exist in a cecum epithelium barrier , where MV is microvesicle, and MC is mitochondrion.

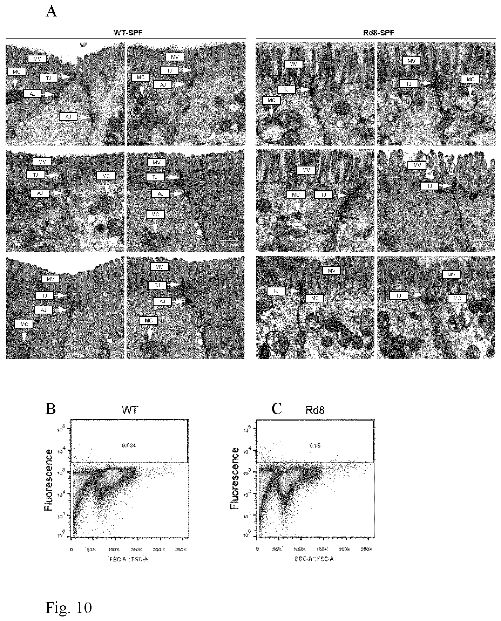

Fig. 10: Fig. 10A is transmission electron microscope observation of an adherent junction (AJ) between a colonic epithelium of Rd8- and WT-SPF mice and a tight junction (TJ), where MV is microvesicle, and MC is mitochondrion; and Fig. 10B is flow cytometer test of a frequency of fluorescence+bacteria/cells in peripheral blood of the Rd8 and WT mice.

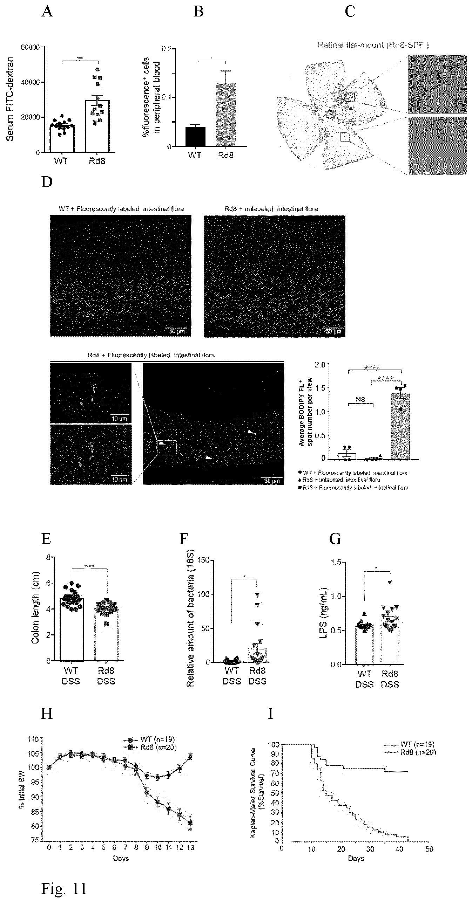

Fig. 11 is intestinal epithelium barrier function breakdown of an Rd8-SPF mouse. Fig. 11A displays that a serum fluorescence intensity of the Rd8 mouse is significantly higher than that of a WT mouse through an intestinal FITC-dextran permeability test ***P<0.001; Fig. 11B is comparison of a percentage of fluorescence+bacteria/cells in peripheral blood of the Rd8 and WT mice through a vanco-bodipy labeled fecal micropopulation transplantation method *P<0.05; Figs.11C-D are detection of vanco-bodipy+bacteria in a retinal lesion of the Rd8 mouse under a fluorescence microscope (C) and immunofluorescent staining (D); Figs. 11F-G are comparison of a colon length and serum bacteria 16srma (F) and bacteria LPS (G) levels of the Rd8 and WT mice 13d after DSS treatment ****P<0.0001; Fig. 11H is weights of the Rd8 (n=20) and WT (n=19) mice treated with 2.5%DSS by monitoring every day; and Fig. 11I is a Kaplan-Meier survival curve of the Rd8 (n=20) and WT (n=19) mice after being treated with 2.5%DSS.

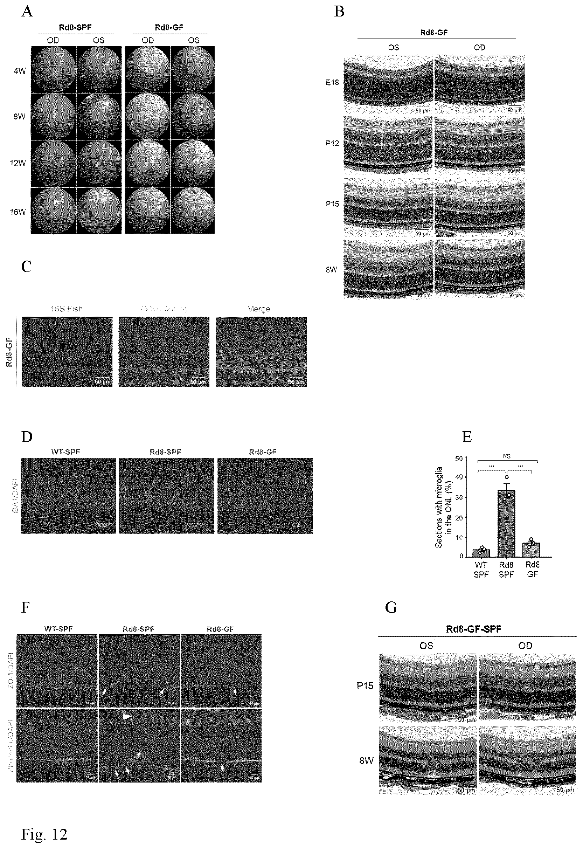

Fig. 12 is a retinal phenotype of Rd8 GF and Rd8 GF SPF mice. Fig. 12A is a representative eye fundus image of Rd8-SPF and Rd8 GF mice in in 4 weeks, 8 weeks, 12 weeks and 16 weeks; Fig. 12B is two-eye retinal histology observation of the Rd8-GF mouse in E18, P12, P15 and 8W; Fig. 12C is fluorescence in situ hybridization staining of retinal bacteria 16srDNA and vanco-bodipy of the Rd8-GF mouse; Fig. 12D is immunofluorescent staining of IBA1 (red) of retinae of Rd8-SPF, Rd8 GF and WT-SPF mice; Fig. 12E is a percentage of IBA1+microglial cell in ONL of Rd8-SPF, Rd8 GF and WT-SPF mice ***P<0.001, where NS represents no significance; Fig. 12F is immunofluorescent staining of ZO-1 (red) and Phalloidin (green) protein of retinae of Rd8-SPF, Rd8-GF and WT-SPF mice; and Fig. 12G is H&E staining detection of a retinal lesion of the Rd8-GF mouse (Rd8-GF-SPF mouse) raised in an SPF environment after bom in P15 and 8W.

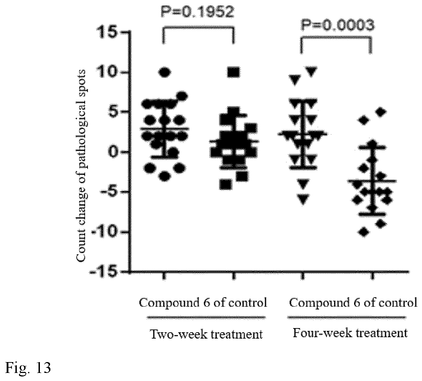

Fig. 13 is a count change of fundus light spots 2 weeks and 4 weeks after intragastric administration in an experiment of intragastric administration therapy of a Crb1^{rd8/rd8} mouse suffering from fundus degenerative retinopathy with an octyl gallate (compound 6).

### DETAILED DESCRIPTION OF THE INVENTION

The technical solutions in the examples of the present disclosure will be described clearly and completely below. In the following provided examples, only a modeling method of raising a Crb1 gene mutation mouse in an SPF environment is adopted, and it is determined that a model retina is infected with bacteria by proving existence of a local inflammatory reaction of the retina and existence of the bacteria in a lesion. It is further proved that the bacteria are from an intestinal tract through the following environment. This specific example does not exclude other modeling methods, for example, raising in an environment with more complex microorganism conditions, or a microorganism from the intestinal tract or a microorganism which is the same as the intestinal microorganism is administered to ocular tissue, so the eye tissue directly or indirectly makes contact with the aforementioned microorganism, and the like.

Apparently, the described examples are merely some rather than all of the examples of the present disclosure. All other examples obtained by those ordinarily skilled in the art based on the examples of the present disclosure without making creative efforts fall within the protection scope of the present disclosure.

### 1. Mouse

A C57BL/6N mouse (Crb1^{rd8/Rd8}, named an Rd8 mouse) and a C57BL/6J mouse (Crb1^{wt/wt}, named a wt mouse) carrying an Rd8 mutation are purchased from Beijing Vital River Laboratory Animal Technology Co., Ltd. and maintained in a specific pathogen free (SPF) condition of an animal facility of Zhongshan Ophthalmic center, Sun Yat-sen University. An animal facility of the First Affiliated Hospital, Sun Yat-sen University produces a germ-free (GF) RD8 mouse by using an embryo of a female RD8 mouse. The GF mouse is kept in a germ-free state, and a facility worker performs microorganism and parasite detection on a fecal sample every week so as to ensure sterility of the GF unit. Mouse genetic typing is performed as above (Mattapallil, et al., 2012). Crb1 genotypes of two mouse strains are verified (Fig. 4A). All animal environments are approved by the institutional animal care and use committee of the Zhongshan Ophthalmic center and conform to "Association for Research in Vision and Ophthalmology (ARVO) Animal Use Statement".

### 2. Method

### 2.1 Histochemistry

The mouse is killed through cervical dislocation, and eye balls are removed and are immobilized in a 4°C phosphate buffer solution (PBS) with 4% paraformaldehyde (PFA) for 24 h. A sample is washed three times with the PBS, dehydrated in a series of alcohols, dehydrated twice in xylene, then buried in paraffin, and continuously sliced in a 10 µm position with a slicer (RM 223; Leica, Wetzlar, Hesse-Darmstadt, Germany). A slice is subjected to hematoxylin and eosin (H&E) staining. An H&E image is obtained by Imager.Z2 (Zeiss).

Eyes are obtained and put in 4%PFA at a room temperature for 5 min, then dissected and fixed with an eye patch for 45 min, colon tissue is obtained, immobilized in 4%PFA at the room temperature for 4 h and flushed with the PBS, then the separated eye patch is infiltrated with 30% sucrose and stays with a colon overnight for cryopreservation, an OCT compound is embedded (Cat. 4583; SAKURA, USA), and it is stored before a -80°C slice. The slice is cut in a 12 µm position for all immunostaining purposes.

The tissue slice is blocked with 10% donkey serum/PBST (0.1%tritonx-100/PBS) for 30 minutes and then incubated overnight with a primary antibody at 4°C. After being washed with the PBST, the slice is incubated with a secondary antibody combined with a fluorescent dye and immobilized with Fluoromount-G (Southern Biotech, Birmingham, AL, USA). Phalloidin (A12379;Thermo-Fisher) staining is performed by using the same immunohistochemistry method, except for missing the secondary antibody. ApoTome (Zeiss) is equipped by using a Zeiss confocal microscope (Zeiss LSM880; Zeiss,Oberkochen, Germany) and Imager.Z2. Main antibodies used in this study are: anti-Crb1 (PA5-66373, ThermoFisher; 1:50), anti-Iba1 (ab178846, Abeam; 1:500), anti-ZO-1 (61-7300, ThermoFisher; 1:500), anti-Occludin (OC-3F10, Invitrogen; 1:200), AlexaFluor 488 phalloidin (A12379, ThermoFisher; 1:500).

### 2.2 Fundus photography

The mouse is anaesthetized and pupils are dilated. A cornea remains moist by applying hydroxypropyl methylcellulose eye drops regularly. A mouse fundus photo is obtained by using a Micron IV mouse fundus camera (Phoenix Research Laboratories, Inc., Pleasanton, CA, USA).

### 2.3 RNA-seq analysis of retinal superior and inferior parts

Total RNA is extracted from the retinal superior and inferior parts by using MasterPure^{™} complete DNA and RNA purification kit (epicentre). An RNA concentration is measured by using a Qbit-RNA-HS analytical kit. A sequencing library conforms to a standard agreement provided by a manufacturer and prepared by using VAHTS^{™} Total RNA seq (H/M/R) library preparation kit (Vazyme, China), and performs sequencing in an MGISEQ 2000RS platform.

Quality control evaluation is performed on raw reads first from FastQC (v0.11.8) and cutadapt (v1.15). Clean reads are aligned with a mouse genome (mm10) by using HISAT2 (v2.1.0). Gene expression data is introduced into a DESeq2 packet of R software (v3.6.1) for differential expression analysis. Differentially expressed genes (DEGs) are introduced into ingenuity pathway analysis (IPA) for function enrichment analysis.

### 2.4 Metagenomics sequencing

A retinal sample is collected, and DNA is extracted by using a MasterPure^{™} complete DNA and RNA purification kit (epicentre). Contents of the stomach, jejunum, ileum, cecum, colon and rectum are collected, and DNA is extracted by using a QIAampPowerFecal DNA kit (QIAGEN). After a concentration is measured, sequencing library preparation is performed on DNA by using a VAHTS^{™} MGI universal DNA library preparation kit (Vazyme, China) according to a standard solution provided by a manufacturer. Metagenomics sequencing is performed by using MGISEQ-2000RS. Quality filtering is performed on raw reads through Trimmomatic (v0.36) and PRINSEQ (v0.20.4). A mouse read (v0.6.1) is deleted by using KneadData (https://bitbucket.org/biobakery/kneaddata). A non-mouse-clear read is mapped into a pre-built MiniKraken database by using Kraken 2 (v2.0.9). Classification results are screened by using a confidence coefficient 0.20. A negative blank control and the sample are processed together. All species existing in the negative blank control are removed.

### 2.5 FITC-dextran in vivo intestinal permeability test

The in vivo progressive test is performed by using an FITC labeled dextran method for evaluating a barrier function. Food and water of the previous night are taken out, and the 8-week-old mouse of every 100 grams (weight) takes orally 50 milligrams of FITC labeled dextran (FD-70;Sigma-Aldrich). A serum is collected 5 h after drug administration, and a fluorescence intensity of each sample is measured (excitation, 492 nm; emission, 525 nm).

### 2.6 Flow cytometry

The WT and Rd8 mice fast overnight with intragastric administration of 1 × 109 E. Bacillus coli (designed as consistently expressing RFP). 6 hours after intragastric administration, euthanasia is performed on the mice, and 400 µL of peripheral blood is gently transferred to a test tube containing 4 mL of an ACK lysis buffer solution (Gibco, USA) and cultured in RT for 3-5 minutes. After being centrifuged in 300 × g for 5 minutes, cells are immobilized and infiltrated (Cytofix/perm solution, BD Biosciences, USA), and the analysis is performed through the flow cytometer (MACS Quant Analyzer 10, MiltenyiBiotec, Germany).

### 2.7 Tissue preparation through an electron microscope

The colon and eyes are collected immediately after euthanasia and immobilized for 1 h at a room temperature in a phosphate buffered glutaraldehyde-paraformaldehyde solution. The colon is cut into 2 mm blocks. An anterior eye segment is cut off, and a posterior eye segment is cut into 2 mm×2 mm blocks. Tissue obtained after dissection is put in a fresh stationary liquid for 12 h, immobilized with 1% osmium tetroxide, dehydrated and then buried in epon-resin. Staining is performed with toluidine blue under an optical microscope, and a region of interest is screened in advance on a micron-thickness slice. Then an 80 nm ultrasonic image is collected, and counterstaining is performed with uranyl acetate and lead citrate. An ultrasonic slice is observed by using a transmission electron microscope.

### 2.8 Fluorescence in situ hybridization (FISH)

This study uses the following oligonucleotide probe: EUB338, 5'-GCTGCCTCCGTAG-GAGT-3' (Amann, et al., 1990). A 5' end of the probe has a primary amino group, and Tetramethyl rhodamine isothiocyanate and the amino group are in covalent binding. A dye oligonucleotide conjugate (100 µM) is stored at -20°C.

A pre-fixed retinal slice is flushed three times with a DEPC processed PBS. After being processed with the 0.2%Triton X-100/DEPC processed PBS, the slice and the probe (500 nM) are hybridized overnight at 37°C and mounted by using Fluoromount-G.

### 2.9 Real-time quantitative PCR detection of a gene expression level in colon tissue

Fresh mouse colon tissue (~1 cm) is subjected to quick freezing in liquid nitrogen, ground and lysed by using an RNA extraction lysis buffer solution. Total RNA is purified by using a Qiagen RNeasy Plus kit and reversely transcripted into cDNA by using a Takara PrimeScript RT kit and a gDNA eraser. qPCR detects expression levels of corresponding genes. Data is standardized as β-actin.

### 2.10 QPCR quantitative detection of a 16srma gene level in plasma

13 days after 2.5%DSS processing, plasma is separated from whole blood of WT and Rd8 mice. About 50 µL of plasma is used for using a MasterPure isolation total nucleic acid^{™} complete DNA and RNA purification kit (epicentre, USA). Settled nucleic acid is dissolved in 20 µL of nuclease-free water. qPCR analysis (ChamQ-SYBR-Color-qPCR-Master-Mix, Vazyme, China) is performed by using a LightCycler 96 system (Roche of USA). DNA concentrations in all samples are extremely low, so each sample with the equal volume is used as a template (4 µL in 20 µL). A total bacterial load is measured by using the following universal 16S rRNA gene primers: 27F 5'- AGAGTTTGATCCTGGCTCAG-3', and 534R 5'- GCATTACCGCGGCTGCTGG-3'.

### 2.11 Enzyme linked immunosorbent assay (ELISA)

Each sample is measured directly by using 100 µL of plasma. An LPS concentration of the plasma is measured by using an enzyme linked immunosorbent kit (SEB526Ge; Cloud-Clone Corp., USA).

### 2.12 Fluorophore vanco-bodipy labeling of colonization of intestinal bacteria in the Rd8 mouse body

A fresh fecal sample is collected in a 50 ml cone-shaped tube with bacteria-free 1xPBS and rotated till being homogeneous. Contents are filtered by using a 0.22 µm filter (Millipore) to remove fecal residues, centrifuged to obtain an intestinal flora and then incubated with vanco-bodipy in RT for 30 minutes. The vanco-bodipy labeled intestinal bacteria are administered to the mouse in PBS in 1×10⁸cfu/ intragastrically. 24 hours after intragastric administration, a mouse retinal slice is observed.

### 2.13 Experimental study of dextran sodium sulfate (DSS) induced colitis

A mouse intestinal inflammation is induced through long-term oral administration of 2.5%DSS (MW 36000-50000d, Yeasen, China) through drinking water. The weight is monitored every day from Day 0 to Day 13, the mouse is killed on Day 13, and a colon length is measured. For survival analysis, the mouse is allowed to drink 2.5%DSS freely in drinking water for 43 days, and a death rate state of the mouse is monitored every 24 hours during the 43 days.

### 2.14 Intestinal symbiont depletion

A broad-spectrum antibiotic mixture of ampicillin (A; 1 g/L; Sigma), metronidazole (M; 1 g/L; neomycin (N; 1 g/L; Sigma) and vancomycin (V; 500 mg/L; a pregnant female mouse is fed with Sigma (AMNV) in drinking water, and a young mouse continues to be fed after weaning. A mouse in control is put in a conventional facility on the same rack.

### 3. Example

### Example 1: modeling

Retinal microenvironment characteristics of Crb1rd8/rd8 (rd8) and Crb1wt/wt (C57BL/J, named wt) mice are observed (Fig. 1A). A phenotype of the Rd8 mouse raised in a specific pathogen free (Rd8-SPF) environment is observed, it is discovered that the mouse shows all typical retinal degenerations, including white spots in an inferior eye and inferior nasal quadrant under examination of an ophthalmofundoscope (Fig. 1B), as well as progressive retinal dysplasia (folds and Weiss ring) and histological retinal degeneration (H&E staining) (Fig. 2A). A result shows that 7 mice out of 32 Rd8 mice have mild retinal dysplasia before birth (E18) (Fig. 2A and Fig. 2B). In addition, all abnormalities in E18 occur unilaterally (Fig. 2A and Fig. 1C), typical lesions such as retinal folds and Weiss ring may be observed in all retinae of the Rd8 mice in P12 (before opening eyes), P15 (after opening eyes) and 8-week old (8W), but no lesion occurs to any one of WT-SPF mice (Fig. 2A and Fig. 2B). Data shows that retinal abnormality of the Rd8 mice has occurred as early as an embryo in E18, and an intraocular environment can drive a retinal phenotype under a Crb1 mutation genetic influence.

### Example 2: detection of whether an in-lesion pathogen exists in a mouse (Rd8-SPF) retina after modeling and a related local immune response

Transcriptomic analysis is performed by using an RNA-seq technology for comparing a gene expression profile of superior (without lesions) and inferior (with lesions) regions of the Rd8-SPF mouse (Fig. 3A). After removing a gene with a differential expression pattern (2-times changes and P<0.05) between retinal superior and inferior regions of the WT-SPF mouse, it is shown that 179 genes (DEG) have an expression difference (P<0.05) of at least two times between the retinal superior and inferior regions of the Rd8-SPF mouse (Fig. 3B and Fig. 2C). In the Rd8-SPF mouse, most of these 179 DEGs are highly expressed in a damaged inferior half region (Fig. 3C). Function analysis for these DEGs by using an ingenuity pathway analysis (IPA) tool shows that genes participating in bacteria and virus recognition are significantly enriched in all DEGs highly expressed in the Rd8-SPF mouse retinal lesion (Fig. 2D). Importantly, the first two genes Ncf4 and Slamf1 with the largest lesion increasing multiple are regulatory factors of phagocyte antibacterial reaction. Data shows that the in-lesion pathogen and the related local immune response exist in the Rd8 retina, and these results are combined with a rich expression of a gene with a proinflammatory function (Fig. 2D) and infiltration of IBA1+microglial cell towards a retinal lesion region (Fig. 2E).

### Example 3: detection of whether bacteria exist in a retinal lesion part of the mouse after modeling

Metagenomic analysis is performed on retinal tissue of the WT-SPF (n=5, age=4 weeks) and Rd8-SPF (n=4, age=4 weeks) mice. It is discovered through the analysis that bacteria DNA contents in the retinae of the WT and Rd8 mice are extremely low, and after all quality control and decontamination steps, no virus and fungus DNA is detected. However, as shown in Fig. 4A, the principal coordinate analysis (PCoA) shows that bacteria compositions of the WT-SPF and Rd8-SPF retinae are significantly different. Importantly, further metagenomic analysis for the retinal superior and inferior regions of the Rd8-SPF (n=4, age=4 weeks) mouse shows that seven types of bacteria are significantly enriched in the retinal inferior region, including hadrus, Bifidobacterium pseudocatenulatum, Megamonasfuniformis, Nitrosoomonas Is79A3, Oscillibacter, Tatumella sp.TA1 and thiobacillus denitrificans (Fig. 4B). In order to directly observe whether bacteria exist in the retinal lesion, fluorescence in situ hybridization is performed on the Rd8-SPF mouse (4-week-old) (FISH) and vanco-bodipy staining analysis, especially for staining a cell wall of gram positive bacteria. As shown in Fig. 4C, the bacteria are found only in the lesion part but not in a normal retinal region. Existence of the in-lesion bacteria is further verified through the transmission electron microscope (TEM) (Fig. 4D). Data shows that the bacteria may be found in the retinal lesion of the Rd8-SPF mouse.

### Example 5: proving that a major defect of the Rd8-SPF mouse retina is an outer blood-retinal barrier

Immunofluorescent staining data proves that CRB1 protein expression of an Rd8 retinal outer limiting membrane is reduced or missing (Fig. 5A). Data proves that a weak expression of CRB1 protein ma be found on a Bruch membrane (Fig. 5A). Through the transmission electron microscope, it is discovered that in the retinal lesion part of the Rd8-SPF mouse, an adherent junction on the outer limiting membrane is broken, which is related to out-migration of an outer nuclear layer (Fig. 5B and Fig. 6A). Examination of the transmission electron microscope further shows adherent junction dissolution in a basal lamina of a retinal pigment epithelium (RPE), a distorted chorion capillary basal lamina and collagen layer disturbance between them, and these two cases are not found in any WT-SPF mouse (Fig. 5C and Fig. 6B). This is related to Bruch membrane breaking and significant decreasing of a Bruch membrane thickness of the Rd8-SPF mouse (Fig. 5C, Fig. 6B and Fig. 5D). In another aspect, no change of tight junction between retinal capillary endothelial cells and retinal pigment epithelial cells is found in examination of the transmission electron microscope. Therefore, a result shows that the major defect of the Rd8-SPF mouse retina is the outer blood-retinal barrier but not an inner blood-retinal barrier.

### Example 6: performing metagenomic analysis on micropopulation of different intestinal parts

All of the seven types of bacteria discovered in the Rd8 retina (Fig. 4B) are previously gastrointestinal (GI) bacteria. Thus, the metagenomic analysis is performed on the micropopulation of different parts (including a stomach, a jejunum, an ileum, a cecum, a colon and a rectum) of a gastrointestinal tract of the WT-SPF (n=5) and Rd8-SPF (n=5) mice (Figs. 8A-F). A microorganism composition in a lower digestive tract of the Rd8-SPF mouse is significantly different from a microorganism composition in the WT-SPF mouse (Fig. 8G). In WT and Rd8 intestinal flora of the cecum (Fig. 8H), colon (Fig. 8I) and rectum (Fig. 8J), Akkermansia mucinphila is determined as the most different bacteria species though it is not traced in the Rd8 retina (Fig. 4B). Actually, the seven types of bacteria in the Rd8 retina are discovered in the lower digestive tract of the WT and Rd8 mice, and the cecum has the most bacteria (Fig. 7A). However, they are hardly detected in an upper digestive tract of the Rd8 mouse. These data shows that these retinal bacteria exist in a gastrointestinal flora of the Rd8-SPF but not of the WT-SPF mouse.

An expression of CRB 1 protein in cecum intestinal cells of a wild-type mouse is identified through immunofluorescent staining, but the expression thereof is significantly weakened in the Rd8 mouse (Fig. 9A). In addition, it is observed that in the Rd8 mouse cecum, Occludin (Figs. 9B-C) expression is significantly missing, and Claudin1 (Figs. 9D-E) expression is not significant. A further examination of the transmission electron microscope shows that a normal tight junction and adherent junction exist in a cecum epithelium barrier (Fig. 9F).

### Example 7: detecting whether a barrier defect exists in the Rd8 mouse colon

Similar to the result of the cecum, it is discovered that the CRB1 protein has a significant expression in a top surface and a bottom surface of a colon intestinal epithelial cell (Fig. 7B). Missing of the CRB1 protein is related to significant decreasing of expressions of Phalloidin (Fig. 7C) and Occludin (Fig. 7D and Fig. 4E), but not related to an expression of Claudin1 (Fig. 7F and Fig. 4G) protein. Importantly, through the transmission electron microscope, it is observed that an adherent junction between colon epitheliums in most of cells cannot be positioned, and a tight junction in the Rd8 mouse seems to be normal (Fig. 7H and Fig. 6A). In the Rd8 mouse colon epitheliums, in addition to the junction change, mitochondrial vacuolation is further observed. Though there is no change of the number of epithelial microvilli (Fig. 7I), the microvilli of the Rd8 mouse become longer (Fig. 7J) and thinner (Fig. 7K), and compared with the WT mouse, the total number of integral adherent junctions is reduced significantly (Fig. 7L). An ultrastructure change of the junctions and the microvilli is also accompanied with significant increasing (Fig. 7M) of Il12a expression in a colon wall, but no change occurs to expressions of Tnfa, Il1b and mucin2 (Fig. 7N). To sum sup, the data shows that the Rd8 mouse has a colon epithelium barrier defect and a related colon wall inflammation.

### Example 8: examining whether an intestinal epithelial barrier defect causes change of Rd8 mouse intestinal permeability and causes micropopulation to migrate towards peripheral blood flow and retinal tissue

An intestinal FITC-dextran permeability test is performed on the WT and Rd8 mice. As shown in Fig. 11A, compared with a fluorescence intensity in WT mouse blood, 5 hours after administering FITC dextran, fluorescence in peripheral blood of the Rd8 mouse is significantly increased. Besides, a Vancompy labeled fecal micropopulation transplantation test discovers that fluorescence+ bacterialcells in the peripheral blood of the Rd8 mouse are significantly increased, compared with 24 hours after fecal transplantation of the WT mouse (Fig. 10B and Fig. 11B). Importantly, Vancomopy+bacteria appearance may be detected in the retinal lesion of the Rd8 mouse (Fig. 11C), but the WT retinal lesion has none (not displaying data), which is further proved through immunofluorescent staining of the iRd8 mouse retinal tissue (Fig. 11D). These data shows that intestinal permeability for polysaccharide molecules and bacteria cells is increased, resulting in that the bacteria are transferred to blood flow and the retinal lesion from an intestinal lumen.

### Example 9: a response experiment of the Rd8 to an intestinal stress in a case that the intestinal permeability is increased

The WT and Rd8 mice are exposed in drinking water containing 1.5% dextran sodium sulfate (DSS), which causes mild colitis of the WT mouse. 13 days after DSS therapy, it is discovered that a colon length of the Rd8 mouse is significantly less than a wild-type mouse (Fig. 11E), and the number of bacteria in blood of the Rd8 mouse is greater than the number of bacteria in blood of the wild-type mouse, which is indicated by serum levels of bacteria 16S rRNA (Fig. 11F) and bacteria LPS (Fig. 11G).

### Example 10: a reverse effect of bacteria on a retinal phenotype of the Rd8 mouse

Though in-lesion bacteria caused by breakdown of an outer blood-retinal barrier and an intestinal epithelial barrier are discovered in the Rd8 mouse retina, whether these bacteria are a reason or an outcome of Rd8 mouse retinal degeneration is not clear yet. Thus, the Rd8 mouse is reseparated in a germ-free (GF) condition, and whether a retinal degeneration phenotype of the mouse changes is detected. As shown in Fig. 12A, a retinal lesion (white spot) discovered in the Rd8-SPF mouse is hardly observed in the germ-free Rd8 (Rd8 GF) mouse. Retinal histology of the Rd8-GF mouse shows that normally developed retinal tissue (Fig. 12B) does not have typical lesions (Fig. 2A and Fig. 1C) discovered in the Rd8-SPF mouse, which proves that the Rd8-GF mouse is free of the retinal degeneration. The fact that no retinal bacteria are detected in the Rd8 GF mouse (Fig. 12C) is related to sudden reducing of microglial cells in a retinal outer nuclear layer (ONL) (Fig. 12D and Fig. 12E) in the Rd8 GF mouse, though breakdown of an outer limiting membrane displayed by ZO-1 and Phalloidin protein staining is discovered both in the Rd8-SPF mouse and Rd8 GF mouse (Fig. 12F). Besides, when the Rd8 GF mouse (Rd8 GF SPF mouse) is raised in an SPF environment after birth, the retinal lesion appears again (Fig. 12G). These data supports the fact that Rd8 mouse retinal degeneration is bacterially dependent.

### Example 11: pharmacodynamic evaluation of intragastric administration therapy for a Crb1^{rd8/rd8} mouse suffering from a fundus degenerative retinopathy with octyl gallate (compound 6)

### Sample for test

| Name of sample for test | Source | Article number | Batch number | Physicochemica l property | Preservation condition |
|---|---|---|---|---|---|
| Compound 6 | Sigma-Aldrich | 48700 | STBJ0496 | White powder | Room temperature |

When a sample for test is prepared, 4 mL of castor oil which is already autoclaved is measured, 1.2 g of compound 6 powder is weighted, 1-2 mL of castor oil is added, full grinding is performed, a ground drug and residual castor oil are all transferred to a 50 mL tube with 36 mL ultrapure water to be oscillated and uniformly mixed for still standing overnight, so a compound 6 suspension with a concentration being 30 mg/mL is prepared, and the prepared drug liquid is labeled, saved at a normal temperature and used up within a month. theoretical weighing sample quantity (mg) = theoretical cencentration (mg/mL) of the sample for test × preparation volume (mL)

### 1.2 Information of an experimental animal

Species and strains: Crb1^{rd8/rd8} mouse
Grade: SPF grade
Quantity and gender: 18 mice, either gender
Weight range: 16-24 g
Supply unit: Gempharmatech Co., Ltd.

### 1.3 Selection of an experimental animal

When drug administration starts, a healthy one-month-old Crb1^{rd8/rd8} mouse is selected for an experiment. When the animal experiment starts, check of a slit lamp is performed, a mouse having a cataract disease is not allowed in the experiment; and a fundus examination is performed, and both eyes of a mouse, one eye fundus of which does not have a light spot lesion, are not allowed in the experiment.

### 1.4 Experiment design

### Group and dose

Animals are hierarchically divided according to weights and then randomly divided into a model control group and a compound 6 group. Design details are shown in the following table:

| Group | Dosage of administration | The number of eyes |
|---|---|---|
| Control group | 0 | 18 |
| Compound 6 group | 0.3 | 18 |

Information related to drug administration and dosage design
Information related to drug administration
Drug administration route and method: intragastric administration, 10.0 mL/kg weight
Drug administration frequency and time limit: once every other day, for 4 weeks
Dosage design
Dosage of the compound 6 is 0.3 g/kg weight for the mouse and is converted according to previous clinical medication.

### Experiment method and steps

One-month-old Crb1^{rd8/rd8} mice are randomly divided into the model control group and the compound 6 drug administration group. A solvent or a compound 6 drug is administered intragastrically to the animals, a dosage of the compound 6 is 0.3 g/kg, once every other day, continuous drug administration for 4 weeks. The number of fundus light slots 2 weeks and 4 weeks after the mouse is administered intragastrically is recorded for analysis and comparison.

### 1.5 Data processing and statistic analysis

A change of the number of fundus light spots of the mice at each time point after drug administration is used as a parameter, and SPSS software is used for the statistic analysis. A statistical result is represented by *x̅* ± *s*; and an inspection level=0.05, and an average of experiment data and statistically processed data remain 2 decimal places.

### 1.6 Experiment result

The mice in the model control group have fundus light spots in the beginning of drug administration, and 2 weeks and 4 weeks after drug administration, which indicates that fundus degenerative retinopathy occurs to the model animals. Compared with the model control group, the number of the light spots changes to 1.31 ± 3.18 2 weeks after intragastric administration of the compound 6, which has no statistic difference compared with 2.88 ± 3.41 of the change of the number of spots of the model control group. The number of fundus light spots of the C57BL/6N mouse 4 weeks after intragastric administration changes to -3.63 ± 4.06, which has a statistic difference compared with 2.24 ± 4.01 of the change of the number of the spots of the model control group (Fig. 13). Detailed results are shown in the following table:

| Group | The number of eyes | Change of spots 2 weeks after drug administration | Change of spots 4 weeks after drug administration |
|---|---|---|---|
| Model control group | 18 | 2.88 ± 3.41 | 2.24 ± 4.01 |
| Compound 6 group | 18 | 1.31 ± 3.18* | -3.63 ± 4.06** |

| | | | |
|---|---|---|---|
| Note: compared with the model control group, *P=0.1518, and **P=0.0005. | | | |

In Week 2 and Week 4 of the experiment, fundus light spots of 2/18 and 1/18 eyes in the compound 6 group cannot be observed clearly due to slight decrease of crystalline lens transparency caused by anesthesia, which is not allowed for statistical count. At the same time, in Week 4 of the experiment, 1/18 eye in the model control group also has decrease of crystalline transparency, so the fundus light spots cannot be observed clearly, and correlation between slight decrease of the crystalline lens transparency and use of the compound 6 is not excluded. A document reports that after the mice are anaesthetized, transparency of a crystalline lens may be decreased, lens abnormalities are common in the Crb1^{rd8/rd8} mice, transparency of 67% of an anterior lens capsule is decreased, and when decrease of the transparency affects observation of the fundus, it is not allowed for statistical count.

The compound 6 can effectively inhibit the fundus degenerative retinopathy of the Crb1^{rd8/rd8} mice in the dosage of 0.3 g/kg.

The above examples are merely for describing the technical solutions of the present disclosure but not for limiting them. Although the present disclosure has been described in detail with reference to the examples, those ordinarily skilled in the art are to understand that changes may still be made for the technical solutions recorded in the above examples, or some or all technical features may be replaced equivalently, and these changes or replacements do not cause essence of the corresponding technical solutions to depart from the scope of the technical solutions of the examples of the present disclosure.

## Claims

1. A treatment method for retinal degeneration, comprising administering an inhibitor or microbicid for a microorganism to eyes or a gastrointestinal tract of a patient.

2. The treatment method according to claim 1, wherein the retinal degeneration is progressive retinal degeneration; preferably, the retinal degeneration is inherited retinal degeneration; more preferably, the inherited retinal degeneration comprises Leber congenital amaurosis (LCA), retinitis pigmentosa (RP), early-onset rod-cone cytodystrophy, rod-rod dystrophy, congenital stationary night blindness and colour blindness and Stargardt disease.

3. The treatment method according to claim 1, wherein the microorganism is one or a combination of two or more of bacteria, archaebacteria, protists, fungi or viruses.

4. The treatment method according to claim 3, wherein the microorganism is the bacteria, and the bacteria are selected from: one or two or more of *Anearostipes*, *Bifidobacterium*, *Megamonas*, *Nitrosomonas*, *Oscillibacter*, *Tatumella*, *Thiobacillus* sp., *Clostridium*, *Acinetobacter*, *Streptococcus*, *Mannheimia*, *Fibrobacter*, *Prevotella*, *Campylobacter*, *Actinomyces*, *Hymenobacter*, *Escherichia*, *Tissierella, Klebsiella*, *Porphyromonas*, *Azospira*, *Aquimarina*, *Achromobacter*, *Acidithiobacillus*, *Burkholderia*, *Marinobacter*, *Treponema*, *Actinosporangium*, *Vibrio*, *Ruminococcus*, *Methanobrevibacter*, *Shigella*, Frankia, *Streptomyces*, *Anaeroplasma* and *Coprococcus.*

5. The treatment method according to claim 4, wherein the bacteria are selected from: Anearostipes hadrus, Bifidobacterium pseudocatenulatum, Nitrosomonas sp.Is79A3, Oscillibacter valericigenes, Tatumella sp.TA1, Megamonas funiformis, Thiobacillus denitrificans and Akkermansia muciniphila.

6. The treatment method according to claim 1, wherein the inhibitor or microbicid for the microorganism comprises a compound, polypeptide, a polynucleotide, a natural plant or a natural plant extract.

7. The treatment method according to claim 1, wherein the inhibitor or microbicid for the microorganism is an antibiotic, preferably, the antibiotic is one or two or more of a β-lactam antibiotic, an aminoglycoside antibiotic, a tetracycline antibiotic, a chloramphenicol antibiotic, a macrolide antibiotic, a glycopeptide antibiotic, a quinolone antibiotic, a nitroimidazole antibiotic, a rifamycin antibiotic, an echinocandin antibiotic, a polyene antibiotic, a pyrimidine antibiotic, an allylamine antibiotic, an azole antibiotic, and other antibiotics.

8. The treatment method according to claim 1, wherein the inhibitor or microbicid for the microorganism is a compound of a formula I (e.g., a formula I-1, a formula I-2, a formula I-3, a formula I-4, and a formula I-5), a formula II (e.g., a formula II-1, a formula II-2, a formula II-3, a formula II-4, a formula II-5, a formula II-6, a formula II-7, a formula II-8, a formula II-9, and a formula II-10), a formula III (e.g., a formula III-1, a formula III-2, and a formula III-3), a formula IV-1 or IV-2 (e.g., a formula IV-3, a formula IV-4, a formula IV-5, and a formula IV-6), a glycoside (e.g., a formula V), or a pharmaceutically acceptable salt or ester thereof, wherein an aglycone of the glycoside is a phenolic compound, a flavonoid, coumarin, benzoic acid, or a sterol, wherein the formula I, the formula II, the formula III, the formula IV-1, the formula IV-2, the formula V, and subformulae thereof are defined in specification.

9. The treatment method according to claim 8, wherein the inhibitor or microbicid for the microorganism is compounds 1-8, and the compounds 1-8 have the following chemical structures: 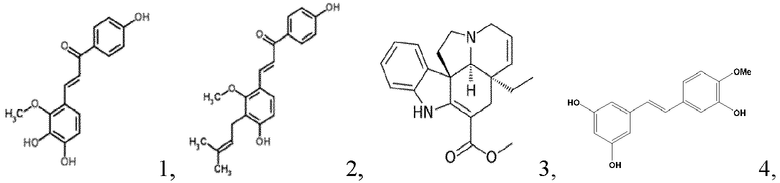 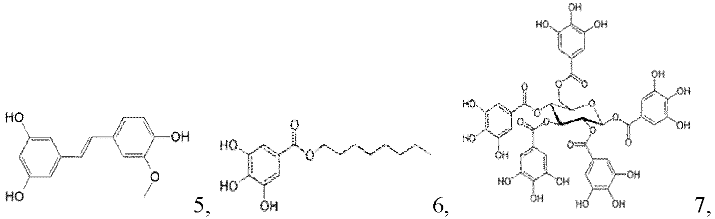 and

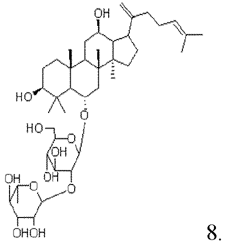

10. The treatment method according to claim 1, wherein the inhibitor or microbicid for the microorganism is a natural plant or a natural plant extract or a combination of the natural plant and the natural plant extract, preferably, the natural plant is selected from one or a combination of two or more of radix paeoniae alba, fructus forsythiae, fructus aurantii, rhizoma rehmanniae, dried tangerine peel, pseudo-ginseng, turtle shells, roots of kirilow rhodiola, dried rehmannia roots, coix seeds, semen cassiae and liquorice.

11. The treatment method according to claim 1, wherein the inhibitor or microbicid for the microorganism is a composition comprising turtle shells, roots of kirilow rhodiola, dried rehmannia roots, coix seeds, radix paeoniae alba, fructus forsythiae, semen cassiae and liquorice, preferably, the composition comprises, by weight, 5-15 parts the turtle shells, 10-30 parts the roots of kirilow rhodiola, 5-15 parts the dried rehmannia roots, 5-15 parts the coix seeds, 8-20 parts the radix paeoniae alba, 5-15 parts the fructus forsythiae, 5-15 parts the semen cassiae and 5-15 parts the liquorice, more preferably, the composition comprises 10 parts the turtle shells, 20 parts the roots of kirilow rhodiola, 10 parts the dried rehmannia roots, 10 parts the coix seeds, 12 parts the radix paeoniae alba, 10 parts the fructus forsythiae, 10 parts the semen cassiae and 9 parts the liquorice.

12. The treatment method according to claim 1, wherein administering to the eyes of the patient comprises administering to aqueous humor, a suspensory ligament, a ciliary body and ciliary muscle in an anterior chamber of each eye of the patient as well as to vitreous humor, a retina, a choroid membrane, an optic nerve, a crystalline lens or an iris in a posterior chamber of each eye of the patient.

13. The treatment method according to claim 1, wherein administering to the gastrointestinal tract of the patient comprises administering to a stomach, a jejunum, an ileum, a cecum, a colon or a rectum of the patient.

14. Use of an inhibitor or microbicid for a microorganism in the preparation of a drug for treating retinal degeneration.

15. The use according to claim 14, wherein the retinal degeneration is progressive retinal degeneration; preferably, the retinal degeneration is inherited retinal degeneration; more preferably, the inherited retinal degeneration comprises Leber congenital amaurosis (LCA), retinitis pigmentosa (RP), early-onset rod-cone cytodystrophy, rod-rod dystrophy, congenital stationary night blindness and colour blindness and Stargardt disease.

16. The use according to claim 14, wherein the microorganism is one or a combination of two or more of bacteria, archaebacteria, protists, fungi or viruses.

17. The use according to claim 16, wherein the microorganism is the bacteria, and the bacteria are selected from: one or two or more of Anearostipes, Bifidobacterium, Megamonas, Nitrosomonas, Oscillibacter, Tatumella, Thiobacillus sp., Clostridium, Acinetobacter, Streptococcus, Mannheimia, Fibrobacter, Prevotella, Campylobacter, Actinomyces, Hymenobacter, Escherichia, Tissierella, Klebsiella, Porphyromonas, Azospira, Aquimarina, Achromobacter, Acidithiobacillus, Burkholderia, Marinobacter, Treponema, Actinosporangium, Vibrio, Ruminococcus, Methanobrevibacter, Shigella, Frankia, *Streptomyces*, Anaeroplasma and Coprococcus.

18. The use according to claim 17, wherein the bacteria are selected from:
Anearostipes hadrus, Bifidobacterium pseudocatenulatum, Nitrosomonas sp.Is79A3,
Oscillibacter valericigenes, Tatumella sp.TA1, Megamonas funiformis, Thiobacillus denitrificans and Akkermansia muciniphila.

19. The use according to claim 14, wherein the inhibitor or microbicid for the microorganism comprises a compound, polypeptide, a polynucleotide, a natural plant or a natural plant extract.

20. The use according to claim 14, wherein the inhibitor or microbicid for the microorganism is an antibiotic, preferably, the antibiotic is one or two or more of a β-lactam antibiotic, an aminoglycoside antibiotic, a tetracycline antibiotic, a chloramphenicol antibiotic, a macrolide antibiotic, a glycopeptide antibiotic, a quinolone antibiotic, a nitroimidazole antibiotic, a rifamycin antibiotic, an echinocandin antibiotic, a polyene antibiotic, a pyrimidine antibiotic, an allylamine antibiotic, an azole antibiotic, and other antibiotics.

21. The use according to claim 14, wherein the inhibitor or microbicid for the microorganism is a compound of a formula I (e.g., a formula I-1, a formula I-2, a formula I-3, a formula I-4, and a formula I-5), a formula II (e.g., a formula II-1, a formula II-2, a formula II-3, a formula II-4, a formula II-5, a formula II-6, a formula II-7, a formula II-8, a formula II-9, and a formula II-10), a formula III (e.g., a formula III-1, a formula III-2, and a formula III-3), a formula IV-1 or IV-2 (e.g., a formula IV-3, a formula IV-4, a formula IV-5, a formula IV-6), a glycoside (e.g., a formula V), or a pharmaceutically acceptable salt or ester thereof, wherein an aglycone of the glycoside is a phenolic compound, a flavonoid, coumarin, benzoic acid, or a sterol, wherein the formula I, the formula II, the formula III, the formula IV-1, the formula IV-2, the formula V, and subformulae thereof are defined in specification.

22. The use according to claim 14, wherein the inhibitor or microbicid for the microorganism is compounds 1-8, having the following chemical structures: 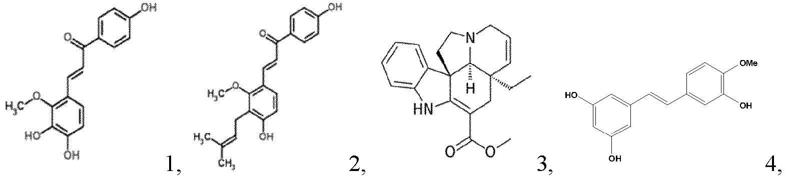 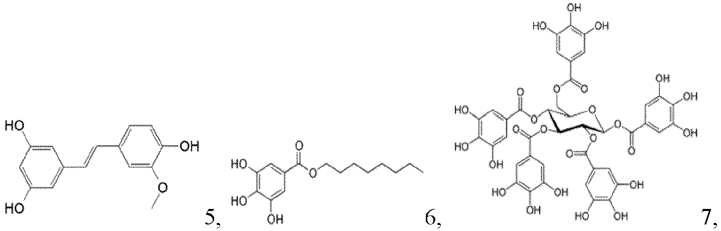 and

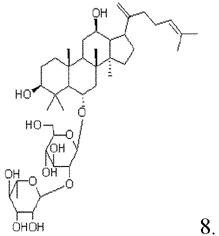

23. The use according to claim 1, wherein the inhibitor or microbicid for the microorganism is a natural plant or a natural plant extract or a combination of the natural plant and the natural plant extract, preferably, the natural plant is selected from one or a combination of two or more of radix paeoniae alba, fructus forsythiae, fructus aurantii, rhizoma rehmanniae, dried tangerine peel, pseudo-ginseng, turtle shells, roots of kirilow rhodiola, dried rehmannia roots, coix seeds, semen cassiae and liquorice.

24. The use according to claim 1, wherein the inhibitor or microbicid for the microorganism is a composition comprising turtle shells, roots of kirilow rhodiola, dried rehmannia roots, coix seeds, radix paeoniae alba, fructus forsythiae, semen cassiae and liquorice, preferably, the composition comprises, by weight, 5-15 parts the turtle shells, 10-30 parts the roots of kirilow rhodiola, 5-15 parts the dried rehmannia roots, 5-15 parts the coix seeds, 8-20 parts the radix paeoniae alba, 5-15 parts the fructus forsythiae, 5-15 parts the semen cassiae and 5-15 parts the liquorice, more preferably, the composition comprises 10 parts the turtle shells, 20 parts the roots of kirilow rhodiola, 10 parts the dried rehmannia roots, 10 parts the coix seeds, 12 parts the radix paeoniae alba, 10 parts the fructus forsythiae, 10 parts the semen cassiae and 9 parts the liquorice.

25. The use according to claim 1, wherein the drug is a preparation for ophthalmic administration or a preparation for gastrointestinal administration, preferably, the preparation for ophthalmic administration is an eye drop, an eye ointment, an eye gel, an ocular insert, an eye solution or an intraocular injection, preferably, the preparation for gastrointestinal administration is a tablet, a pill, powder, a granule, a capsule, a troche, syrup, a solution, an emulsion, a suspension, a controlled release preparation, an aerosol or a film agent.
